(19) Europäisches Patentamt / European Patent Office / Office européen des brevets

(11) **EP 3 328 898 B1**

(12) # EUROPEAN PATENT SPECIFICATION

(45) Date of publication and mention of the grant of the patent:
**06.03.2024 Bulletin 2024/10**

(21) Application number: **16831377.3**

(22) Date of filing: **28.07.2016**

(51) International Patent Classification (IPC):
***A61K 39/395*** *(2006.01)* ***C07K 16/28*** *(2006.01)*
***A61P 27/16*** *(2006.01)*

(52) Cooperative Patent Classification (CPC):
**C07K 16/2863; A61K 38/185; A61K 39/39591; A61P 7/10; A61P 9/00; A61P 25/00; A61P 27/16;**
A61K 2039/505; C07K 2317/75; C07K 2317/94

(86) International application number:
**PCT/US2016/044574**

(87) International publication number:
**WO 2017/019907 (02.02.2017 Gazette 2017/05)**

(54) **TRKB OR TRKC AGONIST COMPOSITIONS AND METHODS FOR THE TREATMENT OF OTIC CONDITIONS**

TRKB- ODER TRKC-AGONISTEN-ZUSAMMENSETZUNGEN UND VERFAHREN ZUR BEHANDLUNG VON ERKRANKUNGEN DES OHRS

COMPOSITIONS D'AGONISTE TRKB OU TRKC ET MÉTHODES DE TRAITEMENT DE TROUBLES OTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2015 US 201562198065 P**

(43) Date of publication of application:
**06.06.2018 Bulletin 2018/23**

(73) Proprietors:
- **Otonomy, Inc.**
  **San Diego, CA 92121 (US)**
- **Saragovi, Horacio Uri**
  **Montréal, Québec H4A 3L8 (CA)**

(72) Inventors:
- **SARAGOVI, Horacio Uri**
  **Montreal, Québec H4A 3L8 (CA)**
- **PIU, Fabrice**
  **San Diego, California 92131 (US)**
- **FOSTER, Alan**
  **San Diego, California 92127 (US)**
- **BLACK, KristenAnn**
  **Solana Beach, California 92075 (US)**

(74) Representative: **HGF**
**HGF Limited**
**1 City Walk**
**Leeds LS11 9DX (GB)**

(56) References cited:

| | |
|---|---|
| **WO-A2-2009/132050** | **WO-A2-2009/132050** |
| **WO-A2-2010/011609** | **WO-A2-2010/086828** |
| **US-A1- 2002 039 995** | **US-A1- 2002 176 859** |
| **US-A1- 2014 004 119** | **US-B2- 8 030 297** |

- **RUDOLF GLUECKERT ET AL: "Nanoparticle mediated drug delivery of rolipram to tyrosine kinase B positive cells in the inner ear with targeting peptides and agonistic antibodies", FRONTIERS IN AGING NEUROSCIENCE, vol. 7, 19 May 2015 (2015-05-19), pages 1-18, XP055530403, DOI: 10.3389/fnagi.2015.00071**
- **AMELIA TORCELLO-GÓMEZ ET AL: "Adsorption of antibody onto Pluronic F68-covered nanoparticles: link with surface properties", SOFT MATTER, vol. 7, no. 18, 1 January 2011 (2011-01-01), pages 8450-8461, ISSN: 1744-683X, DOI: 10.1039/c1sm05570d**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The complete document including Reference Table(s) and the Sequence Listing(s) can be downloaded from the EPO website

## Description

### SEQUENCE LISTING

The instant application contains a Sequence Listing which has been submitted electronically in ASCII format. Said ASCII copy, created on July 28, 2016, is named 37173-833_601_SL.txt and is 31,180 bytes in size.

### BACKGROUND OF THE INVENTION

Vertebrates have a pair of ears, placed symmetrically on opposite sides of the head. The ear serves as both the sense organ that detects sound and the organ that maintains balance and body position. The ear is generally divided into three portions: the outer ear, auris media (or middle ear) and the auris interna (or inner ear).
US2002/039995 discloses a pharmaceutical composition comprising TrkB or TrkC agonists for the treatment of diseases affecting the ear including ototoxin-induced hearing impairments.
WO2010/011609 discloses compositions and methods for the treatment of otic diseases or conditions with antimicrobial agent compositions and formulations administered locally to an individual afflicted with an otic disease or condition, through direct application of these compositions and formulations onto or via perfusion into the targeted auris structure(s).
WO2009/132050 discloses compositions and methods for the treatment of otic disorders with immunomodulating agents and auris pressure modulators. In these methods, the auris compositions and formulations are administered locally to an individual afflicted with an otic disorder, through direct application of the immunomodulating and/or auris pressure modulating compositions and formulations onto the auris media and/or auris interna target areas, or via perfusion into the auris media and/or auris interna structures.
WO2010/086828 discloses TrkB agonist antibodies. The discosure further relates to therapeutic methods for use of these antibodies and antigen-binding portions thereof to improve nerve function, including treatment of peripheral neuropathies, such as Charcot-Marie-Tooth disease.

### SUMMARY OF THE INVENTION

The present invention provides an otic pharmaceutical composition comprising a therapeutically effective amount of a TrkB agonist, wherein the TrkB agonist is an antibody or a binding fragment thereof comprising light chain complementarity-determining regions (CDRs) comprising SEQ ID NOs: 14-16 and heavy chain CDRs comprising SEQ ID NOs: 17-19; and a pharmaceutical acceptable carrier, for use in a method of treating an otic condition, wherein the otic condition is selected from ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome, vestibular neuronitis, tinnitus, presbycusis, and microvascular compression syndrome, wherein the otic composition is capable of being administered on or near the round window membrane via intratympanic injection. In one embodiment, preferably the otic condition is sensorineural hearing loss.

In one embodiment of the invention, the otic pharmaceutical composition of the invention for use according to the invention, further comprises two or more characteristics selected from: (i) between about 0.001% and about 60% by weight of the TrkB agonist, or pharmaceutically acceptable prodrug or salt thereof; (ii) between about 14% and about 21% by weight of a polyoxyethylene-polyoxypropylene triblock copolymer; (iii) sterile water, q.s., buffered to provide a pH between about 5.5 and about 8.0; (iv) a gelation temperature between about 19 °C and about 42 °C; and (v) an apparent viscosity of about 100,000 cP to about 500,000 cP.

In a further embodiment of the invention, the antibody or binding fragment thereof is a monoclonal antibody, a diabody, a linear antibody, a single-chain antibody, a bi-specific antibody, a multispecific antibody formed from antibody fragments, a tandem antibody, a chimeric antibody, a murine antibody, a humanized antibody, a veneered antibody, a F(ab')2 fragment, a Fab' fragment, a Fab fragment, a Fv fragment, a rIgG fragment, or a scFv fragment.

In a further embodiment of the invention, the composition comprises between about 14% and about 17% by weight of a polyoxyethylene-polyoxypropylene triblock copolymer, and provides extended sustained release of the TrkB agonist over a period of from about 1 week to about 3 weeks. Preferably, the polyoxyethylene-polyoxypropylene triblock copolymer comprises poloxamer 407, poloxamer 188, poloxamer 237, or poloxamer 338. More preferably, the polyoxyethylene-polyoxypropylene copolymer is poloxamer 407.

In a further embodiment of the invention, the pharmaceutical composition is an auris-acceptable thermoreversible gel.

In a further embodiment of the invention, the composition has a gelation temperature of between about 19 °Cand about 42 °C.

Disclosed are antibodies or binding fragments thereof which comprise complementarity-determining regions (CDRs) of antibodies selected from the group consisting of 2B7, A5, E2, 6.1.2, 6.4.1, 2345, 2349, 2.5.1, 2344, 2345,

2248, 2349, 2250, 2253, 2256, 1D7, TAM-163, C20, A10, 7F5, 11E1, 17D11, 19E12, 36D1, 38B8, T1-HuC1, RN1026A, A2, 4B12, 4A6, TOA1, 37D12, 19H8(1), 1F8, 23B8, 18H6, 29D7, 5G5D2B5, 6B72C5, B13B15.1, C6D11.1, C10C3.1, C9N9.1, C4120.1, and A10F17.1. The TrkB agonist used in the invention is an antibody or a binding fragment thereof comprising light chain complementarity-determining regions (CDRs) comprising SEQ ID NOs: 14-16 and heavy chain CDRs comprising SEQ ID NOs: 17-19 which corresponds to the CDRs of antibody C2. Disclosed and not claimed are antibodies or a binding fragments thereof comprising complementarity-determining regions (CDRs) of antibodies selected from the group consisting of 1D7, TAM-163, 7F5, 11E1, 17D11, 19E12, 36D1, 38B8, 37D12, 19H8(1), 1F8, 23B8, 18H6, 29D7, 2B7, A5, 6.1.2, 6.4.1, 2345, 2349, 2.5.1, 2344, 2248, 2250, 2253, and 2256.

Disclosed but not claimed is a non-natural TrkC agonist which is an antibody selected from the group consisting of 2B7, A5, 6.1.2, 6.4.1, 2345, 2349, 2.5.1, 2344, 2248, 2250, 2253, and 2256. The non-natural TrkC agonist is an antibody selected from the group consisting of 2B7, A5, E2, 6.1.2, 6.4.1, 2345, 2349, 2.5.1, 2344, 2345, 2248, 2349, 2250, 2253, and 2256. The non-natural TrkC agonist is an antibody selected from the group consisting of 2B7, A5, E2, 6.1.2, 6.4.1, 2345, 2349, 2.5.1, and 2344.

Disclosed, but not claimed, the non-natural TrkB agonist is an antibody selected from the group consisting of 1D7, TAM-163, 7F5, 11E1, 17D11, 19E12, 36D1, 38B8, 37D12, 19H8(1), 1F8, 23B8, 18H6, and 29D7. In some embodiments, the non-natural TrkB agonist is antibody C2. Disclosed, but not claimed, the non-natural TrkB agonist is an antibody is selected from the group consisting of 1D7, TAM-163, C20, A10, 7F5, 11E1, 17D11, 19E12, 36D1, 38B8, T1-HuC1, RN1026A, A2, 4B12, 4A6, TOA1, 37D12, 19H8(1), 1F8, 23B8, 18H6, 29D7, 5G5D2B5, 6B72C5, B13B15.1, C6D11.1, C10C3.1, C9N9.1, C4120.1, and A10F17.1.

The non-natural TrkB agonist recognizes and binds to an epitope on TrkB, and disclosed but not claimed, the non-natural TrkC agonist recognizes and binds to an epitope on TrkC. The epitopes recognized and bound by non-natural TrkB or TrkC agonists, are distinct from the epitopes recognized and bound by naturally occurring TrkB or TrkC agonists. The epitopes recognized and bound by non-natural TrkB or TrkC agonists, are the same as the epitopes recognized and bound by naturally occurring TrkB or TrkC agonists. The epitopes recognized and bound by non-natural TrkB or TrkC agonists, are at the ectodomain of the target TrkB or TrkC receptors.

Dislcosed but not claimed, the non-natural TrkC agonist recognizes an epitope in domain 4 and/or domain 5 of TrkC. Disclosed but not claimed the non-natural TrkC agonist recognizes an epitope in domain 5 of TrkC. Disclosed but not claimed the non-natural TrkC agonist recognizes an epitope in domain 4 of TrkC. Disclosed but not claimed, the non-natural TrkC agonist recognizes an epitope comprising SEQ ID NO: 1.

Disclosed but not claimed , the non-natural TrkB agonist recognizes an epitope comprising SEQ ID NO: 118.

Disclosed but not claimed, the non-natural TrkC agonist is 2B7. 2B7 is a monoclonal antibody that binds to the full length TrkC receptor and does not bind to the truncated TrkC receptor TrkC.T1. The 2B7 is a monoclonal antibody that specifically recognizes and binds a juxtamembrane region, a peptide within the juxtamembrane region, or a peptide having the amino acid sequence ESTDNFILFDEVSPTPPI (SEQ ID NO. 1), of TrkC; or, a fragment, portion, variant or derivative of the monoclonal antibody, wherein said fragment, portion, variant or derivative specifically binds the juxtamembrane region, a peptide within the juxtamembrane region, or a peptide having the amino acid sequence ESTDNFILFDEVSPTPPI (SEQ ID NO. 1), of TrkC, and wherein the antibody 2B7 does not bind domain 5 of TrkC. The 2B7 monoclonal antibody or fragment, portion, variant or derivative thereof comprises complementarity-determining regions (CDRs) or hypervariable domains of an antibody produced by a hybridoma strain deposited under ATCC deposit number 090310-02.

Disclosed but not claimed , the non-natural TrkC agonist is A5. In some embodiments, the A5 is an antibody comprising heavy chain complementarity-determining regions (CDRs) comprising: (a) a CDR1 of the formula GYTFTSYXaaXaaH (SEQ ID NO:2), wherein Xaa at position 8 is R or W, and Xaa at position 9 is I, L, R, or M; (b) a CDR2 of the formula EIYPSNXaaRTNYNEKFXaaS (SEQ ID NO:3), wherein Xaa at position 7 is A, T, S, or G; and Xaa at position 16 is K or E; and (c) a CDR3 of the formula KYYYGNXaaXaaRSWYFDV (SEQ ID NO:4), wherein Xaa at position 7 is T or S; wherein Xaa at position 8 is R, Q, K, S, or Y; wherein the agonist anti-TrkC antibody is not an antibody comprising a heavy chain CDRs comprising a CDR1 region of SEQ ID NO:5, a CDR2 region of SEQ ID NO:6, and a CDR3 region of SEQ ID NO:7.

Disclosed but not claimed, the non-natural TrkC agonist is a human antibody selected from the group consisting of antibodies 6.1.2, 6.4.1, 2345, 2349, 2.5.1, and 2344.

Disclosed but not claimed, the antibodies 6.1.2., 6.4.1, 2345, 2349, 2.5.1, and 2344, are produced by hybridoma strains deposited under ATCC deposit numbers PTA-2150, PTA-2146, PTA-2153, PTA-2151, and PTA-2144, respectively. In some embodiments, the non-natural TrkC agonist is a murine antibody selected from the group consisting of antibodies 2248, 2250, 2253, and 2256.

Disclosed but not claimed, the antibodies 2248, 2250, 2253, and 2256 are produced by hybridoma strains deposited under ATCC deposit numbers PTA-2147, PTA-2149, PTA-2145, and PTA-2152, respectively. In some embodiments, the human antibody recognizes an epitope in domain 5 of TrkC.

Disclosed but not claimed, the murine antibody recognizes an epitope in domain 5 of TrkC.

Disclosed but not claimed, the non-natural TrkB agonist is 38B8 and wherein 38B8 is an isolated monoclonal TrkB agonist antibody produced by the hybridoma strain deposited under ATCC deposit number PTA-8766.

Disclosed but not claimed, the non-natural TrkB agonist is TAM-163.

Disclosed but not claimed, the CDRs comprise heavy chain CDR1, CDR2, and CDR3 and/or light chain CDR1, CDR2, and CDR3 and the CDRs are selected from SEQ ID NOs: 2-13 and 20-116.

Disclosed but not claimed, the non-natural TrkB agonist is selected from a group consisting of 7,8-Dihydroxyflavone , 7,8,3'-Trihydroxyflavone, 4'-Dimethylamino-7,8-dihydroxyflavone , Deoxygedunin, LM-22A4, TDP6, 3,7-Dihydroxyflavone, 3,7,8,2'-Tetrahydroxyflavone, 4'-Dimethylamino-7,8-dihydroxyflavone, 5,7,8-Trihydroxyflavone, 7,3'-Dihydroxyflavone, 7,8,2'-Trihydroxyflavone, N,N',N"Tris(2-hydroxyethyl)-1,3,5-benzenetricarboxamide, N-[2-(5-Hydroxy-1H-indol-3-yl)ethyl]-2-oxo-3-piperidinecarboxamide, N-acetylserotonin, and Amitryptiline.

Disclosed but not claimed, the TrkB or TrkC agonist is a naturally occurring neurotrophic agent with one or more mutations or modifications in amino acid residues. In some embodiments, the TrkB or TrkC agonist is a naturally occurring neurotrophic agent with one or more mutations in amino acid residues. In some embodiments, the TrkB or TrkC agonist is a naturally occurring neurotrophic agent with one or more modifications in amino acid residues. In some instances, the one or more modifications comprise chemical modifications.

Disclosed but not claimed, the non-natural TrkB or TrkC agonist is a chemically modified analog of a neurotrophic agent, wherein the neurotrophic agent is brain-derived neurotrophic factor (BDNF), ciliary neurotrophic factor (CNTF), glial cell-line derived neurotrophic factor (GDNF), neurotrophin-3, neurotrophin-4, fibroblast growth factor (FGF), or insulin-like growth factor (IGF).

Disclosed but not claimed, the neurotrophic agent is modified by phosphorylation or sulfurylation at serine, threonine, or tyrosine residues, by incorporating unnatural amino acids, by incorporating heavy amino acids, by incorporating D-amino acids, by biotinylation, by cyclisations, by acylation, by dimethylation, by amidation, by derivatization, by conjugation to carrier proteins, by pegylation, or by branching of peptide.

Disclosed but not claimed, the chemically modified analog of a neurotrophic agent recognizes and binds to an epitope of a TrkB or a TrkC receptor, with same affinity as an unmodified neurotrophic agent. Disclosed but not claimed, the chemically modified analog of a neurotrophic agent activates signals by a TrkB or a TrkC receptor, with comparable efficacy as an unmodified neurotrophic agent.

Disclosed but not claimed, the chemically modified analog of a neurotrophic agent recognizes and binds to an epitope of a TrkB or a TrkC receptor, with higher affinity compared to an unmodified neurotrophic agent. Disclosed but not claimedthe chemically modified analog of a neurotrophic agent has improved stability, longer circulation time, and reduced immunogenicity compared to an unmodified neurotrophic agent.

Disclosed but not claimed, the non-natural TrkB or TrkC agonist is released from the composition or device for a period of at least 3 days. Disclosed but not claimed, the non-natural TrkB or TrkC agonist is released from the composition or device for a period of at least 5 days.

Disclosed but not claimed, administering the otic composition comprising the non-natural TrkB or TrkC agonist treats sensorineural hearing loss, by inducing auris neuronal cell growth. Disclosed but not claimed, the otic condition is characterized by damaged ribbon synapse.

## BRIEF DESCRIPTION OF FIGURES

The invention is defined by the claims. Any aspects of the following figures which do not relate to theTrkB agonist of the claims is provided for comparative purposes only.

**Fig. 1** illustrates the anatomy of the ear.

**Fig. 2A** and **Fig. 2B** illustrate perilymph concentrations of BDNF (Fig. 2A) and NT3 (Fig. 2B) after a single intratympanic injection of 0.1% BDNF (1.05 mg/ml) or 0.1% NT3 (1.05 mg/ml) to rats.

**Fig**. **3** illustrates perilymph concentrations of TrkC agonist antibody following a single intratympanic injection of 0.1% TrkC agonist antibody (1 mg/ml) (triangles) or 1% TrkC agonist antibody (10 mg/ml) (squares) to rats.

**Fig. 4** illustrates perilymph concentrations of human IgG following a single intratympanic injection of 0.1% Hu IgG (circles) and1.0% Hu IgG (squares) to rats.

**Fig. 5** illustrates dose-dependent increase of p-ERK in 3T3 cells expressing human TrkC by NT-3 and test antibodies.

**Fig. 6** illustrates dose-dependent increase of p-ERK in HEK293 cells expressing human TrkB by BDNF and test antibodies.

**Fig. 7** illustrates neutrophic effects of Trk agonists in rat spiral ganglion neurons in culture.

**Fig. 8A** and **Fig. 8B** shows 2B7 binding to the full length and not to the truncated form of human TrkC.

## DETAILED DESCRIPTION OF THE INVENTION

The invention is defined by the claims and any other aspects, configurations or embodiments set forth herein not falling within the scope of the claims are for information only. Any references in the description to methods of treatment refer to the compounds, pharmaceutical compositions and medicaments of the present invention for use in a method for treatment of the human (or animal) body by therapy.

Provided herein are otic pharmaceutical compositions for use in treating or ameliorating hearing loss or reduction resulting from destroyed, stunted, malfunctioning, damaged, fragile or missing hair cells, neurons and their connections in the inner ear.

Disclosed herein are compositions, formulations, methods, uses, kits, and delivery devices for treating an otic condition. The otic condition is ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome, vestibular neuronitis, tinnitus, presbycusis, or microvascular compression syndrome. Disclosed herein are compositions, formulations, methods, uses, kits, and delivery devices for treating otic conditions which need repair of damaged ribbon synapses.

Also disclosed herein, are controlled release otic pharmaceutical compositions and formulations for treating otic conditions, including but not limited to ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome, vestibular neuronitis, tinnitus, presbycusis, presbycusis, and microvascular compression syndrome. The formulations described herein provide a constant, sustained, extended, or delayed rate of release of a TrkB or TrkC agonist into the otic environment and thus avoid any variability in drug exposure in treatment of ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome, vestibular neuronitis, tinnitus, presbycusis, presbycusis, or microvascular compression syndrome.

Further disclosed herein are otic formulations that are sterilized with stringent sterility requirements and are suitable for otic administration. In some aspects, the auris compatible compositions described herein are substantially free of pyrogens and/or microbes.

Disclosed herein are otic formulations that meet certain criteria for pH, osmolarity, ionic balance, sterility, endotoxin and/or pyrogen levels. The otic pharmaceutical compositions described herein are compatible with the otic environment and are suitable for administration to humans.

By way of non-limiting example, the use of the following commonly used solvents should be limited, reduced or eliminated when formulating agents for administration to the ear: alcohols, propylene glycol, and cyclohexane. Thus, an otic pharmaceutical composition or formulation disclosed herein is free or substantially free of alcohols, propylene glycol, and cyclohexane. Disclosed is an otic pharmaceutical composition or formulation which comprises less than about 50 ppm of each of alcohols, propylene glycol, and cyclohexane. Disclosed is an otic pharmaceutical composition or formulation which comprises less than about 25 ppm of each of alcohols, propylene glycol, and cyclohexane. Disclosed is an otic pharmaceutical composition or formulation which comprises less than about 20 ppm of each of alcohols, propylene glycol, and cyclohexane.

Disclosed is an otic composition or formulation which comprises less than about 10 ppm of each of alcohols, propylene glycol, and cyclohexane. Disclosed is an otic pharmaceutical composition or formulation which comprises less than about 5 ppm of each of alcohols, propylene glycol, and cyclohexane. Disclosed is an otic pharmaceutical composition or formulation which comprises less than about 1 ppm of each of alcohols, propylene glycol, and cyclohexane.

Further, otic preparations require particularly low concentrations of several potentially-common contaminants that are known to be ototoxic. Other dosage forms, while seeking to limit the contamination attributable to these compounds, do not require the stringent precautions that otic preparations require. For example, the following contaminants should be absent or nearly absent from otic preparations: arsenic, lead, mercury, and tin. Thus an otic pharmaceutical composition or formulation disclosed herein is free or substantially free of arsenic, lead, mercury, and tin. An otic pharmaceutical composition or formulation disclosed herein comprises less than about 50 ppm of each of arsenic, lead, mercury, and tin. An otic pharmaceutical composition or formulation disclosed herein comprises less than about 25 ppm of each of arsenic, lead, mercury, and tin. In some embodiments, an otic pharmaceutical composition or formulation disclosed herein comprises less than about 20 ppm of each of arsenic, lead, mercury, and tin. An otic pharmaceutical composition or formulation disclosed herein comprises less than about 10 ppm of each of arsenic, lead, mercury, and tin. An otic pharmaceutical composition or formulation disclosed herein comprises less than about 5 ppm of each of arsenic, lead, mercury, and tin. An otic pharmaceutical composition or formulation disclosed herein comprises less than about 1 ppm of each of arsenic, lead, mercury, and tin.

**Certain Definitions**

The term "auris-acceptable" with respect to a formulation, composition or ingredient, as used herein, includes having no persistent detrimental effect on the auris interna (or inner ear) of the subject being treated. By "auris-pharmaceutically acceptable," as used herein, refers to a material, such as a carrier or diluent, which does not abrogate the biological activity or properties of the compound in reference to the auris interna (or inner ear), and is relatively or is reduced in toxicity to the auris interna (or inner ear), i.e., the material is administered to an individual without causing undesirable biological effects or interacting in a deleterious manner with any of the components of the composition in which it is contained.

As used herein, amelioration or lessening of the symptoms of a particular otic disease, disorder or condition by administration of a particular compound or pharmaceutical composition refers to any decrease of severity, delay in onset, slowing of progression, or shortening of duration, whether permanent or temporary, lasting or transient that is attributed to or associated with administration of the compound or composition.

"Antioxidants" are auris-pharmaceutically acceptable antioxidants, and include, for example, butylated hydroxytoluene (BHT), sodium ascorbate, ascorbic acid, sodium metabisulfite and tocopherol. Antioxidants enhance chemical stability where required. Antioxidants are also used to counteract the ototoxic effects of certain therapeutic agents, including agents that are used in combination with the TrkB or TrkC agonists disclosed herein.

"Auris interna" refers to the inner ear, including the cochlea and the vestibular labyrinth, and the round window that connects the cochlea with the middle ear.

"Auris-interna bioavailability" refers to the percentage of the administered dose of compounds disclosed herein that becomes available in the inner ear of the animal or human being studied.

"Auris media" refers to the middle ear, including the tympanic cavity, auditory ossicles and oval window, which connects the middle ear with the inner ear.

"Balance disorder" refers to a disorder, illness, or condition which causes a subject to feel unsteady, or to have a sensation of movement. Included in this definition are dizziness, vertigo, disequilibrium, and pre-syncope. Diseases which are classified as balance disorders include, but are not limited to, Ramsay Hunt's Syndrome, Meniere's Disease, mal de debarquement, benign paroxysmal positional vertigo, and labyrinthitis.

"Blood plasma concentration" refers to the concentration of compounds provided herein in the plasma component of blood of a subject.

"Carrier materials" are excipients that are compatible with the TrkB or TrkC agonist, the auris interna and the release profile properties of the auris-acceptable pharmaceutical formulations. Such carrier materials include, e.g., binders, suspending agents, disintegration agents, filling agents, surfactants, solubilizers, stabilizers, lubricants, wetting agents, diluents, and the like. "Auris-pharmaceutically compatible carrier materials" include, but are not limited to, acacia, gelatin, colloidal silicon dioxide, calcium glycerophosphate, calcium lactate, maltodextrin, glycerine, magnesium silicate, polyvinylpyrrolidone (PVP), cholesterol, cholesterol esters, sodium caseinate, soy lecithin, taurocholic acid, phosphatidylcholine, sodium chloride, tricalcium phosphate, dipotassium phosphate, cellulose and cellulose conjugates, sugars sodium stearoyl lactylate, carrageenan, monoglyceride, diglyceride, pregelatinized starch, and the like.

The term "diluent" refers to chemical compounds that are used to dilute the TrkB or TrkC agonist prior to delivery and which are compatible with the auris interna.

"Dispersing agents," and/or "viscosity modulating agents" are materials that control the diffusion and homogeneity of the TrkB or TrkC agonist through liquid media. Examples of diffusion facilitators/dispersing agents include but are not limited to hydrophilic polymers, electrolytes, Tween® 60 or 80, PEG, polyvinylpyrrolidone (PVP; commercially known as Plasdone®), and the carbohydrate-based dispersing agents such as, for example, hydroxypropyl celluloses (e.g., HPC, HPC-SL, and HPC-L), hydroxypropyl methylcelluloses (e.g., HPMC K100, HPMC K4M, HPMC K15M, and HPMC K100M), carboxymethylcellulose sodium, methylcellulose, hydroxyethylcellulose, hydroxypropylcellulose, hydroxypropylmethylcellulose phthalate, hydroxypropylmethylcellulose acetate stearate (HPMCAS), noncrystalline cellulose, magnesium aluminum silicate, triethanolamine, polyvinyl alcohol (PVA), vinyl pyrrolidone/vinyl acetate copolymer (S630), 4-(1,1,3,3-tetramethylbutyl)-phenol polymer with ethylene oxide and formaldehyde (also known as tyloxapol), poloxamers (e.g., Pluronics F68®, F88®, and F108®, which are block copolymers of ethylene oxide and propylene oxide); and poloxamines (e.g., Tetronic 908®, also known as Poloxamine 908®, which is a tetrafunctional block copolymer derived from sequential addition of propylene oxide and ethylene oxide to ethylenediamine (BASF Corporation, Parsippany, N.J.)), polyvinylpyrrolidone K12, polyvinylpyrrolidone K17, polyvinylpyrrolidone K25, or polyvinylpyrrolidone K30, polyvinylpyrrolidone/vinyl acetate copolymer (S-630), polyethylene glycol, e.g., the polyethylene glycol has a molecular weight of about 300 to about 6000, or about 3350 to about 4000, or about 7000 to about 5400, sodium carboxymethylcellulose, methylcellulose, polysorbate-80, sodium alginate, gums, such as, e.g., gum tragacanth and gum acacia, guar gum, xanthans, including xanthan gum, sugars, cellulosics, such as, sodium carboxymethylcellulose, methylcellulose, sodium carboxymethylcellulose, polysorbate-80, sodium alginate, polyethoxylated sorbitan monolaurate, polyethoxylated sorbitan monolaurate, povidone, carbomers, polyvinyl alcohol (PVA), alginates, chitosans and combinations thereof.

Plasticizers such as cellulose or triethyl cellulose are also be used as dispersing agents. Dispersing agents useful in liposomal dispersions and self-emulsifying dispersions of the TrkB or TrkC agonists disclosed herein are dimyristoyl phosphatidyl choline, natural phosphatidyl choline from eggs, natural phosphatidyl glycerol from eggs, cholesterol and isopropyl myristate.

"Drug absorption" or "absorption" refers to the process of movement of the TrkB or TrkC agonists from the localized site of administration, by way of example only, the round window membrane of the inner ear, and across a barrier (the round window membranes, as described below) into the auris interna or inner ear structures. The terms "coadministration" or the like, as used herein, are meant to encompass administration of the TrkB or TrkC agonists to a single patient, and are intended to include treatment regimens in which the TrkB or TrkC agonists are administered by the same or different route of administration or at the same or different time.

The terms "effective amount" or "therapeutically effective amount," as used herein, refer to a sufficient amount of the TrkB or TrkC agonist being administered that would be expected to relieve to some extent one or more of the symptoms of the disease or condition being treated. For example, the result of administration of the TrkB or TrkC agonist disclosed herein is reduction and/or alleviation of the signs, symptoms, or causes of tinnitus or balance disorders. For example, an "effective amount" for therapeutic uses is the amount of TrkB or TrkC agonist, including a formulation as disclosed herein required to provide a decrease or amelioration in disease symptoms without undue adverse side effects. The term "therapeutically effective amount" includes, for example, a prophylactically effective amount. An "effective amount" of a modulator of neuron and/or hair cells of the auris composition disclosed herein is an amount effective to achieve a desired pharmacologic effect or therapeutic improvement without undue adverse side effects. It is understood that "an effective amount" or "a therapeutically effective amount" varies, in some embodiments, from subject to subject, due to variation in metabolism of the compound administered, age, weight, general condition of the subject, the condition being treated, the severity of the condition being treated, and the judgment of the prescribing physician. It is also understood that "an effective amount" in an extended-release dosing format may differ from "an effective amount" in an immediate release dosign format based upon pharmacokinetic and pharmacodynamic considerations.

The terms "enhance" or "enhancing" refers to an increase or prolongation of either the potency or duration of a desired effect of TrkB or TrkC agonist, or a diminution of any adverse symptomatology that is consequent upon the administration of the therapeutic agent. Thus, in regard to enhancing the effect of the TrkB or TrkC agonists disclosed herein, the term "enhancing" refers to the ability to increase or prolong, either in potency or duration, the effect of other therapeutic agents that are used in combination with the TrkB or TrkC agonist disclosed herein. An "enhancing-effective amount," as used herein, refers to an amount of TrkB or TrkC agonist or other therapeutic agent which is adequate to enhance the effect of another therapeutic agent or TrkB or TrkC agonist of the target auris structure in a desired system. When used in a patient, amounts effective for this use will depend on the severity and course of the disease, disorder or condition, previous therapy, the patient’s health status and response to the drugs, and the judgment of the treating physician.

The term "inhibiting" includes preventing, slowing, or reversing the development of a condition, for example, or advancement of a condition in a patient necessitating treatment.

The terms "kit" and "article of manufacture" are used as synonyms.

"Pharmacodynamics" refers to the factors which determine the biologic response observed relative to the concentration of drug at the desired site within the auris media and/or auris interna.

"Pharmacokinetics" refers to the factors which determine the attainment and maintenance of the appropriate concentration of drug at the desired site within the auris media and/or auris interna.

The term "TrkB or TrkC agonist" include agents that recognize and bind to one or more epitopes on TrkB or TrkC receptor. The TrkB or TrkC agonist, as disclosed herein, is an antibody. The TrkB or TrkC agonists are agents that promote the growth and/or regeneration of neurons and their processes and connections and/or the hair cells of the auris. As disclosed herein, a TrkB or TrkC agonist provides therapeutic benefit (e.g., alleviation of hearing loss) by promoting the growth and/or regeneration and/or phenotypic maintenance of auris sensory cells and their processes and connections (e.g., neurons and/or the hair cells) of the auris. As disclosed herein, a TrkB or TrkC agonist provides therapeutic benefit (e.g., alleviation of tinnitus due to acoustic trauma) by treating and/or reversing damage to auris sensory cells (e.g., dysfunction of neurons and/or hair cells of the auris) or reducing or delaying further damage (e.g., cell death) to auris sensory cells (e.g., by exerting an otoprotectant effect or a trophic effect).

TrkB or TrkC agonists include "neurotrophic agent" which means a chemically modified analog of a naturally occurring neurotrophic agent (e.g., BDNF, NT3, NT 4/5, IGF), or a naturally occurring neurotrophic agent with one or more mutations in amino acid residues, that promotes the survival, growth and/or regeneration of auris sensory cells (e.g., neurons and/or the hair cells of the auris). A neurotrophic agent reduces or inhibits oxidative damage and/or osteoneogenesis and/or degeneration of auris sensory cells. A neurotrophic agent maintains healthy auris sensory cells (e.g., after a surgical implant of a medical device). As disclosed herein, a neurotrophic agent is an immunosuppresant (e.g., an immunosuppresant used during otic surgery). As disclosed herein, a neurotrophic agent is a growth factor (e.g., a growth factor used after an implantation procedure to promote growth of auris cells).

In prophylactic applications, compositions comprising the TrkB or TrkC agonists described herein are administered to a patient susceptible to or otherwise at risk of a particular disease, disorder or condition. For example, such conditions include and are not limited to ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome, vestibular neuronitis, tinnitus and microvascular compression syndrome, synaptopahty, drug-induced neurodegeration of otic neurons. Such an amount is defined to be a "prophylactically effective amount or dose." In this use, the precise amounts also depend on the patient's state of health, weight, and the like.

As used herein, a "pharmaceutical device" includes any composition described herein that, upon administration to an ear, provides a reservoir for extended release of an active agent described herein.

The term "substantially low degradation products" means about 10% by weight of the active agent are degradation products of the active agent. As disclosed herein, the term means less than 10% by weight of the active agent are degradation products of the active agent. As disclosed herein, the term means less than 9% by weight of the active agent are degradation products of the active agent. As disclosed herein, the term means less than 8% by weight of the active agent are degradation products of the active agent. As disclosed herein, the term means less than 7% by weight of the active agent are degradation products of the active agent. As disclosed herein , the term means less than 6% by weight of the active agent are degradation products of the active agent. As disclosed herein , the term means less than 5% by weight of the active agent are degradation products of the active agent. As disclosed herein , the term means less than 4% by weight of the active agent are degradation products of the active agent. As disclosed herein , the term means less than 3% by weight of the active agent are degradation products of the active agent. As disclosed herein , the term means less than 2% by weight of the active agent are degradation products of the active agent. As disclosed herein , the term means less than 1% by weight of the active agent are degradation products of the active agent. As disclosed herein , any individual impurity (e.g., metal impurity, degradation products of active agent and/or excipients, or the like) present in a formulation described herein is less than 5%, less than 2%, or less than 1% by weight of the active agent. In some embodiments the formulation does not contain precipitate during storage or change in color after manufacturing and storage.

As used herein, the term "antibody" means an immunoglobulin molecule capable of specific binding to a target, such as a carbohydrate, polynucleotide, lipid, polypeptide, etc., through at least one antigen recognition site, located in the variable region of the immunoglobulin molecule. As used herein, the term encompasses not only intact polyclonal or monoclonal antibodies, but also fragments thereof (such as Fab, Fab', $F(ab')_2$, Fv), single chain (ScFv), mutants thereof, fusion proteins comprising an antibody portion, and any other modified configuration of the immunoglobulin molecule that comprises an antigen recognition site. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgAl and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known.

As used herein the term "monoclonal antibody" refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to polyclonal antibody preparations, which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method.

As used herein the term "human antibody" means an antibody having an amino acid sequence corresponding to that of an antibody produced by a human and/or has been made using any of the techniques for making human antibodies known in the art or disclosed herein. This definition of a human antibody includes antibodies comprising at least one human heavy chain polypeptide or at least one human light chain polypeptide. Human antibodies can be produced using various techniques known in the art. As disclosed herein , the human antibody is selected from a phage library, where that phage library expresses human antibodies. Human antibodies can also be made by introducing human immunoglobulin loci into transgenic animals, e.g., mice in which the endogenous immunoglobulin genes have been partially or completely inactivated. Alternatively, the human antibody may be prepared by immortalizing human B lymphocytes that produce an antibody directed against a target antigen (such B lymphocytes may be recovered from an individual or may have been immunized in vitro).

The term "veneered" versions of the antibodies provided herein may also be used in some embodiments. The process of veneering involves selectively replacing FR residues from, e.g., a murine heavy or light chain variable region, with human FR residues in order to provide an antibody that comprises an antigen binding portion which retains substantially all of the native FR protein folding structure. Veneering techniques are based on the understanding that the antigen binding characteristics of an antigen binding portion are determined primarily by the structure and relative disposition of the heavy and light chain CDR sets within the antigen-association surface. Thus, antigen association specificity can be preserved in a humanized antibody only wherein the CDR structures, their interaction with each other and their interaction with the rest of the variable region domains are carefully maintained. By using veneering techniques, exterior (e.g., solvent-accessible) FR residues which are readily encountered by the immune system are selectively replaced with human residues to provide a hybrid molecule that comprises either a weakly immunogenic, or substantially non-immunogenic veneered surface. It should be understood that veneered versions of the antibodies disclosed herein are encompassed by the present disclosure.

The term "antigen-binding portion" or "antigen-binding fragment" of an antibody (or simply "antibody portion" or "antibody fragment"), as used herein, refers to one or more fragments of an antibody that retain the ability to specifically bind to an antigen (e.g., juxtamembrane region domain of TrkC). It has been shown that the antigen-binding function of an antibody can be performed by fragments of a full-length antibody. Such antibody embodiments may also be bispecific, dual specific, or multi-specific formats; specifically binding to two or more different antigens. Examples of binding fragments encompassed within the term "antigen-binding portion" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment, which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules. Such single chain antibodies are also intended to be encompassed within the present invention. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites.

It should be understood that the antibodies described herein include fragments, portions, variants or derivatives thereof, such as single-chain antibodies or Fab fragments, that retain the same binding properties (e.g. specificity or affinity) of the full-length antibodies.

The term "otic intervention" means an external insult or trauma to one or more auris structures and includes implants, otic surgery, injections, cannulations, or the like. Implants include auris-interna or auris-media medical devices, examples of which include cochlear implants, hearing sparing devices, hearing-improvement devices, short electrodes, micro-prostheses or piston-like prostheses; needles; stem cell transplants; drug delivery devices; any cell-based therapeutic; or the like. Otic surgery includes middle ear surgery, inner ear surgery, tympanostomy, cochleostomy, labyrinthotomy, mastoidectomy, stapedectomy, stapedotomy, endolymphatic sacculotomy or the like. Injections include intratympanic injections, intracochlear injections, injections across the round window membrane or the like. Cannulations include intratympanic, intracochlear, endolymphatic, perilymphatic or vestibular cannulations or the like.

A "prodrug" refers to a TrkB or TrkC agonist that is converted into the parent drug *in vivo.* As disclosed herein, a prodrug is enzymatically metabolized by one or more steps or processes to the biologically, pharmaceutically or therapeutically active form of the compound. To produce a prodrug, a pharmaceutically active compound is modified such that the active compound will be regenerated upon *in vivo* administration. As disclosed herein, the prodrug is designed to alter the metabolic stability or the transport characteristics of a drug, to mask side effects or toxicity, or to alter other characteristics or properties of a drug. Compounds provided herein, in some embodiments, are derivatized into suitable prodrugs.

"Solubilizers" refer to auris-acceptable compounds such as triacetin, triethylcitrate, ethyl oleate, ethyl caprylate, sodium lauryl sulfate, sodium doccusate, vitamin E TPGS, dimethylacetamide, N-methylpyrrolidone, N-hydroxyethylpyrrolidone, polyvinylpyrrolidone, hydroxypropylmethyl cellulose, hydroxypropyl cyclodextrins, ethanol, n-butanol, isopropyl alcohol, cholesterol, bile salts, polyethylene glycol 200-600, glycofurol, transcutol, propylene glycol, and dimethyl isosorbide and the like that assist or increase the solubility of the TrkB or TrkC agonists disclosed herein.

"Stabilizers" refers to compounds such as any antioxidation agents, buffers, acids, preservatives and the like that are compatible with the environment of the auris interna. Stabilizers include but are not limited to agents that will do any of (1) improve the compatibility of excipients with a container, or a delivery system, including a syringe or a glass bottle, (2) improve the stability of a component of the composition, or (3) improve formulation stability.

"Steady state," as used herein, is when the amount of drug administered to the auris interna is equal to the amount of drug eliminated within one dosing interval resulting in a plateau or constant levels of drug exposure within the targeted structure.

As used herein, the term "subject" is used to mean an animal, preferably a mammal, including a human or non-human. The terms patient and subject may be used interchangeably.

"Surfactants" refer to compounds that are auris-acceptable, such as sodium lauryl sulfate, sodium docusate, Tween 60 or 80, triacetin, vitamin E TPGS, sorbitan monooleate, polyoxyethylene sorbitan monooleate, polysorbates, polaxomers, bile salts, glyceryl monostearate, copolymers of ethylene oxide and propylene oxide, e.g., Pluronic® (BASF), and the like. Some other surfactants include polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.As disclosed herein, surfactants are included to enhance physical stability or for other purposes.

The terms "treat," "treating" or "treatment," as used herein, include alleviating, abating or ameliorating a disease or condition, for example tinnitus, symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

Other objects, features, and advantages of the methods and compositions described herein will become apparent from the following detailed description. It should be understood, however, that the detailed description and the specific examples, while indicating specific embodiments, are given by way of illustration only.

### Anatomy of the Ear

As shown in Figure 1, the outer ear is the external portion of the organ and is composed of the pinna (auricle), the auditory canal (external auditory meatus) and the outward facing portion of the tympanic membrane, also known as the ear drum. The pinna, which is the fleshy part of the external ear that is visible on the side of the head, collects sound waves and directs them toward the auditory canal. Thus, the function of the outer ear, in part, is to collect and direct sound waves towards the tympanic membrane and the middle ear.

The middle ear is an air-filled cavity, called the tympanic cavity, behind the tympanic membrane. The tympanic membrane, also known as the ear drum, is a thin membrane that separates the external ear from the middle ear. The middle ear lies within the temporal bone, and includes within this space the three ear bones (auditory ossicles): the malleus, the incus and the stapes. The auditory ossicles are linked together via tiny ligaments, which form a bridge across the space of the tympanic cavity. The malleus, which is attached to the tympanic membrane at one end, is linked to the incus at its anterior end, which in turn is linked to the stapes. The stapes is attached to the oval window, one of two windows located within the tympanic cavity. A fibrous tissue layer, known as the annular ligament connects the stapes to the oval window. Sound waves from the outer ear first cause the tympanic membrane to vibrate. The vibration is transmitted across to the cochlea through the auditory ossicles and oval window, which transfers the motion to the fluids in the auris interna. Thus, the auditory ossicles are arranged to provide a mechanical linkage between the tympanic membrane and the oval window of the fluid-filled auris interna, where sound is transformed and transduced to the auris interna for further processing. Stiffness, rigidity or loss of movement of the auditory ossicles, tympanic membrane or oval window leads to hearing loss, e.g. otosclerosis, or rigidity of the stapes bone.

The tympanic cavity also connects to the throat via the eustachian tube. The eustachian tube provides the ability to equalize the pressure between the outside air and the middle ear cavity. The round window, a component of the auris interna but which is also accessible within the tympanic cavity, opens into the cochlea of the auris interna. The round window is covered by round window membrane, which consists of three layers: an external or mucous layer, an intermediate or fibrous layer, and an internal membrane, which communicates directly with the cochlear fluid. The round window, therefore, has direct communication with the auris interna via the internal membrane.

Movements in the oval and round window are interconnected, i.e. as the stapes bone transmits movement from the tympanic membrane to the oval window to move inward against the auris interna fluid, the round window (round window membrane) is correspondingly pushed out and away from the cochlear fluid. This movement of the round window allows movement of fluid within the cochlea, which leads in turn to movement of the cochlear inner hair cells, allowing hearing signals to be transduced. Stiffness and rigidity in round window membrane leads to hearing loss because of the lack of ability of movement in the cochlear fluid. Recent studies have focused on implanting mechanical transducers onto the round window, which bypasses the normal conductive pathway through the oval window and provides amplified input into the cochlear chamber.

Auditory signal transduction takes place in the auris interna. The fluid-filled auris interna, or inner ear, consists of two major components: the cochlear and the vestibular apparatus. The auris interna is located in part within the osseous or bony labyrinth, an intricate series of passages in the temporal bone of the skull. The vestibular apparatus is the organ of balance and consists of the three semi-circular canals and the vestibule. The three semi-circular canals are arranged relative to each other such that movement of the head along the three orthogonal planes in space can be detected by the movement of the fluid and subsequent signal processing by the sensory organs of the semi-circular canals, called the crista ampullaris. The crista ampullaris contains hair cells and supporting cells, and is covered by a dome-shaped gelatinous mass called the cupula. The hairs of the hair cells are embedded in the cupula. The semi-circular canals detect dynamic equilibrium, the equilibrium of rotational or angular movements.

When the head turns rapidly, the semicircular canals move with the head, but endolymph fluid located in the membranous semi-circular canals tends to remain stationary. The endolymph fluid pushes against the cupula, which tilts to one side. As the cupula tilts, it bends some of the hairs on the hair cells of the crista ampullaris, which triggers a sensory impulse. Because each semicircular canal is located in a different plane, the corresponding crista ampullaris of each semi-circular canal responds differently to the same movement of the head. This creates a mosaic of impulses that are transmitted to the central nervous system on the vestibular branch of the vestibulocochlear nerve. The central nervous system interprets this information and initiates the appropriate responses to maintain balance. Of importance in the central nervous system is the cerebellum, which mediates the sense of balance and equilibrium.

The vestibule is the central portion of the auris interna and contains mechanoreceptors bearing hair cells that ascertain static equilibrium, or the position of the head relative to gravity. Static equilibrium plays a role when the head is motionless or moving in a straight line. The membranous labyrinth in the vestibule is divided into two sac-like structures, the utricle and the saccule. Each structure in turn contains a small structure called a macula, which is responsible for maintenance of static equilibrium. The macula consists of sensory hair cells, which are embedded in a gelatinous mass (similar to the cupula) that covers the macula. Grains of calcium carbonate, called otoliths, are embedded on the surface of the gelatinous layer.

When the head is in an upright position, the hairs are straight along the macula. When the head tilts, the gelatinous mass and otoliths tilts correspondingly, bending some of the hairs on the hair cells of the macula. This bending action initiates a signal impulse to the central nervous system, which travels via the vestibular branch of the vestibulocochlear nerve, which in turn relays motor impulses to the appropriate muscles to maintain balance.

The cochlea is the portion of the auris interna related to hearing. The cochlea is a tapered tube-like structure which is coiled into a shape resembling a snail. The inside of the cochlea is divided into three regions, which is further defined by the position of the vestibular membrane and the basilar membrane. The portion above the vestibular membrane is the scala vestibuli, which extends from the oval window to the apex of the cochlea and contains perilymph fluid, an aqueous liquid low in potassium and high in sodium content. The basilar membrane defines the scala tympani region, which extends from the apex of the cochlea to the round window and also contains perilymph. The basilar membrane contains thousands of stiff fibers, which gradually increase in length from the round window to the apex of the cochlea. The fibers of the basement membrane vibrate when activated by sound. In between the scala vestibuli and the scala tympani is the cochlear duct, which ends as a closed sac at the apex of the cochlea. The cochlear duct contains endolymph fluid, which is similar to cerebrospinal fluid and is high in potassium.

The organ of Corti, the sensory organ for hearing, is located on the basilar membrane and extends upward into the cochlear duct. The organ of Corti contains hair cells, which have hairlike projections that extend from their free surface, and contacts a gelatinous surface called the tectorial membrane. Although hair cells have no axons, they are surrounded by sensory nerve fibers that form the cochlear branch of the vestibulocochlear nerve (cranial nerve VIII).

As discussed, the oval window, also known as the elliptical window communicates with the stapes to relay sound waves that vibrate from the tympanic membrane. Vibrations transferred to the oval window increases pressure inside the fluid-filled cochlea via the perilymph and scala vestibuli/scala tympani, which in turn causes the round window membrane to expand in response. The concerted inward pressing of the oval window/outward expansion of the round window allows for the movement of fluid within the cochlea without a change of intra-cochlear pressure. However, as vibrations travel through the perilymph in the scala vestibuli, they create corresponding oscillations in the vestibular membrane. These corresponding oscillations travel through the endolymph of the cochlear duct, and transfer to the basilar membrane. When the basilar membrane oscillates, or moves up and down, the organ of Corti moves along with it. The hair cell receptors in the Organ of Corti then move against the tectorial membrane, causing a mechanical deformation in the stereocilia of the hair cells. The deflection of stereocilia produces depolarization of the hair cells and a graded release of the neurotransmitter glutamate at the hair cell ribbon synapses. Glutamate activates receptors on the cochlea afferent fibers that connect to inner hair cells as part of ribbon synapses. The afferent fibers are dendrites from spiral ganglion neurons and their depolarization by glutamate is carried along the afferent fibers to the cell bodies where an action potential can be evoked. Action potentials in spiral ganglion neurons are transmitted via their axons which form the auditory (VIII$^{th}$ cranial) nerve to the central nervous system where these signals are perceived as sound. In this way, sound produces a mechanical stimulus within the cochlea that is transduced to an electrical signal by the organ of Corti that is perceived as sound by the central nervous system.

**Diseases**

Otic disorders produce symptoms which include but are not limited to hearing loss, nystagmus, vertigo, tinnitus, inflammation, infection and congestion. The otic disorders which are treated with the compositions disclosed herein are numerous and include ototoxicity, chemotherapy induced hearing loss, excitotoxicity, sensorineural hearing loss, noise induced hearing loss, Meniere's Disease/Syndrome, endolymphatic hydrops, labyrinthitis, Ramsay Hunt's Syndrome,

vestibular neuronitis, tinnitus, presbycusis, and microvascular compression syndrome.

**Excitotoxicity**

Excitotoxicity refers to the death or damaging of neurons and/or otic hair cells by glutamate and/or similar substances.

Glutamate is the most abundant excitatory neurotransmitter in the central nervous system. Pre-synaptic neurons release glutamate upon stimulation. It flows across the synapse, binds to receptors located on post-synaptic neurons, and activates these neurons. The glutamate receptors include the NMDA, AMPA, and kainate receptors. Glutamate transporters are tasked with removing extracellular glutamate from the synapse. Certain events (e.g. ischemia or stroke) can damage the transporters. This results in excess glutamate accumulating in the synapse. Excess glutamate in synapses results in the over-activation of the glutamate receptors.

The AMPA receptor is activated by the binding of both glutamate and AMPA. Activation of certain isoforms of the AMPA receptor results in the opening of ion channels located in the plasma membrane of the neuron. When the channels open, $Na^+$ and $Ca^{2+}$ ions flow into the neuron and $K^+$ ions flow out of the neuron.

The NMDA receptor is activated by the binding of both glutamate and NMDA. Activation of the NMDA receptor, results in the opening of ion channels located in the plasma membrane of the neuron. However, these channels are blocked by $Mg^{2+}$ ions. Activation of the AMPA receptor results in the expulsion of $Mg^{2+}$ ions from the ion channels into the synapse. When the ion channels open, and the $Mg^{2+}$ ions evacuate the ion channels, $Na^+$ and $Ca^{2+}$ ions flow into the neuron, and $K^+$ ions flow out of the neuron.

Excitotoxicity occurs when the NMDA receptor and AMPA receptors are over-activated by the binding of excessive amounts of ligands, for example, abnormal amounts of glutamate. The over-activation of these receptors causes excessive opening of the ion channels under their control. This allows abnormally high levels of $Ca^{2+}$ and $Na^+$ to enter the neuron. The influx of these levels of $Ca^{2+}$ and $Na^+$ into the neuron causes the neuron to fire more often, resulting in a rapid buildup of free radicals and inflammatory compounds within the cell. The free radicals eventually damage the mitochondria, depleting the cell's energy stores. Furthermore, excess levels of $Ca^{2+}$ and $Na^+$ ions activate excess levels of enzymes including, but not limited to, phospholipases, endonucleases, and proteases. The over-activation of these enzymes results in damage to the cytoskeleton, plasma membrane, mitochondria, and DNA of the sensory neuron. As disclosed hereina TrkB or TrkC agonist is a functional glutamate receptor antagonist that reduces or inhibits excessive neuronal firing and/or neuronal cell death by modulating glutamate receptor responses and/or modifying the expression of glutamate receptors and/or their associated proteins. Disclosed herein but not claimed, is a pharmaceutical composition for use in the treatment of a disease of the ear characterized by the dysfunction of an NMDA receptor.

**Tinnitus**

As used herein, "tinnitus" refers to a disorder characterized by the perception of sound in the absence of any external stimuli. In certain instances, tinnitus occurs in one or both ears, continuously or sporadically, and is most often described as a ringing sound. It is most often used as a diagnostic symptom for other diseases. There are two types of tinnitus: objective and subjective. The former is a sound created in the body which is audible to anyone. The latter is audible only to the affected individual. Studies estimate that over 50 million Americans experience some form of tinnitus. Of those 50 million, about 12 million experience severe tinnitus.

There are several treatments for tinnitus. Lidocaine, administered by IV, reduces or eliminates the noise associated with tinnitus in about 60-80% of sufferers. Selective neurotransmitter reuptake inhibitors, such as nortriptyline, sertraline, and paroxetine, have also demonstrated efficacy against tinnitus. Benzodiazepines are also prescribed to treat tinnitus. As disclosed herein, a TrkB or TrkC agonist reduces or inhibits auris sensory cell damage and/or death associated with tinnitus.

**Sensorineural Hearing Loss**

Sensorineural hearing loss is a type of hearing loss which results from defects (congenital and acquired) in the vestibulocochlear nerve (also known as cranial nerve VIII), or sensory cells of the inner ear. The majority of defects of the inner ear are defects of otic hair cells and sensory neurons.

Aplasia of the cochlea, chromosomal defects, and congenital cholesteatoma are examples of congenital defects which can result in sensorineural hearing loss. By way of non-limiting example, inflammatory diseases (e.g. suppurative labyrinthitis, meningitis, mumps, measles, viral syphilis, and autoimmune disorders), Meniere's Disease, exposure to ototoxic drugs (e.g. aminoglycosides, loop diuretics, antimetabolites, salicylates, and cisplatin), physical trauma, presbyacusis, and acoustic trauma (prolonged exposure to sound in excess of 90 dB) can all result in acquired sensorineural hearing loss.

If the defect resulting in sensorineural hearing loss is a defect in the auditory pathways, the sensorineural hearing loss is called central hearing loss. If the defect resulting in sensorineural hearing loss is a defect in the auditory pathways, the sensorineural hearing loss is called cortical deafness. As disclosed herein, a TrkB or TrkC agonist is a neurotrophic agent (e.g., BDNF, GDNF) that promotes growth of auris sensory cells and their processes and connections and reduces or reverses sensorineural hearing loss.

**Noise Induced Hearing Loss**

Noise induced hearing loss (NIHL) is caused upon exposure to sounds that are too loud or loud sounds that last an extended period of time. Long or repeated or impulse exposure to sounds at or above 85 decibels can cause hearing loss. Hearing loss may also occur from prolonged exposure to loud noises, such as loud music, heavy equipment or machinery, airplanes, gunfire or other human-based noises. NIHL causes damage to the hair cells and/or the auditory nerve. The hair cells are small sensory cells that convert sound energy into electrical signals that travel to the brain. Impulse sound can result in immediate hearing loss that may be permanent. This kind of hearing loss may be accompanied by tinnitus-a ringing, buzzing, or roaring in the ears or head which may subside over time. Hearing loss and tinnitus may be experienced in one or both ears, and tinnitus may continue constantly or occasionally throughout a lifetime. Continuous exposure to loud noise also damages the structure of hair cells and sensory neurons, resulting in permanent hearing loss and tinnitus, although the process occurs more gradually than for impulse noise.

As disclosed herein, but not claimed, an otoprotectant can reverse, reduce or ameliorate NIHL. Examples of otoprotectants that treat or prevent NIHL include, but are not limited to, otoprotectants described herein.

**Ototoxicity**

Ototoxicity refers to hearing loss caused by a toxin. The hearing loss may be due to trauma to otic hair cells, the cochlea, and/or the cranial nerve VIII. Multiple drugs are known to be ototoxic. Often ototoxicity is dose-dependent. It may be permanent or reversible upon withdrawal of the drug.

Known ototoxic drugs include, but are not limited to, the aminoglycoside class of antibiotics (e.g. gentamicin, and amikacin), some members of the macrolide class of antibiotics (e.g erythromycin), some members of the glycopeptide class of antibiotics (e.g. vancomycin), salicylic acid, nicotine, some chemotherapeutic agents (e.g. actinomycin, bleomycin, cisplatin, carboplatin and vincristine), and some members of the loop diuretic family of drugs (e.g. furosemide), 6-hydroxy dopamine (6-OH DPAT), 6,7-dinitroquinoxaline-2,3-dione (DNQX) or the like.

Chemotherapeutic agents and the aminoglycoside class of antibiotics induce the production of reactive oxygen species ("ROS"). ROS can damage cells directly by damaging DNA, polypeptides, and/or lipids. Antioxidants prevent damage of ROS by preventing their formation or scavenging free radicals before they can damage the cell. Both chemotherapeutic agents and the aminoglycoside class of antibiotics are also thought to damage the ear by binding melanin in the stria vascularis of the inner ear. In some instances, hearing loss induced by chemotherapy agents such as cisplatin, actinomycin, bleomycin, carboplatin, oxaliplatin and vincristine is referred to as chemotherapy induced hearing loss.

Salicylic acid is classified as ototoxic as it inhibits the function of the polypeptide prestin. Prestin mediates outer otic hair cell motility by controlling the exchange of chloride and carbonate across the plasma membrane of outer otic hair cells. It is only found in the outer otic hair cells, not the inner otic hair cells. Accordingly, disclosed herein is the use of controlled release auris-compositions comprising otoprotectants (e.g. antioxidants) to prevent, ameliorate or lessen ototoxic effects of chemotherapy, including but not limited to cisplatin treatment, aminoglycoside or salicylic acid administration, or other ototoxic agents.

**Endolymphatic Hydrops**

Endolymphatic hydrops refers to an increase in the hydraulic pressure within the endolymphatic system of the inner ear. The endolymph and perilymph are separated by thin membranes which contain multiple nerves. Fluctuation in pressure stresses the membranes and the nerves they house. If the pressure is great enough, disruptions may form in these membranes. This results in a mixing of the fluids which can lead to a depolarization blockade and transient loss of function. Changes in the rate of vestibular nerve firing often lead to vertigo. Further, the organ of Corti may also be affected. Distortions of the basilar membrane and the inner and outer hair cells can lead to hearing loss and/or tinnitus.

Causes include metabolic disturbances, hormonal imbalances, autoimmune disease, and viral, bacterial, or fungal infections. Symptoms include hearing loss, vertigo, tinnitus, and aural fullness. Nystagmus may also be present. Treatment includes systemic administration of benzodiazepine, diuretics (to decrease the fluid pressure), corticosteroids, and/or anti-bacterial, anti-viral, or anti-fungal agents.

**Labyrinthitis**

Labyrinthitis is an inflammation of the labyrinths of the ear which contain the vestibular system of the inner ear. Causes include bacterial, viral, and fungal infections. It may also be caused by a head injury or allergies. Symptoms of labyrinthitis include difficulty maintaining balance, dizziness, vertigo, tinnitus, and hearing loss. Recovery may take one to six weeks; however, chronic symptoms may be present for years.

There are several treatments for labyrinthitis. Prochlorperazine is often prescribed as an antiemetic. Serotonin-reuptake inhibitors have been shown to stimulate new neural growth within the inner ear. Additionally, treatment with antibiotics is prescribed if the cause is a bacterial infection, and treatment with corticosteroids and antivirals is recommended if the condition is caused by a viral infection.

**Meniere’s Disease**

Meniere’s Disease is an idiopathic condition characterized by sudden attacks of vertigo, nausea and vomiting that may last for 3 to 24 hours, and may subside gradually. Progressive hearing loss, tinnitus and a sensation of pressure in the ears accompanies the disease through time. The cause of Meniere’s disease is likely related to an imbalance of inner ear fluid homeostasis, including an increase in production or a decrease in reabsorption of inner ear fluid.

Studies of the vasopressin (VP)-mediated aquaporin 2 (AQP2) system in the inner ear suggest a role for VP in inducing endolymph production, thereby increasing pressure in the vestibular and cochlear structures. VP levels were found to be upregulated in endolymphatic hydrops (Meniere’s Disease) cases, and chronic administration of VP in guinea pigs was found to induce endolymphatic hydrops. Treatment with VP antagonists, including infusion of OPC-31260 (a competitive antagonist of V2-R) into the scala tympani resulted in a marked reduction of Meniere’s disease symptoms. Other VP antagonists include WAY-140288, CL-385004, tolvaptan, conivaptan, SR 121463A and VPA 985. (Sanghi et al. Eur. Heart J. (2005) 26:538-543; Palm et al. Nephrol. Dial Transplant (1999) 14:2559-2562).

Other studies suggest a role for estrogen-related receptor β/NR3B2 (ERR/Nr3b2) in regulating endolymph production, and therefore pressure in the vestibular/cochlear apparatus. Knock-out studies in mice demonstrate the role of the polypeptide product of the Nr3b2 gene in regulating endolymph fluid production. Nr3b2 expression has been localized in the endolymph-secreting strial marginal cells and vestibular dark cells of the cochlea and vestibular apparatus, respectively. Moreover, conditional knockout of the Nr3b2 gene results in deafness and diminished endolymphatic fluid volume. Treatment with antagonists to ERR/Nr3b2 may assist in reducing endolymphatic volume, and thus alter pressure in the auris interna structures.

Other treatments may be aimed at dealing with the immediate symptoms and prevention of recurrence. Low-sodium diets, avoidance of caffeine, alcohol, and tobacco have been advocated. Medications that may temporarily relieve vertigo attacks include antihistamines (including meclizine and other antihistamines), and central nervous system agents, including barbiturates and/or benzodiazepines, including lorazepam or diazepam. Other examples of drugs that may be useful in relieving symptoms include muscarinic antagonists, including scopolamine. Nausea and vomiting may be relieved by suppositories containing antipsychotic agents, including the phenothiazine agent prochlorperazine.

Surgical procedures that have been used to relieve symptoms include the destruction of vestibular and/or cochlear function to relieve vertigo symptoms. These procedures aim to either reduce fluid pressure in the inner ear and/or to destroy inner ear balance function. An endolymphatic shunt procedure, which relieves fluid pressure, may be placed in the inner ear to relieve symptoms of vestibular dysfunction. Other treatments include gentamicin application, which when injected into the eardrum destroys sensory hair cell function, thereby eradicating inner ear balance function. Severing of the vestibular nerve may also be employed, which while preserving hearing, may control vertigo. As disclosed herein" an auris sensory cell modulator promotes growth of hair cells and allows a subject to regain inner ear balance function.

**Meniere’s Syndrome**

Meniere’s Syndrome, which displays similar symptoms as Meniere’s disease, is attributed as a secondary affliction to another disease process, e.g. thyroid disease or inner ear inflammation due to syphilis infection. Meniere’s syndrome, thus, are secondary effects to various process that interfere with normal production or resorption of endolymph, including endocrine abnormalities, electrolyte imbalance, autoimmune dysfunction, medications, infections (e.g. parasitic infections) or hyperlipidemia. Treatment of patients afflicted with Meniere’s Syndrome is similar to Meniere’s Disease.

**Ramsay Hunt’s Syndrome (Herpes Zoster Infection)**

Ramsay Hunt’s Syndrome is caused by a herpes zoster infection of the auditory nerve. The infection may cause severe ear pain, hearing loss, vertigo, as well as blisters on the outer ear, in the ear canal, as well as on the skin of the

face or neck supplied by the nerves. Facial muscles may also become paralyzed if the facial nerves are compressed by the swelling. Hearing loss may be temporary or permanent, with vertigo symptoms usually lasting from several days to weeks.

Treatment of Ramsay Hunt's syndrome includes administration of antiviral agents, including acyclovir. Other antiviral agents include famciclovir and valacyclovir. Combination of antiviral and corticosteroid therapy may also be employed to ameliorate herpes zoster infection. Analgesics or narcotics may also be administered to relieve the pain, and diazepam or other central nervous system agents to suppress vertigo. Capsaicin, lidocaine patches and nerve blocks are optionally used. Surgery may also be performed on compressed facial nerves to relieve facial paralysis.

### Microvascular Compression Syndrome

Microvascular compression syndrome (MCS), also called "vascular compression" or "neurovascular compression", is a disorder characterized by vertigo and tinnitus. It is caused by the irritation of Cranial Nerve VIII by a blood vessel. Other symptoms found in subjects with MCS include, but are not limited to, severe motion intolerance, and neuralgic like "quick spins". MCS is treated with carbamazepine, TRILEPTAL®, and baclofen. It can also be surgically treated.

### Vestibular Neuronitis

Vestibular neuronitis, or vestibular neuropathy, is an acute, sustained dysfunction of the peripheral vestibular system. It is theorized that vestibular neuronitis is caused by a disruption of afferent neuronal input from one or both of the vestibular apparatuses. Sources of this disruption include viral infection and acute localized ischemia of the vestibular nerve and/or labyrinth.

The most significant finding when diagnosing vestibular neuronitis is spontaneous, unidirectional, horizontal nystagmus. It is often accompanied by nausea, vomiting, and vertigo. It is, however, generally not accompanied by hearing loss or other auditory symptoms.

There are several treatments for vestibular neuronitis. H1-receptor antagonists, such as dimenhydrinate, diphenhydramine, meclizine, and promethazine, diminish vestibular stimulation and depress labyrinthine function through anticholinergic effects. Benzodiazepines, such as diazepam and lorazepam, are also used to inhibit vestibular responses due to their effects on the GABAA receptor. Anticholinergics, for example scopolamine, are also prescribed. They function by suppressing conduction in the vestibular cerebellar pathways. Finally, corticosteroids (i.e. prednisone) are prescribed to ameliorate the inflammation of the vestibular nerve and associated apparatus.

### Presbycusis

Age-related hearing loss (presbycusis) is the loss of hearing that gradually occurs with ageing. It is one of the most common conditions affecting older and elderly adults. Approximately one in three people in the United States between the ages of 65 and 74 has hearing loss, and nearly half of those older than 75 have difficulty hearing. Having trouble hearing can make it hard to understand and follow a doctor's advice, respond to warnings, and hear phones, doorbells, and smoke alarms. Hearing loss can also make it hard to enjoy talking with family and friends, leading to feelings of isolation. Age-related hearing loss most often occurs in both ears, affecting them equally.

There are many causes of age-related hearing loss. Most commonly, it arises from changes in the inner ear as one ages, but it can also result from changes in the middle ear, or from complex changes along the nerve pathways from the ear to the brain. Certain medical conditions and medications may also play a role. Presbycusis may result from a gradual loss of spiral ganglion neuron afferent fibers and their synapses with hair cells (ribbon synapses), causing a disconnection between the sensory cells that detect sound and the auditory nerve that transmits this information to the auditory brain. Loss of spiral ganglion neurons and hair cells also occurs. Prior exposure to loud noise or other otic insults may exacerbate this ageing process, leading to an accelerated loss of hearing. Presbycusis also involves "hidden hearing loss", an inability to detect sound against a background noise ("speech-in-noise") despite a lack of marked changes in hearing thresholds. These more subtle decrements in hearing have been associated with a loss of spiral ganglion neuron afferent fibers and their synaptic connections with hair cells (ribbon synapses).

### Pharmaceutical Agents

Disclosed herein are otic compositions or formulations, comprising TrkB or TrkC agonists, that modulate the degeneration of auris sensory cells (e.g., neurons and their processes and connections and/or hair cells of the auris) and promote their reconnection. Otic compositions or formulations, comprising TrkB or TrkC agonists, described herein reduce or delay or reverse the degeneration of auris sensory cells (e.g., neurons and their processes and connections

and/or cells of the auris). Also disclosed herein are controlled release otic compositions, comprising TrkB or TrkC agonists, for treating or ameliorating hearing loss or reduction resulting from destroyed, stunted, malfunctioning, damaged, fragile or missing hair cells in the inner ear. Additionally provided herein are otic compositions or formulations that promote the growth and/or regeneration of auris sensory cells (e.g., neurons and their processes and connections and/or hair cells of the auris). As disclosed herein, TrkB or TrkC agonists are otoprotectants and reduce, reverse or delay damage to auris sensory cells (e.g., neurons and their processes and connections and/or hair cells of the auris). As disclosed herein, TrkB or TrkC agonists repair damage to the afferent sensory fibers and their ribbon synapses.

Otic and vestibular disorders have causes and symptoms that are responsive to the TrkB or TrkC agonists disclosed herein.

The TrkB or TrkC comprising otic compositions or formulations disclosed herein are optionally targeted directly to otic structures where treatment is needed; for example, one embodiment contemplated is the direct application of the formulations disclosed herein onto the round window membrane or the crista fenestrae cochlea of the auris interna, allowing direct access and treatment of the auris interna, or inner ear components. The formulation disclosed herein is applied directly to the oval window. As disclosed herein, , direct access is obtained through microinjection directly into the auris interna, for example, through cochlear microperfusion. Such embodiments also optionally comprise a drug delivery device, wherein the drug delivery device delivers the TrkB or TrkC agonist formulations through use of a needle and syringe, a pump, a microinjection device, an auris-acceptable in situ forming spongy material or any combination thereof.

The TrkB or TrkC agonist formulations disclosed herein further comprise otoprotectants that reduce, inhibit or ameliorate the ototoxicity of pharmaceutical agents disclosed herein, or reduce, inhibit or ameliorate the effects of other environmental factors, including excessive noise and the like. Examples of otoprotectants include, and are not limited to, otoprotectants described herein, thiols and/or thiol derivatives and/or pharmaceutically acceptable salts, or derivatives (e.g. prodrugs) thereof.

Moreover, some pharmaceutical excipients, diluents or carriers are potentially ototoxic. For example, benzalkonium chloride, a common preservative, is ototoxic and therefore potentially harmful if introduced into the vestibular or cochlear structures. In formulating a controlled release TrkB or TrkC agonist formulation, it is advised to avoid or combine the appropriate excipients, diluents or carriers to lessen or eliminate potential ototoxic components from the formulation, or to decrease the amount of such excipients, diluents or carriers. Optionally, a controlled release TrkB or TrkC agonist formulation includes otoprotective agents, such as antioxidants, alpha lipoic acid, calcium, fosfomycin or iron chelators, to counteract potential ototoxic effects that may arise from the use of specific therapeutic agents or excipients, diluents or carriers.

**Tropomyosin receptor kinase (Trk) agonists**

Trk tyrosine kinase receptors are multi-domain single-transmembrane receptors that play an important role in a wide spectrum of neuronal responses including survival, differentiation, growth and regeneration. Trk receptors are widely distributed in the central nervous system and the peripheral nervous system, and play a key role in neuronal survival, differentiation and maintenance of proper function. The relevance of Trk receptor function has been demonstrated in a number of neurodegenerative models, including stroke, spinal cord injury, optic nerve axotomy, glaucoma and amyotrophic lateral sclerosis.

There are three members of the Trk family: TrkA, TrkB, and TrkC, encoded, respectively, by the genes Ntrk1, Ntrk2, and Ntrk3 in rat or mouse genomic nomenclature, by NTRK1, NTRK2, and NTRK3 in human genomic nomenclature. The extracellular domains of native TrkA, TrkB and TrkC receptors have five functional domains that have been defined with reference to homologous or otherwise similar structures identified in various other proteins. The domains have been designated starting at the N-terminus of the amino acid sequence of the mature Trk receptors as 1) a first cysteine-rich domain extending from amino acid position 1 to about amino add position 32 of human TrkA, from amino acid position 1 to about amino acid position 36 of human TrkB, and from amino acid position 1 to about amino add position 48 of human TrkC; 2) a leucine-rich domain stretching from about amino add 33 to about amino add to about amino acid 104 in TrkA; from about amino acid 37 to about amino acid 108 in TrkB, and from about amino add 49 to about amino acid 120 in TrkC; 3) a second cysteine-rich domain from about amino acid 105 to about amino add 157 in TrkA: from about amino acid 109 to about amino acid 164 in TrkB; and from about amino acid 121 to about amino acid 177 in TrkC; 4) a first immunoglobulin-like domain stretching from about amino acid 176 to about amino acid 234 in TrkA; from about amino acid 183 to about amino acid 239 in TrkB; and from about amino acid 196 to about amino acid 257 in TrkC; and 5) a second immunoglobulin-like domain extending from about amino acid 264 to about amino add 330 in TrkA; from about amino acid 270 to about amino acid 334 in TrkB; and from about amino acid 288 to about amino acid 351 in TrkC.

The tropomyosin receptor kinases are high affinity receptors for naturally occurring neurotrophins, a family of protein growth factors which includes nerve growth factor (NGF), brain derived neurotrophic factor (BDNF), neurotrophin-3 (NT-3) and neurotrophins-4/5 (NT-4/5). NT-3, BDNF and NGF are essential growth factors for the development and maintenance of the nervous system.

A Trk receptor ectodomain termed D5 comprises the main neurotrophin binding site and is required for ligand-dependent receptor activation. Such receptor sites that define ligand-binding and functional-activation are termed "hot spots". Previously, it has been demonstrated that artificial ligands, such as antibodies, that bind to a receptor hot spot could be functionally active. For example, an agonistic mAb 5C3 directed to a hot spot of the TrkA D5 domain has been reported in LeSauteur et al., 1996, J. Neurosci. 16: 1308-1316.

Mature neurotrophins bind a selective Trk receptor with relatively high affinity (e.g. TrkB-BDNF, TrkA-NGF and TrkC-NT-3). TrkC is the preferred receptor for NT-3 and mediates the multiple effects of NT-3, including neuronal death or survival, and cellular differentiation. The Trk receptor has tyrosine kinase catalytic activity that is associated with the survival and differentiation of neurotrophic signals. Neurotrophin-induced Trk activity affords trophic (growth/survival) responses via MAPK and AKT, whereas PLC-$\gamma$ and fibroblast growth factor receptor substrate-2 (FRS-2) activity are involved in differentiation.

All mature neurotrophins also bind to $p75^{NTR}$, a neurotrophin receptor which binds all neurotrophins with low affinity but, in complex with the ubiquitous protein sortilin, makes a high-affinity receptor for precursor of mature neurotrophins or proneurotrophins. $p75^{NTR}$ is not a receptor protein-tyrosine kinase and recruits intracellular signaling different from that activated by Trks. $p75^{NTR}$ signaling is generally atrophic, promoting apoptosis, inhibiting neurite growth, and depressing synaptic strength. Unlike Trks, $p75^{NTR}$ is expressed on glial cells as well as on neurons. In the peripheral nervous system, $p75^{NTR}$ is expressed on Schwann cells after axotomy. It is known that the $p75^{NTR}$ receptor can affect Trk-binding or function, although the mechanism is not fully understood. It has been shown that $p75^{NTR}$ can unmask a cryptic "hot spot" of Trk receptors, suggesting the notion of allosteric regulation.

Described herein are otic compositions comprising non-natural agonists for TrkB or TrkC receptors. As disclosed herein" suitable non-natural agonists for TrkB or TrkC receptors include antibodies, binding fragments, variants, and derivatives, thereof. As disclosed herein, suitable non-natural agonists for TrkB or TrkC receptors include chemically modified analogs of neurotrophic agents. As disclosed herein, suitable non-natural agonists for TrkB or TrkC receptors include chimeras of antibodies and naturally occurring neurotrophic agents. As disclosed herein suitable non-natural agonists for TrkB or TrkC receptors include chimeras of antibodies (e.g., bi-specific antibodies) and chemically modified analogs of neurotrophic agents.

## TrkB receptor agonist antibody

TrkB is one of the most widely distributed neurotrophin receptors in the brain, whose expression is high in such areas as the neocortex, hippocampus, striatum, and brainstem. It is a multidomain transmembrane protein that consists of an extracellular ligand binding domain, a transmembrane region, and an intracellular tyrosine kinase domain. BDNF binding to TrkB induces autophosphorylation of TrkB and, subsequently, phosphorylation of several mediator kinases, including extracellular signal regulated kinase [mitogen-activated protein kinase (MAPK)], phosphatidylinositol 3-kinase/Akt, phospholipase C-$\gamma$, and their downstream targets.

As disclosed herein, the otic composition comprises a non-natural TrkB agonist. As disclosed herein non-natural TrkB agonists include agonist antibodies, fragments, variants, and derivatives, thereof. In some embodiments, suitable agonist antibodies are selective for TrkB and bind with affinities similar to or greater than naturally-occurring NT4 and BDNF polypeptides.

As disclosed herein, the non-natural TrkB agonist binds to domain 1 and domain 4 of the TrkB receptor. As disclosed herein but not claimed, the CDRs comprise at least 80%, 85%, 90%, 91%, 92%, 93%, 94%, 95%, 96%, 97%, 98%, 99%, or 100% sequence identity to a CDR selected from SEQ ID NOs: 14-37 and 74-116. As disclosed herein but not claimed, the CDRs comprise at least 80% sequence identity to a CDR selected from SEQ ID NOs: 14-37 and 74-116. As disclosed herein but not claimed, the CDRs comprise at least 85% sequence identity to a CDR selected from SEQ ID NOs: 14-37 and 74-116. As disclosed herein but not claimed, the CDRs comprise at least 90% sequence identity to a CDR selected from SEQ ID NOs: 14-37 and 74-116. As disclosed herein but not claimed, the CDRs comprise at least 95% sequence identity to a CDR selected from SEQ ID NOs: 14-37 and 74-116. As disclosed herein but not claimed, the CDRs comprise at least 96% sequence identity to a CDR selected from SEQ ID NOs: 14-37 and 74-116. As disclosed herein but not claimed, the CDRs comprise at least 97% sequence identity to a CDR selected from SEQ ID NOs: 14-37 and 74-116. In some instances, the CDRs are selected from SEQ ID NOs: 14-37 and 74-116. As disclosed herein but not claimed, the CDRs comprise at least 98% sequence identity to a CDR selected from SEQ ID NOs: 14-37 and 74-116. As disclosed herein but not claimed, the CDRs comprise at least 99% sequence identity to a CDR selected from SEQ ID NOs: 14-37 and 74-116. As disclosed herein but not claimed, the CDRs are selected from SEQ ID NOs: 14-37 and 74-116.

As disclosed herein but not claimed, the non-natural TrkB agonist is an antibody that selectively binds to TrkB receptor. As disclosed herein but not claimed, the non-natural TrkB agonist is an antibody that does not bind to TrkA or

TrkC receptors. In some embodiments, the non-natural TrkB agonist is an antibody that does not bind to the neurotrophic receptor $p75^{NTR}$.

As disclosed herein, binding of a non-natural TrkB agonist to TrkB receptor results in increased levels of phosphorylated TrkB, phosphorylated MAPK, phosphorylated Akt, phosphorylated ERK1/2, and phosphorylated phospholipase C-γ. In some embodiments, binding of a non-natural TrkB agonist to TrkB receptor leads to improved neuronal survival. As disclosed herein, administration of an otic composition comprising a non-natural TrkB agonist that binds to TrkB receptor leads to improved neuronal survival and treats or prevents an otic condition. As disclosed herein, administration of an otic composition comprising a non-natural TrkB agonist that binds to TrkB receptor leads to improved neuronal survival and treats or prevents an otic condition that requires reconnection of afferent sensory fibers and repair of ribbon synapses. As disclosed herein, administration of an otic composition comprising a non-natural TrkB agonist that binds to TrkB receptor treats or prevents presbycusis (age related hearing loss). As disclosed herein, administration of an otic composition comprising a non-natural TrkB agonist that binds to TrkB receptor leads to improved neuronal survival and treats sensorineural hearing loss.

As disclosed herein , the binding affinity of a TrkB agonist to TrkB receptor is about 0.10 to about 0.80 nM, about 0.15 to about 0.75 nM and about 0.18 to about 0.72 nM, about 1 nM to about 1.5 nM, about 2 nM to about 5 nM, about 10 nM to about 20 nM, about 30 nM to about 50 nM, about 75 nM to about 100 nM, about 125 nM to about 150 nM, about 160 nM to about 200 nM.As disclosed herein, the binding affinity is about 2 pM, about 5 pM, about 10 pM, about 15 pM, about 20 pM, about 40 pM, or greater than about 40 pM. As disclosed herein, the binding affinity is between about 2 pM and 22 pM. As disclosed herein, the binding affinity is less than about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. As disclosed herein, the binding affinity is about 10 nM. As disclosed herein, the binding affinity is less than about 10 nM. As disclosed herein, the binding affinity is about 0.1 nM or about 0.07 nM. As disclosed herein, the binding affinity is less than about 0.1 nM or less than about 0.07 nM. As disclosed herein, the binding affinity is any of about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM to any of about 2 pM, about 5 pM, about 10 pM, about 15 pM, about 20 pM, or about 40 pM. As disclosed herein, the binding affinity is any of about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. As disclosed herein, the binding affinity is about 2 pM, about 5 pM, about 10 pM, about 15 pM, about 20 pM, about 40 pM, or greater than about 40 pM. As disclosed herein, the binding affinity falls within any range bound by any of these values, for example, between about 175 nM and about 180 nM. As disclosed herein, the binding affinity is 100 nM. In some embodiments, the binding affinity is 200 nM.

As disclosed herein, the off-rate (or $k_{off}$) of a TrkB agonist to TrkB receptor is between about $10^{-1}$ and about $10^{-6}$ $s^{-1}$. As disclosed herein, the off-rate (or $k_{off}$) of a TrkB agonist to TrkB receptor is between about $10^{-2}$ and about $10^{-6}$ $s^{-1}$, about $10^{-3}$ and about $10^{-6}$ $s^{-1}$, about $10^{-4}$ and about $10^{-6}$ $s^{-1}$, about $10^{-2}$ and about $10^{-5}$ $s^{-1}$, about $10^{-2}$ and about $10^{-4}$ $s^{-1}$, about $10^{-2}$ and about $10^{-3}$ $s^{-1}$, about $10^{-3}$ and about $10^{-5}$ $s^{-1}$, about $10^{-3}$ and about $10^{-4}$ $s^{-1}$, about $10^{-4}$ and about $10^{-5}$ $s^{-1}$, about $10^{-1}$ and about $10^{-5}$ $s^{-1}$, about $10^{-1}$ and about $10^{-4}$ $s^{-1}$, about $10^{-1}$ and about $10^{-3}$ $s^{-1}$, or about $10^{-1}$ and about $10^{-2}$ $s^{-1}$. As disclosed herein , the off-rate (or $k_{off}$) of a TrkB agonist to TrkB receptor is about $10^{-1}$ $s^{-1}$, about $10^{-2}$ $s^{-1}$, about $10^{-3}$ $s^{-1}$, about $10^{-4}$ $s^{-1}$, about $10^{-5}$ $s^{-1}$, or about $10^{-6}$ $s^{-1}$.

**TrkB receptor agonist compounds**

As disclosed herein but not claimed, the otic composition comprises a TrkB agonist compound. As disclosed herein but not claimed, the TrkB agonist is a compound selected from a group consisting of 7,8-Dihydroxyflavone , 7,8,3'-Trihydroxyflavone, 4'-Dimethylamino-7,8-dihydroxyflavone , Deoxygedunin, LM-22A4, TDP6, 3,7-Dihydroxyflavone, 3,7,8,2'-Tetrahydroxyflavone, 4'-Dimethylamino-7,8-dihydroxyflavone, 5,7,8-Trihydroxyflavone, 7,3'-Dihydroxyflavone, 7,8,2'-Trihydroxyflavone, N,N',N"Tris(2-hydroxyethyl)-1,3,5-benzenetricarboxamide, N-[2-(5-Hydroxy-1H-indol-3-yl)ethyl]-2-oxo-3-piperidinecarboxamide, N-acetylserotonin, and Amitryptiline. As disclosed herein but not claimed, the TrkB agonist compound is in microparticulate form. As disclosed herein , administration of an otic composition comprising a TrkB agonist compound that binds to TrkB receptor leads to improved neuronal survival and treats or prevents an otic condition. As disclosed herein , administration of an otic composition comprising a TrkB agonist compound that binds to TrkB receptor leads to improved neuronal survival and treats or prevents an otic condition that requires repair of ribbon synapses. As disclosed herein but not claimed, administration of an otic composition comprising a TrkB agonist compound that binds to TrkB receptor treats or prevents presbycusis (age related hearing loss). As disclosed herein but not claimed, administration of an otic composition comprising a TrkB agonist compound that binds to TrkB receptor leads to improved neuronal survival and treats sensorineural hearing loss.

## TrkC receptor agonist antibody

TrkC is a transmembrane receptor with intrinsic tyrosine kinase catalytic activity that triggers "positive" signaling cascades that activate mediators phospho-AKT, phospho-Erk, and phospho-PLC-$\gamma$. In the inner ear, activation of TrkC receptors promotes growth of sensory neurons and their afferent fibers during development and helps to establish appropriate connections with hair cells through ribbon synapses that are important for inner ear function. Following noise trauma in the adult, TrkC receptor activation restores afferent fiber growth and reestablishment of ribbon synapses.

As disclosed herein but not claimed, the otic composition comprises non-natural TrkC agonists. As disclosed herein but not claimed, non-natural TrkC agonists include agonist antibodies, fragments, variants, and derivatives, thereof. As disclosed herein but not claimed, suitable agonist antibodies are selective for TrkC and bind with affinities similar to or greater than naturally-occurring neurotrophic agent NT3. As disclosed herein but not claimed, the non-natural TrkC agonist is an antibody that selectively binds to TrkC receptor. As disclosed herein but not claimed, the non-natural TrkC agonist is an antibody that does not bind to TrkA or TrkB receptors. As disclosed herein but not claimed, the non-natural TrkC agonist is an antibody that does not bind to the neurotrophic receptor $p75^{NTR}$. As disclosed herein but not claimed, the non-natural TrkC agonist binds to the full length TrkC receptor. In some instances, the non-natural TrkC agonist does not bind to the truncated TrkC receptor, TrkC.T1. As disclosed herein but not claimed, the non-natural TrkC agonist is a small molecule. As disclosed herein but not claimed, the non-natural TrkC agonist is a small molecule that does not bind to the truncated TrkC receptor, TrkC.T1. As disclosed herein but not claimed, the non-natural TrkC agonist is a small molecule that binds only to the full length TrkC receptor.

As disclosed herein but not claimed, the non-natural TrkC agonist is antibody 2B7, A5, E2, 6.1.2, 6.4.1, 2345, 2349, 2.5.1, 2344, 2345, 2248, 2349, 2250, 2253, or 2256. As disclosed herein but not claimed, the non-natural TrkC agonist is antibody 2B7, A5, 6.1.2, 6.4.1, 2345, 2349, 2.5.1, 2344, 2248, 2250, 2253, or 2256. As disclosed herein but not claimed, the non-natural TrkC agonist is antibody 2B7, A5, E2, 6.1.2, 6.4.1, 2345, 2349, 2.5.1, or 2344. As disclosed herein but not claimed, the non-natural TrkC agonist is antibody A5, or antibody 2B7.

As disclosed herein but not claimed, the non-natural TrkC agonist is an antibody or a binding fragment thereof that specifically binds an epitope bound by one or more antibodies selected from the group consisting of 2B7, A5, E2, 6.1.2, 6.4.1, 2345, 2349, 2.5.1, 2344, 2345, 2248, 2349, 2250, 2253, or 2256. As disclosed herein but not claimed, the non-natural TrkC agonist is an antibody or a binding fragment thereof that specifically binds an epitope bound by one or more antibodies selected from the group consisting of 2B7, A5, 6.1.2, 6.4.1, 2345, 2349, 2.5.1, 2344, 2248, 2250, 2253, or 2256. As disclosed herein but not claimed, the non-natural TrkC agonist is an antibody or a binding fragment thereof that specifically binds an epitope bound by one or more antibodies selected from the group consisting of 2B7, A5, E2, 6.1.2, 6.4.1, 2345, 2349, 2.5.1, or 2344. As disclosed herein but not claimed, the non-natural TrkC agonist is an antibody or a binding fragment thereof that specifically binds an epitope bound by one or more antibodies selected from the group consisting of A5 or 2B7.

As disclosed herein but not claimed, an epitope comprises D1, D2, D3, D4 and/or D5 of TrkC. As disclosed herein but not claimed, an epitope comprises D1, D2, D3, D4, D5 or a combination thereof of TrkC. As disclosed herein but not claimed, an epitope comprises D4 and/or D5 of TrkC. In some cases, the non-natural TrkC agonist is an antibody or a binding fragment thereof that specifically binds to D1, D2, D3, D4 and/or D5 of TrkC. In some cases, the non-natural TrkC agonist is an antibody or a binding fragment thereof that specifically binds to D1, D2, D3, D4, D5 or a combination thereof of TrkC. In some cases, the non-natural TrkC agonist is an antibody or a binding fragment thereof that specifically binds to D4 and/or D5 of TrkC. In some cases, the non-natural TrkC agonist is an antibody or a binding fragment thereof that specifically binds to D 1 of TrkC. In some cases, the non-natural TrkC agonist is an antibody or a binding fragment thereof that specifically binds to D2 of TrkC. In some cases, the non-natural TrkC agonist is an antibody or a binding fragment thereof that specifically binds to D3 of TrkC. In some cases, the non-natural TrkC agonist is an antibody or a binding fragment thereof that specifically binds to D4 of TrkC. In some cases, the non-natural TrkC agonist is an antibody or a binding fragment thereof that specifically binds to D5 of TrkC.

As disclosed herein but not claimed, an epitope comprises ESTDNFILFDEVSPTPPI (SEQ ID NO. 1) of TrkC. In some cases, the non-natural TrkC agonist is an antibody or a binding fragment thereof that specifically binds SEQ ID NO: 1.

As disclosed herein but not claimed, the non-natural TrkC agonist is antibody 2B7, as described in U.S. patent publication number 20140004119 (application serial number 13/820,715). As disclosed herein but not claimed, the 2B7 antibody binds to full length TrkC. As disclosed herein but not claimed, the 2B7 antibody does not bind to the truncated TrkC receptor, TrkC.T1. As disclosed herein but not claimed, the 2B7 antibody binds to one or more specific epitopes near the juxtamembrane region of human TrkC. As disclosed herein but not claimed, the 2B7 antibody binds specifically to the region between the transmembrane domain and the D5 domain of human, rat or mouse TrkC. As disclosed herein but not claimed, the binding epitope for the 2B7 antibody is the sequence ESTDNFILFDEVSPTPPI (SEQ ID NO: 1), of TrkC. As disclosed herein but not claimed, the 2B7 antibody does not bind to TrkA, TrkB, or $p75^{NTR}$. As disclosed herein but not claimed, the antibody 2B7 is produced by the hybridoma having ATCC patent deposit designation 090310-02,

said fragments, portions, variants or derivatives binding specifically to the same epitope as the monoclonal antibody. As disclosed herein but not claimed, the 2B7 antibody comprises complementarity-determining regions (CDRs) and/or hypervariable domains of an antibody produced by a hybridoma having ATCC patent deposit designation 090310-02. As disclosed herein but not claimed, the monoclonal antibody produced by the hybridoma having ATCC patent deposit designation 090310-02 or antigen-binding fragments, portions, variants or derivatives thereof is humanized, veneered, or chimeric.

As disclosed herein but not claimed, the non-natural TrkC agonist comprises A5 antibody and its derivatives. As disclosed herein but not claimed, the non-natural TrkC agonist is A5 antibody. The antibody A5 corresponds to the antibody A5 described in European patent publication no. EP2402756 (application serial number EP 11183081.6). As disclosed herein but not claimed, the A5 antibody binds to the TrkC receptor. As disclosed herein but not claimed, the A5 antibody binds to one or more binding epitopes of the TrkC receptor. As disclosed herein but not claimed, the A5 antibody comprises a light chain that is encoded by a polynucleotide that is produced by a host cell with a deposit number of ATCC No. PTA-5682. As disclosed herein but not claimed, the A5 antibody comprises a heavy chain that is encoded by a polynucleotide that is produced by a host cell with a deposit number of ATCC No. PTA-5683. As disclosed herein but not claimed the A5 antibody comprises, (a) antibody A5; (b) a fragment or a region of the antibody A5; (c) a light chain of the antibody A5(SEQ ID NO. 8); (c) a heavy chain of the antibody A5 (SEQ ID NO. 9); (d) one or more variable region(s) from a light chain and/or a heavy chain of the antibody A5; (e) one or more CDR(s) (one, two, three, four, five or six CDRs) of antibody A5 and (f) an antibody comprising any one of (b) through (e). As disclosed herein but not claimed, the A5 antibody is of any one or more of (a) through (e). As disclosed herein but not claimed, A5 antibody further comprises the human heavy chain IgG2a constant region containing the following mutations: A330P331 to S330S331 (amino acid numbering with reference to the wildtype IgG2a sequence; see Eur. J. Immunol. (1999) 29:2613-2624); and the human light chain kappa constant region.

As disclosed herein but not claimed, the non-natural TrkC agonist is a human antibody selected from the group consisting of 6.1.2 (PTA-2148), 6.4.1 (PTA-2150), 2345 (PTA-2146), 2349 (PTA-2153), 2.5.1 (PTA-2151) and 2344 (PTA-2144). As disclosed herein but not claimed, the non-natural TrkC agonist is a murine antibody selected from a group consisting of 2248 (PTA-2147), 2250 (PTA-2149), 2253 (PTA-2145) and 2256 (PTA-2152). The antibodies 6.1.2 (PTA-2148), 6.4.1 (PTA-2150), 2345 (PTA-2146), 2349 (PTA-2153), 2.5.1 (PTA-2151), 2344 (PTA-2144), of 2248 (PTA-2147), 2250 (PTA-2149), 2253 (PTA-2145), and 2256 (PTA-2152) correspond to the antibodies 6.1.2 (PTA-2148), 6.4.1 (PTA-2150), 2345 (PTA-2146), 2349 (PTA-2153), 2.5.1 (PTA-2151), 2344 (PTA-2144), of 2248 (PTA-2147), 2250 (PTA-2149), 2253 (PTA-2145), and 2256 (PTA-2152) described in U.S. Patent Number 7384632.As disclosed herein but not claimed, the non-natural TrkC agonist is a human or murine antibody selected from the group consisting of 6.1.2 (PTA-2148), 6.4.1 (PTA-2150), 2345 (PTA-2146), 2349 (PTA-2153), 2.5.1 (PTA-2151), 2344 (PTA-2144), of 2248 (PTA-2147), 2250 (PTA-2149), 2253 (PTA-2145), and 2256 (PTA-2152), which recognizes and binds to an epitope on the D5 domain of TrkC receptor. As disclosed herein but not claimed, the non-natural TrkC agonist is a human or murine antibody selected from the group consisting of 6.1.2 (PTA-2148), 6.4.1 (PTA-2150), 2345 (PTA-2146), 2349 (PTA-2153), 2.5.1 (PTA-2151), 2344 (PTA-2144), of 2248 (PTA-2147), 2250 (PTA-2149), 2253 (PTA-2145), and 2256 (PTA-2152), which does not recognize or bind to any epitope on the TrkA or TrkB receptors. As disclosed herein but not claimed, the non-natural TrkC agonist is a human or murine antibody selected from the group consisting of 6.1.2 (PTA-2148), 6.4.1 (PTA-2150), 2345 (PTA-2146), 2349 (PTA-2153), 2.5.1 (PTA-2151), 2344 (PTA-2144), of 2248 (PTA-2147), 2250 (PTA-2149), 2253 (PTA-2145), and 2256 (PTA-2152), which recognizes and binds epitopes on the D5 and D4 domains of TrkC receptor.

As disclosed herein but not claimed, the non-natural TrkC agonist is an antibody or a binding fragment thereof comprising complementarity-determining regions (CDRs) of antibodies selected from 2B7, A5, E2, 6.1.2, 6.4.1, 2345, 2349, 2.5.1, 2344, 2345, 2248, 2349, 2250, 2253, and 2256. In some instances, the CDRs comprise heavy chain CDR1, CDR2, and CDR3 and/or light chain CDR1, CDR2, and CDR3 as illustrated in Table 2. In some instances, the CDRs comprise at least 80%, 85%, 90%, 91%, 92%, 93%, 94%, 95%, 96%, 97%, 98%, 99%, or 100% sequence identity to a CDR selected from SEQ ID NOs: 2-13 and 38-73. In some instances, the CDRs comprise at least 80% sequence identity to a CDR selected from SEQ ID NOs: 2-13 and 38-73. In some instances, the CDRs comprise at least 85% sequence identity to a CDR selected from SEQ ID NOs: 2-13 and 38-73. In some instances, the CDRs comprise at least 90% sequence identity to a CDR selected from SEQ ID NOs: 2-13 and 38-73. In some instances, the CDRs comprise at least 95% sequence identity to a CDR selected from SEQ ID NOs: 2-13 and 38-73. In some instances, the CDRs comprise at least 96% sequence identity to a CDR selected from SEQ ID NOs: 2-13 and 38-73. In some instances, the CDRs comprise at least 97% sequence identity to a CDR selected from SEQ ID NOs: 2-13 and 38-73. In some instances, the CDRs comprise at least 98% sequence identity to a CDR selected from SEQ ID NOs: 2-13 and 38-73. In some instances, the CDRs are selected from SEQ ID NOs: 2-13 and 38-73. In some instances, the CDRs comprise at least 99% sequence identity to a CDR selected from SEQ ID NOs: 2-13 and 38-73. In some instances, the CDRs are selected from SEQ ID NOs: 2-13 and 38-73.

As disclosed herein but not claimed, binding of a non-natural TrkC agonist antibody to TrkC results in increased

levels of phosphorylated TrkC, phosphorylated Akt, phosphorylated Erk, and phosphorylated phospholipase C-$\gamma$. As disclosed herein but not claimed, binding of a non-natural TrkC agonist to TrkC receptor leads to improved neuronal survival. As disclosed herein but not claimed, administration of an otic composition comprising a non-natural TrkC agonist that binds to TrkC receptor leads to improved neuronal survival and treats or prevents an otic condition. As disclosed herein but not claimed, administration of an otic composition comprising a non-natural TrkC agonist that binds to TrkC receptor leads to improved neuronal survival and treats or prevents an otic condition that requires reconnection of afferent sensory fibers and repair of ribbon synapses. As disclosed herein but not claimed, administration of an otic composition comprising a non-natural TrkC agonist that binds to TrkC receptor treats or prevents presbycusis (age related hearing loss). As disclosed herein but not claimed, administration of an otic composition comprising a non-natural TrkC agonist that binds to TrkC receptor leads to improved neuronal survival and treats sensorineural hearing loss. As disclosed herein but not claimed, the binding affinity of a TrkC agonist to TrkC receptor is about 0.10 to about 0.80 nM, about 0.15 to about 0.75 nM and about 0.18 to about 0.72 nM, about 1 nM to about 1.5 nM, about 2 nM to about 5 nM, about 10 nM to about 20 nM, about 30 nM to about 50 nM, about 75 nM to about 100 nM, about 125 nM to about 150 nM, about 160 nM to about 200 nM. As disclosed herein but not claimed, the binding affinity is about 2 pM, about 5 pM, about 10 pM, about 15 pM, about 20 pM, about 40 pM, or greater than about 40 pM. As disclosed herein but not claimed, the binding affinity is between about 2 pM and 22 pM. As disclosed herein but not claimed, the binding affinity is less than about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. As disclosed herein but not claimed, the binding affinity is about 10 nM. As disclosed herein but not claimed, the binding affinity is less than about 10 nM. In other embodiments, the binding affinity is about 0.1 nM or about 0.07 nM. In other embodiments, the binding affinity is less than about 0.1 nM or less than about 0.07 nM. As disclosed herein but not claimed, the binding affinity is any of about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM to any of about 2 pM, about 5 pM, about 10 pM, about 15 pM, about 20 pM, or about 40 pM. As disclosed herein but not claimed, the binding affinity is any of about 10 nM, about 5 nM, about 1 nM, about 900 pM, about 800 pM, about 700 pM, about 600 pM, about 500 pM, about 400 pM, about 300 pM, about 200 pM, about 150 pM, about 100 pM, about 90 pM, about 80 pM, about 70 pM, about 60 pM, about 50 pM, about 40 pM, about 30 pM, about 10 pM. As disclosed herein but not claimed, the binding affinity is about 2 pM, about 5 pM, about 10 pM, about 15 pM, about 20 pM, about 40 pM, or greater than about 40 pM. As disclosed herein but not claimed, the binding affinity falls within any range bound by any of these values, for example, between about 175 nM and about 180 nM. As disclosed herein but not claimed, the binding affinity is 100 nM. As disclosed herein but not claimed, the binding affinity is 200 nM.

disclosed herein but not claimed, the off-rate (or $k_{off}$) of a TrkC agonist to TrkC receptor is between about $10^{-1}$ and about $10^{-6}$ $s^{-1}$. As disclosed herein but not claimed, the off-rate (or $k_{off}$) of a TrkC agonist to TrkC receptor is between about $10^{-2}$ and about $10^{-6}$ $s^{-1}$, about $10^{-3}$ and about $10^{-6}$ $s^{-1}$, about $10^{-4}$ and about $10^{-6}$ $s^{-1}$, about $10^{-2}$ and about $10^{-5}$ $s^{-1}$, about $10^{-2}$ and about $10^{-4}$ $s^{-1}$, about $10^{-2}$ and about $10^{-3}$ $s^{-1}$, about $10^{-3}$ and about $10^{-5}$ $s^{-1}$, about $10^{-3}$ and about $10^{-4}$ $s^{-1}$, about $10^{-4}$ and about $10^{-5}$ $s^{-1}$, about $10^{-1}$ and about $10^{-5}$ $s^{-1}$, about $10^{-1}$ and about $10^{-4}$ $s^{-1}$, about $10^{-1}$ and about $10^{-3}$ $s^{-1}$, or about $10^{-1}$ and about $10^{-2}$ $s^{-1}$. As disclosed herein but not claimed, the off-rate (or $k_{off}$) of a TrkC agonist to TrkC receptor is about $10^{-1}$ $s^{-1}$, about $10^{-2}$ $s^{-1}$, about $10^{-3}$ $s^{-1}$, about $10^{-4}$ $s^{-1}$, about $10^{-5}$ $s^{-1}$, or about $10^{-6}$ $s^{-1}$.

In some instances, the binding affinity (or $K_A$) is calculated as:

$$K_A = ([\text{Ab-receptor}]/([\text{Ab}]*[\text{receptor}])) = 1/K_D$$

in which Ab-receptor is the antibody-receptor conjugate, Ab is the TrkC/TrkB agonist, and receptor is the TrkC/TrkB receptor. In some cases, $K_D$ (or the equilibrium dissociation constant) is calculated as a ratio of $k_{off}/k_{on}$.

As disclosed herein , the binding affinity is determined by one or more techniques well-known in the art. Suitable techniques include, e.g., surface plasmon resonance (Biacore3000™ surface plasmon resonance (SPR) system, Biacore, Inc.) equipped with pre-immobilized streptavidin sensor chips, which allows determination of the rate constants for binding ($k_a$) and dissociation ($k_d$) of an agonist to a TrkC receptor; isothermal titration calorimetry (ITC); Octet® (ForteBio), KinExA® (Kinetic Exclusion Assay, KinExA 3000, Sapidyne Instruments, Inc.), flow cytometry and ELISA.

As disclosed herein , the antibodies described herein are produced by hybridoma strains as outlined in Table 1.

**Table 1: Hybridoma strains for producing TrkB or TrkC agonists**

| Antibody | Antibody ATCC Deposit Number |
|---|---|
| 38B8 | PTA-8766 |

(continued)

| Antibody | Antibody ATCC Deposit Number |
|---|---|
| 2B7 | 090310-02 |
| A5, light chain | PTA-5682 |
| A5, heavy chain | PTA-5683 |
| 6.1.2 | PTA-2148 |
| 6.4.1 | PTA-2150 |
| 2345 | PTA-2146 |
| 2349 | PTA-2153 |
| 2.5.1 | PTA-2151 |
| 2344 | PTA-2144 |
| 2248 | PTA-2147 |
| 2250 | PTA-2149 |
| 2253 | PTA-2145 |
| 2256 | PTA-2152 |

The antibodies described herein have amino acid sequences as listed in Table 2

**Table 2: SEQ ID NOs. corresponding to TrkB or TrkC agonist antibodies and their binding fragments thereof**

| SEQ ID NO. | Description |
|---|---|
| 1 | ESTDNFILFDEVSPTPPI, binding epitope for antibody 2B7, on TrkC receptor |
| 2 | a CDR1 of antibody A5 (M1) of the formula GYTFTSYXaaXaaH, wherein Xaa at position 8 is R or W, and Xaa at position 9 is I, L, R, or M |
| 3 | a CDR2 of antibody A5 (M1) of the formula EIYPSNXaaRTNYNEKFXaaS, wherein Xaa at position 7 is A, T, S, or G; and Xaa at position 16 is K or E |
| 4 | a CDR3 of antibody A5 (M1) of the formula KYYYGNXaaXaaRSWYFDV, wherein Xaa at position 7 is T or S; wherein Xaa at position 8 is R, Q, K, S, or Y |
| 5 | GYTFTSYWMH, a CDR of antibody A5 (M1) |
| 6 | EIYPSNGRTNYNEKFK, a CDR of antibody A5 (M1) |
| 7 | KYYYGNSYRSWYFDV, a CDR of antibody A5 (M1) |
| 8 | CDR1 of light chain of human antibody 6.4.1 (M2) KSSQSVSYSSNNKNYLA |
| 9 | CDR2 of light chain of human antibody 6.4.1 (M2) WASTRES |
| 10 | CDR3 of light chain of human antibody 6.4.1 (M2) QQHYNTPLT |
| 11 | CDR1 of heavy chain of human antibody 6.4.1 (M2) ISTYYWN |
| 12 | CDR2 of heavy chain of human antibody 6.4.1 (M2) RIYTSGSTNYNPSLKS |
| 13 | CDR3 of heavy chain of human antibody 6.4.1 (M2) DGGYSNPFD |

(continued)

| SEQ ID NO. | Description |
|---|---|
| 14 | CDR1 of light chain of antibody C2 (M3)<br>RTSENVYSNLA |
| 15 | CDR2 of light chain of antibody C2 (M3)<br>AASNLQS |
| 16 | CDR3 of light chain of antibody C2 (M3)<br>QHFWGSPFT |
| 17 | CDR1 of heavy chain of antibody C2 (M3)<br>NYDII |
| 18 | CDR2 of heavy chain of antibody C2 (M3)<br>PYNDGT |
| 19 | CDR3 of heavy chain of antibody C2 (M3)<br>LLKYRRFRYYAIDY |
| 20 | CDR1 of light chain of antibody TAM-163<br>RASQTISNNLH |
| 21 | CDR2 of light chain of antibody TAM-163<br>SASLAIS |
| 22 | CDR3 of light chain of antibody TAM-163<br>QQSNSWPNT |
| 23 | CDR1 of heavy chain of antibody TAM-163<br>GYSFTAYFMN |
| 24 | CDR2 of heavy chain of antibody TAM-163<br>RINPNNGDTFYTQKFKG |
| 25 | CDR3 of heavy chain of antibody TAM-163<br>RDYFGAMDY |
| 26 | CDR1 of light chain of antibody A10 (M4)<br>RSSQSLVHSNGNTYLH |
| 27 | CDR2 of light chain of antibody A10 (M4)<br>KVSNRFS |
| 28 | CDR3 of light chain of antibody A10 (M4)<br>SQGTHVPYT |
| 29 | CDR1 of heavy chain of antibody A10 (M4)<br>DYEMH |
| 30 | CDR2 of heavy chain of antibody A10 (M4)<br>TIDPETAGTAYNQKFKG |
| 31 | CDR3 of heavy chain of antibody A10 (M4)<br>VTTWFAY |
| 32 | CDR1 of light chain of antibody C20 (M5)<br>RSSQSLIHSNGNTYLH |
| 33 | CDR2 of light chain of antibody C20 (M5)<br>KVSNRFS |
| 34 | CDR3 of light chain of antibody C20 (M5)<br>SQSTHVPFT |

(continued)

| SEQ ID NO. | Description |
|---|---|
| 35 | CDR1 of heavy chain of antibody C20 (M5)<br>SYDIN |
| 36 | CDR2 of heavy chain of antibody C20 (M5)<br>WIYPRDGSIKFNEKFKG |
| 37 | CDR3 of heavy chain of antibody C20 (M5)<br>RGRLLLYGFAY |
| 38 | CDR1 of light chain of murine antibody 2250<br>RASKSVSTSGYSYMH |
| 39 | CDR2 of light chain of murine antibody 2250<br>LVSNLES |
| 40 | CDR3 of light chain of murine antibody 2250<br>QHIRELTRS |
| 41 | CDR1 of heavy chain of murine antibody 2250<br>FWIEWVK |
| 42 | CDR2 of heavy chain of murine antibody 2250<br>EILPGSDNTNYNEKFKG |
| 43 | CDR3 of heavy chain of murine antibody 2250<br>KNRNYYGNYVV |
| 44 | CDR1 of light chain of murine antibody 2253<br>SASSSVSYMY |
| 45 | CDR2 of light chain of murine antibody 2253<br>STSNLAS |
| 46 | CDR3 of light chain of murine antibody 2253<br>QQRSSYPLT |
| 47 | CDR1 of heavy chain of murine antibody 2253<br>FWIEWVK |
| 48 | CDR2 of heavy chain of murine antibody 2253<br>EILPGSDNTNYNEKFKG |
| 49 | CDR3 of heavy chain of murine antibody 2253<br>KNRNYYGNYVV |
| 50 | CDR1 of light chain of murine antibody 2256 and its variants<br>RASESVXaaDXaaYGISFXaaXaa, wherein Xaa at position 7 is V or I, Xaa at position 9 is N or S, Xaa at position 15 is M or L, and Xaa at position 16 is N, T or A. |
| 51 | CDR2 of light chain of murine antibody 2256 and its variants<br>AASNXaaGS, wherein Xaa at position 5 is Q, L or R. |
| 52 | CDR3 of light chain of murine antibody 2256 and its variants<br>QQSKXaaVPRT, wherein Xaa at position 5 is E or T. |
| 53 | CDR1 of heavy chain of murine antibody 2256 and its variants |
| | YXaaXaaHWVK, where Xaa at position 2 is W or M, and Xaa at position 3 is M, I or L |
| 54 | CDR2 of heavy chain of murine antibody 2256 and its variants<br>EIYPSNXaaRTNYNEKFXaaS, wherein Xaa at position 7 is G, S, A or T, and Xaa at position 16 is K or E. |

(continued)

| SEQ ID NO. | Description |
|---|---|
| 55 | CDR3 of heavy chain of murine antibody 2256 and its variants KYYYGNXaaXaaRSWYFDV, wherein Xaa at position 7 is S or T, and Xaa at position 8 is Y or R. |
| 56 | CDR1 of light chain of human antibody 2345 RASQSVSSNYLT |
| 57 | CDR2 of light chain of human antibody 2345 GASSRAT |
| 58 | CDR3 of light chain of human antibody 2345 QQYGRSPPIT |
| 59 | CDR1 of heavy chain of human antibody 2345 SGGYYWS |
| 60 | CDR2 of heavy chain of human antibody 2345 YIFYSGRTYYNPSLKS |
| 61 | CDR3 of heavy chain of human antibody 2345 ERIAAAGADYYYNGLDV |
| 62 | CDR1 of light chain of human antibody 2349 RASQSGSSTYLA |
| 63 | CDR2 of light chain of human antibody 2349 GASSRAT |
| 64 | CDR3 of light chain of human antibody 2349 QQYGRSPPIT |
| 65 | CDR1 of heavy chain of human antibody 2349 SGYYYWS |
| 66 | CDR2 of heavy chain of human antibody 2349 YIYYSGSTYYNPSLKS |
| 67 | CDR3 of heavy chain of human antibody 2349 ERIAAAGTDYYYNGLAV |
| 68 | CDR1 of light chain of human antibody 6.1.2 RASQGIRNDLG |
| 69 | CDR2 of light chain of human antibody 6.1.2 AASSLQS |
| 70 | CDR3 of light chain of human antibody 6.1.2 LQHNSLPLT |
| 71 | CDR1 of heavy chain of human antibody 6.1.2 SGGYYWS |
| 72 | CDR2 of heavy chain of human antibody 6.1.2 YIYYSGSTNYNPSLKS |
| 73 | CDR3 of heavy chain of human antibody 6.1.2 DRDYDSTGDYYSYYGMDV |
| 74 | CDR1 of light chain of antibody RN1026A RASENVYSNLA |
| 75 | CDR2 of light chain of antibody RN1026A AASNLQS |

(continued)

| SEQ ID NO. | Description |
|---|---|
| 76 | CDR3 of light chain of antibody RN1026A<br>QHFWGSPFT |
| 77 | CDR1 of heavy chain of antibody RN1026A<br>GYTFTNYDII |
| 78 | CDR2 of heavy chain of antibody RN1026A<br>YINPYNRRREYNEKF |
| 79 | CDR3 of heavy chain of antibody RN1026A<br>LLKYRRFRYYAIDY |
| 80 | CDR1 of light chain of antibody Tl-HuCl<br>RASENVYSNLA |
| 81 | CDR2 of light chain of antibody Tl-HuCl<br>AASNLAD |
| 82 | CDR3 of light chain of antibody Tl-HuCl<br>QHFWYSPFT |
| 83 | CDR1 of heavy chain of antibody Tl-HuCl<br>NYDII |
| 84 | CDR2 of heavy chain of antibody Tl-HuCl<br>PYNDGT |
| 85 | CDR3 of heavy chain of antibody Tl-HuCl<br>LLKYRRFSYYAIDY |
| 86 | CDR1 of light chain of antibody A2<br>RASENVYSNLA |
| 87 | CDR2 of light chain of antibody A2<br>AASNLQS |
| 88 | CDR3 of light chain of antibody A2<br>QHFWYS PWT |
| 89 | CDR1 of heavy chain of antibody A2<br>NYDII |
| 90 | CDR2 of heavy chain of antibody A2<br>PYNDGT |
| 91 | CDR3 of heavy chain of antibody A2<br>LLKYRRFRYYAIDY |
| 92 | CDR1 of light chain of antibody 4A6<br>HASENVYSNLA |
| 93 | CDR2 of light chain of antibody 4A6<br>AASNLQS |
| 94 | CDR3 of light chain of antibody 4A6<br>QHFWGSPFT |
| 95 | CDR1 of heavy chain of antibody 4A6<br>NYDII |
| 96 | CDR2 of heavy chain of antibody 4A6<br>PYNRRR |

(continued)

| SEQ ID NO. | Description |
|---|---|
| 97 | CDR3 of heavy chain of antibody 4A6<br>LLKYRRFRYYAIDY |
| 98 | CDR1 of light chain of antibody 4B12<br>RASEPVYSNVA |
| 99 | CDR2 of light chain of antibody 4B12<br>AASNLQS |
| 100 | CDR3 of light chain of antibody 4B12<br>QHFWGSPFT |
| 101 | CDR1 of heavy chain of antibody 4B12<br>NYDII |
| 102 | CDR2 of heavy chain of antibody 4B12<br>PYNGRR |
| 103 | CDR3 of heavy chain of antibody 4B12<br>LLKYRRFRYYAIDY |
| 104 | CDR1 of heavy chain of antibody TOA-1<br>AYFMN |
| 105 | CDR1 of light chain of antibody 6B72C5<br>CSLSSQHSTYTIE |
| 106 | CDR2 of light chain of antibody 6B72C5<br>LKKDGSH |
| 107 | CDR3 of light chain of antibody 6B72C5<br>CGVGDTIKEQFVYV |
| 108 | CDR1 of heavy chain of antibody 6B72C5<br>SGFNIKDTYMH |
| 109 | CDR2 of heavy chain of antibody 6B72C5<br>IDPAHNNIKYDPKFQGK |
| 110 | CDR3 of heavy chain of antibody 6B72C5<br>CTGSLGRGDYF |
| 111 | CDR1 of light chain of antibody 5G5D2B5<br>CRSSTGAVTTSNYAS |
| 112 | CDR2 of light chain of antibody 5G5D2B5<br>GGTNNRA |
| 113 | CDR3 of light chain of antibody 5G5D2B5<br>CALCYSNHLV |
| 114 | CDR1 of heavy chain of antibody 5G5D2B5<br>SGFTFSNYAMS |
| 115 | CDR2 of heavy chain of antibody 5G5D2B5<br>ISSGGSTYYPDSVKGR |
| 116 | CDR3 of heavy chain of antibody 5G5D2B5<br>CARGRGLRLRSYYYALDY |

**Neurotrophic Agents**

Described herein are otic compositions comprising a TrkB or TrkC agonist, wherein the agonist is a neurotrophic agent. As disclosed herein , the TrkB or TrkC agonist is a neurotrophic agent that selectively binds to TrkB receptor. As disclosed herein , the TrkB or TrkC agonist is a neurotrophic agent that does not bind to TrkA or TrkC receptors. As disclosed herein , the TrkB or TrkC agonist is a neurotrophic agent that does not bind to the neurotrophic receptor $p75^{NTR}$. As disclosed herein , the TrkB agonist is a neurotrophic agent that does not bind to the neurotrophic receptor $p75^{NTR}$.

As disclosed herein , a neurotrophic agent is an agent that promotes the growth of tissue and/or neurons and their processes and connections and/or hair cells of the auris. As disclosed herein , a neurotrophic agent is an agent that promotes the survival of neurons and their processes and connections and otic hair cells, and/or the growth of neurons and their processes and connections and otic hair cells. As disclosed herein , the neurotrophic agent which promotes the survival of otic hair cells is a growth factor. As disclosed herein , the growth factor is a neurotroph. In certain instances, neurotrophs are growth factors which prevent cell death, prevent cell damage, repair damaged neurons and their processes and connections and otic hair cells, and/or induce differentiation in progenitor cells. As disclosed herein , the neurotroph is brain-derived neurotrophic factor (BDNF), ciliary neurotrophic factor (CNTF), glial cell-line derived neurotrophic factor (GDNF), neurotrophin-3, neurotrophin-4, and/or combinations thereof. As disclosed herein , the growth factor is a fibroblast growth factor (FGF), an insulin-like growth factor (IGF), an epidermal growth factor (EGF), a platlet-derived growth factor (PGF) and/or agonists thereof. As disclosed herein , the growth factor is an agonist of the fibroblast growth factor (FGF) receptor, the insulin-like growth factor (IGF) receptor, the epidermal growth factor (EGF) receptor, and/or the platlet-derived growth factor. As disclosed herein , the growth factor is hepatocyte growth factor.

As disclosed herein , the neurotrophic agent is BDNF. As disclosed herein , the neurotrophic agent is GDNF. In certain instances, BDNF and GDNF are neurotrophic agents that promote the survival of existing neurons and their processes and connections (e.g. spiral ganglion neurons), and otic hair cells by repairing damaged cells, inhibiting the production of ROS, and/or inhibiting cell death. As disclosed herein , the neurotrophic agent also promotes the differentiation of neural and otic hair cell progenitors. Further, As disclosed herein , the neurotrophic agent protects the Cranial Nerve VIII from degeneration. As disclosed herein , the neurotrophic agent BDNF is administered in conjunction with fibroblast growth factor. In some cases, BDNF comprises a naturally occurring BDNF with one or more mutations or modifications (e.g., chemical modifications such as phosphorylation, incorporation of unnatural amino acids, biotinylation, cyclisation, and the like) in amino acid residues. In some cases, GDNF comprises a naturally occurring GDNF with one or more mutations or modifications (e.g., chemical modifications such as phosphorylation, incorporation of unnatural amino acids, biotinylation, cyclisation, and the like) in amino acid residues.

As disclosed herein , the neurotrophic agent is neurotrophin-3 (NT-3). As disclosed herein , neurotrophin-3 promotes the survival of existing neurons and their processes and connections and otic hair cells, and promotes the differentiation of neural and otic hair cell progenitors. Further, As disclosed herein , neurotrophin-3 protects the VIII nerve from degeneration.

As disclosed herein , the neurotrophic agent is a naturally occurring neurotrophic agent with one or more mutations or modifications (e.g., chemical modifications such as phosphorylation, incorporation of unnatural amino acids, biotinylation, cyclisation, and the like) in amino acid residues. In some instances, the neurotrophic agent is neurotrophin-3. In some instances, neurotrophin-3 has amino acid sequence:

YAEHKSHRGEYSVCDSESLWVTDKSSAIDIRGHQVTVLGEIKTGNSPVKQYFYETRC
KE
ARPVKNGCRGIDDKHWNSQCKTSQTYVRALTSENNKLVGWRWIRIDTSCVCALSRK
IGRT (SEQ ID NO: 117).

As disclosed herein , a naturally occurring neurotrophin-3 with one or more mutations in amino acid residues comprise one or more mutations at amino acid position 3, 4, 5, 6, 11, 15, 17, 19, 22, 23, 24, 25, 26, 28, 31, 33, 34, 36, 38, 40, 42, 43, 44, 45, 46, 47, 48, 49, 51, 54, 56, 59, 61, 63, 64, 65, 68, 71, 72, 73, 74, 76, 78, 80, 83, 87, 89, 91, 92, 93, 94, 95, 96, 97, 103, 105, 114, 115, or a combination thereof, in which the amino acid position is according to SEQ ID NO: 117.

As disclosed herein , a naturally occurring neurotrophin-3 with one or more mutations in amino acid residues comprise one or more mutations at amino acid residue E3, H4, K5, S6, Y11, D15, E17, L19, T22, D23, K24, S25, S26, 128, R31, H33, Q34, T36, L38, E40, R42, T43, G44, N45, S46, P47, V48, K49, Y51, E54, R56, E59, R61, V63, K64, N65, R68, D71, D72, K73, H74, N76, Q78, K80, Q83, R87, L89, S91, E92, N93, N94, K95, L96, V97, R103, D105, R114,

K115, or a combination thereof, in which the amino acid residue is according to SEQ ID NO: 117.

As disclosed herein , a naturally occurring neurotrophin-3 with one or more mutations in amino acid residues comprise one or more mutations: E3A, H4D, H4A/H7A/R8A/E10A, E3A/K5A/S6A, K5A, S6A, Y11A, D15A, E17A, L19A, E17A/L19A, T22Q, D23A, K24A, S25Q, S26K, S25K/S26Y, I28Q, R31A, H33A, R31A/H33A, Q34A, Q34E, T36E, L38E, E40A, R42A, T43A, R42A/T43A, G44A, N45A, S46A, P47A, V48A, K49A, N45A/S46A/K49A/Y51A, Y51A, Y51F, E54A, R56A, E59A, E59A/R61A, K58A/E59A, R61A, V63A, K64A, N65A, K64A/N65A, R68A, D71A, D71A/K73A/H74A, D71A/H74A, D72A, K73A, H74A, N76A, Q78A, K80A, Q83A, K80A/Q83A, R87M, L89E, S91M, S91E, S91A/E92A, E92A, N93A, N94A, N93A/N94A, K95A, L96A, V97E, R103A, R103M, R103K, D105A, R114A, K115A, R114A/K115A, or a combination thereof, in which the amino acid residue is according to SEQ ID NO: 117.

As disclosed herein , a naturally occurring neurotrophin-3 with one or more mutations in amino acid residues comprises a NGF-swap of YAEHKS (SEQ ID NO: 119) to SSSHPIF (SEQ ID NO: 120).

As disclosed herein , a naturally occurring neurotrophin-3 with one or more mutations comprises NT-$3_{(1-119)}$ or NT-$3_{(1-117)}$ as described in PCT Pub. No. WO9803546.

As disclosed herein , a naturally occurring neurotrophin-3 with one or more mutations comprises a NT-3 mutant described in Urfer, et al., "The binding epitopes of neurotrophin-3 to its receptors TrkC and gp75 and the design of a multifunctional human neurotrophin," EMBO 13(24): 5896-5909 (1994).

As disclosed herein , the neurotrophic agent is a pan-neurotrophin. In some instances, a pan-neurotrophin is a synthetic trophic factor engineered by combining one or more domains of nerve growth factor (NGF), brain-derived neurotrophic factor (BDNF), and neurotrophin 3 (NT-3). In some instances, a pan-neurotrophin is pan-neurotrophin 1 (PNT-1), described in Ilag, et al., "Pan-neurotrophin 1: A genetically engineered neurotrophic factor displaying multiple specificities in peripheral neurons in vitro and in vivo," PNAS 92: 607-611 (1995). In some cases, a pan-neurotrophin is a pan-neurotrophin described in Ibanez, et al, "An extended surface of binding to Trk tyrosine kinase receptors in NGF and BDNF allows the engineering of a multifunctional pan-neurotrophin," EMBO 12(6): 2281-2293 (1993).

As disclosed herein , the neurotrophic agent is a chimeric neurotrophic agent. In some cases, a chimeric neurotrophic agent comprises, for example, one or more domains of nerve growth factor (NGF) and one or more domains of brain-derived neurotrophic factor (BDNF). In some cases, a neurotrophic agent is a chimeric neurotrophic agent described in Ibanez, et al., "Chimeric molecules with multiple neurotrophic activities reveal structural elements determining the specificities of NGF and BDNF," EMBO 10(8): 2105-2110 (1991).

As disclosed herein , the neurotrophic agent is CNTF. As disclosed herein , CNTF promotes the synthesis of neurotransmitters and the growth of neurites. As disclosed herein , CNTF is administered in conjunction with BDNF. In some cases, CNTF comprises a naturally occurring CNTF with one or more mutations or modifications (e.g., chemical modifications such as phosphorylation, incorporation of unnatural amino acids, biotinylation, cyclisation, and the like) in amino acid residues.

As disclosed herein , the neurotrophic agent is GDNF. Further, As disclosed herein , cells treated with exogenous GDNF have higher survival rates after trauma than untreated cells.

As disclosed herein , the neurotrophic agent is an epidermal growth factor (EGF). As disclosed herein , the EGF is heregulin (HRG). As disclosed herein , HRG stimulates the proliferation of utricular sensory epithelium. As disclosed herein , HRG-binding receptors are found in the vestibular and auditory sensory epithelium. In some cases, an epidermal growth factor (e.g., heregulin) comprises a naturally occurring epidermal growth factor (e.g., heregulin) with one or more mutations or modifications (e.g., chemical modifications such as phosphorylation, incorporation of unnatural amino acids, biotinylation, cyclisation, and the like) in amino acid residues.

As disclosed herein , the neurotrophic agent is an insulin-like growth factor (IGF). As disclosed herein , the IGF is IGF-1. As disclosed herein , the IGF-1 is mecasermin. As disclosed herein , IGF-1 attenuates the damage induced by exposure to an aminoglycoside. As disclosed herein , IGF-1 stimulates the differentiation and/or maturation of cochlear ganglion cells. In some cases, an insulin-like growth factor (e.g., IGF-1) comprises a naturally occurring insulin-like growth factor (e.g., IGF-1) with one or more mutations or modifications (e.g., chemical modifications such as phosphorylation, incorporation of unnatural amino acids, biotinylation, cyclisation, and the like) in amino acid residues.

As disclosed herein , the FGF receptor agonist is FGF-2. As disclosed herein , the IGF receptor agonist is IGF-1. Both the FGF and IGF receptors are found in the cells comprising the utricle epithelium.

As disclosed herein , the neurotrophic agent is hepatocyte growth factor (HGF). As disclosed herein , HGF protects cochlear hair cells from noise-induced damage and reduces noise-exposure-caused ABR threshold shifts. In some cases, a hepatocyte growth factor comprises a naturally occurring hepatocyte growth factor with one or more mutations or modifications (e.g., chemical modifications such as phosphorylation, incorporation of unnatural amino acids, biotinylation, cyclisation, and the like) in amino acid residues.

As disclosed herein , the neurotrophic agents are selected from Erythropoietin (EPO), Granulocyte-colony stimulating factor (G-CSF), Granulocyte-macrophage colony stimulating factor (GM-CSF), Growth differentiation factor-9 (GDF9), Insulin-like growth factor (IGF), Myostatin (GDF-8), Platelet-derived growth factor (PDGF), Thrombopoietin (TPO), Transforming growth factor alpha (TGF-$\alpha$), Transforming growth factor beta (TGF-$\beta$), Vascular endothelial growth

factor (VEGF) or combinations thereof. In some cases, the neurotrophic agents selected from Erythropoietin (EPO), Granulocyte-colony stimulating factor (G-CSF), Granulocyte-macrophage colony stimulating factor (GM-CSF), Growth differentiation factor-9 (GDF9), Insulin-like growth factor (IGF), Myostatin (GDF-8), Platelet-derived growth factor (PDGF), Thrombopoietin (TPO), Transforming growth factor alpha (TGF-$\alpha$), Transforming growth factor beta (TGF-$\beta$), or Vascular endothelial growth factor (VEGF) comprise one or more mutations or modifications in amino acid residues.

As disclosed herein , the neurotrophic agents described herein are chemically modified analogs of naturally occurring neurotrophic agents. Exemplary chemical modifications include, but are not limited to, phosphorylation or sulfurylation at serine, threonine, or tyrosine residues, by incorporating unnatural amino acids, by incorporating heavy amino acids, by incorporating D-amino acids, by biotinylation, by cyclisations, by acylation, by dimethylation, by amidation, by derivatization, by conjugation to carrier proteins, or by branching of peptide.

As disclosed herein , administration of the otic composition comprising a neurotrophic agent as described herein ameliorates hearing loss or reduction resulting from destroyed, stunted, malfunctioning, damaged, fragile or missing hair cells in the inner ear. As disclosed herein , administration of the otic composition comprising a neurotrophic agent as described herein ameliorates hearing loss or reduction resulting from destroyed, stunted, malfunctioning, damaged, fragile or missing hair cells in the inner ear, wherein the neurotrophic agent is chemically modified. As disclosed herein , the hearing loss is sensorineural hearing loss.

As disclosed herein , one or more of the neurotropic agents described herein are produced, for example, in a host cell system or a cell-free system. As disclosed herein , one or more of the neurotropic agents described herein are produced recombinantly through a host cell system. In some instances, the host cell is a eukaryotic cell (e.g., mammalian cell, insect cells, yeast cells or plant cell) or a prokaryotic cell (e.g., gram-positive bacterium or a gram-negative bacterium).

As disclosed herein , a eukaryotic host cell is a mammalian host cell. In some cases, a mammalian host cell is a stable cell line, or a cell line that has incorporated a genetic material of interest into its own genome and has the capability to express the product of the genetic material after many generations of cell division. In other cases, a mammalian host cell is a transient cell line, or a cell line that has not incorporated a genetic material of interest into its own genome and does not have the capability to express the product of the genetic material after many generations of cell division.

Exemplary mammalian host cells include 293T cell line, 293A cell line, 293FT cell line, 293F cells , 293 H cells, A549 cells, MDCK cells, CHO DG44 cells, CHO-S cells, CHO-K1 cells, Expi293F™ cells, Flp-In™ T-REx™ 293 cell line, Flp-In™-293 cell line, Flp-In™-3T3 cell line, Flp-In™-BHK cell line, Flp-In™-CHO cell line, Flp-In™-CV-1 cell line, Flp-In™-Jurkat cell line, FreeStyle™ 293-F cells, FreeStyle™ CHO-S cells, GripTite™ 293 MSR cell line, GS-CHO cell line, HepaRG™ cells, T-REx™ Jurkat cell line, Per.C6 cells, T-REx™-293 cell line, T-REx™-CHO cell line, and T-REx™-HeLa cell line.

As disclosed herein , a eukaryotic host cell is an insect host cell. Exemplary insect host cell include *Drosophila* S2 cells, Sf9 cells, Sf21 cells, High Five™ cells, and expresSF+® cells.

As disclosed herein , a eukaryotic host cell is a yeast host cell. Exemplary yeast host cells include *Pichiapastoris* yeast strains such as GS1 15, KM71H, SMD1168, SMD1168H, and X-33, and *Saccharomyces cerevisiae* yeast strain such as INVSc1.

As disclosed herein , a eukaryotic host cell is a plant host cell. In some instances, the plant cells comprise a cell from algae. Exemplary plant cell lines include strains from Chlamydomonas reinhardtii 137c, or Synechococcus elongatus PPC 7942.

As disclosed herein , a host cell is a prokaryotic host cell. Exemplary prokaryotic host cells include BL21, Mach1™, DH10B™, TOP10, DH5$\alpha$, DH10Bac™, OmniMax™, MegaX™, DH12S™, INV110, TOP10F', INV$\alpha$F, TOP10/P3, ccdB Survival, PIR1, PIR2, Stbl2™, Stbl3™, or Stbl4™.

some instances, suitable polynucleic acid molecules or vectors for the production of a neurotropic agent described herein include any suitable vectors derived from either a eukaryotic or prokaryotic sources. Exemplary polynucleic acid molecules or vectors include vectors from bacteria (e.g., *E. coli*), insects, yeast (e.g., *Pichia pastoris*), algae, or mammalian source. Bacterial vectors include, for example, pACYC177, pASK75, pBAD vector series, pBADM vector series, pET vector series, pETM vector series, pGEX vector series, pHAT, pHAT2, pMal-c2, pMal-p2, pQE vector series, pRSET A, pRSET B, pRSET C, pTrcHis2 series, pZA31-Luc, pZE21-MCS-1, pFLAG ATS, pFLAG CTS, pFLAG MAC, pFLAG Shift-12c, pTAC-MAT-1, pFLAG CTC, or pTAC-MAT-2.

Insect vectors include, for example, pFastBac1, pFastBac DUAL, pFastBac ET, pFastBac HTa, pFastBac HTb, pFastBac HTc, pFastBac M30a, pFastBact M30b, pFastBac, M30c, pVL1392, pVL1393, pVL1393 M10, pVL1393 M11, pVL1393 M12, FLAG vectors such as pPolh-FLAG1 or pPolh-MAT 2, or MAT vectors such as pPolh-MAT1, or pPolh-MAT2.

Yeast vectors include, for example, Gateway® pDEST™ 14 vector, Gateway®pDEST™ 15 vector, Gateway®pDEST™ 17 vector, Gateway®pDEST™ 24 vector, Gateway®pYES-DEST52 vector, pBAD-DEST49 Gateway®destination vector, pAO815 *Pichia* vector, pFLD1 *Pichi pastoris* vector, pGAPZA, B, & C *Pichia pastoris* vector, pPIC3.5K *Pichia* vector, pPIC6 A, B, & C *Pichia* vector, pPIC9K *Pichia* vector, pTEF 1/Zeo, pYES2 yeast vector, pYES2/CT yeast

vector, pYES2/NT A, B, & C yeast vector, or pYES3/CT yeast vector.

Algae vectors include, for example, pChlamy-4 vector or MCS vector.

Mammalian vectors include, for example, transient expression vectors or stable expression vectors. Exemplary mammalian transient expression vectors include p3xFLAG-CMV 8, pFLAG-Myc-CMV 19, pFLAG-Myc-CMV 23, pFLAG-CMV 2, pFLAG-CMV 6a,b,c, pFLAG-CMV 5.1, pFLAG-CMV 5a,b,c, p3xFLAG-CMV 7.1, pFLAG-CMV 20, p3xFLAG-Myc-CMV 24, pCMV-FLAG-MAT1, pCMV-FLAG-MAT2, pBICEP-CMV 3, or pBICEP-CMV 4. Exemplary mammalian stable expression vectors include pFLAG-CMV 3, p3xFLAG-CMV 9, p3xFLAG-CMV 13, pFLAG-Myc-CMV 21, p3xFLAG-Myc-CMV 25, pFLAG-CMV 4, p3xFLAG-CMV 10, p3xFLAG-CMV 14, pFLAG-Myc-CMV 22, p3xFLAG-Myc-CMV 26, pBICEP-CMV 1, or pBICEP-CMV 2.

In some instances, a cell-free system is used for the production of a neurotropic agent described herein. In some cases, a cell-free system comprises a mixture of cytoplasmic and/or nuclear components from a cell and is suitable for in vitro nucleic acid synthesis. In some instances, a cell-free system utilizes prokaryotic cell components. In other instances, a cell-free system utilizes eukaryotic cell components. Nucleic acid synthesis is obtained in a cell-free system based on, for example, Drosophila cell, Xenopus egg, or HeLa cells. Exemplary cell-free systems include E. coli S30 Extract system, E. coli T7 S30 system, or PURExpress®.

As disclosed herein , one or more neurotropic agents described herein are chemically synthesized. Exemplary synthesis techniques include, for example, solid phase technique developed by R. B. Merrified which permits the peptide to be built residue by residue from the carboxyl terminal amino acid to the amino terminal amino acid either manually or with an automated, commercially available synthesizer, and techniques described in Stewart, J. M. et al., Solid Phase Peptide Synthesis (Pierce Chemical Co., 2d ed., 1984), and Bodanszky, M. et al., The Practice of Peptide Synthesis (Springer-Verlag, 1984).

**Combination therapy**

As disclosed herein , otic composition or device described herein comprising TrkB or TrkC agonists, further comprises one or more active agents and/or a second therapeutic agent including but not limited to anti-emetic agents, antimicrobial agents, antioxidants, anti-septic agents or the like.

**Otic Surgery and Implants**

As disclosed herein , the otic formulations, compositions or devices described herein are used in combination with (e.g., implantation, short-term use, long-term use, or removal of) implants (e.g., cochlear implants). As used herein, implants include auris-interna or auris-media medical devices, examples of which include cochlear implants, hearing sparing devices, hearing-improvement devices, short electrodes, micro-prostheses or piston-like prostheses; needles; stem cell transplants; drug delivery devices; any cell-based therapeutic; or the like. In some instances, the implants are used in conjunction with a patient experiencing hearing loss. In some instances, the hearing loss is present at birth. In some instances, the hearing loss is associated with conditions such as AIED, bacterial meningitis or the like that lead to osteoneogenesis and/or nerve damage with rapid obliteration of cochlear structures and profound hearing loss.

In some instances, an implant is an immune cell or a stem cell transplant in the ear. In some instances, an implant is a small electronic device that has an external portion placed behind the ear, and a second portion that is surgically placed under the skin that helps provide a sense of sound to a person who is profoundly deaf or severely hard-of-hearing. By way of example, such cochlear medical device implants bypass damaged portions of the ear and directly stimulate the auditory nerve. In some instances cochlear implants are used in single sided deafness. In some instances cochlear implants are used for deafness in both ears.

As disclosed herein , administration of a TrkB or TrkC agonist composition or device described herein in combination with an otic intervention (e.g., an intratympanic injection, a stapedectomy, a medical device implant or a cell-based transplant) delays or prevents collateral damage to auris structures, e.g., irritation, cell damage, cell death, osteoneogeneis and/or further neuronal degeneration, caused by the external otic intervention (e.g., installation of an external device and/or cells in the ear). As disclosed herein , administration of a TrkB or TrkC agonist composition or device described herein in combination with an implant allows for a more effective restoration of hearing loss compared to an implant alone.

As disclosed herein , administration of a TrkB or TrkC agonist composition or device described herein reduces damage to cochlear structures caused by underlying conditions (e.g., bacterial meningitis, autoimmune ear disease (AIED)) allowing for successful cochlear device implantation. As disclosed herein , administration of a TrkB or TrkC agonist composition or device described herein, in conjunction with otic surgery, medical device implantation and/or cell transplantation, reduces or prevents cell damage and/or death (e.g., auris sensory hair cell death and/or damage) associated with otic surgery, medical device implantation and/or cell transplantation.

As disclosed herein , administration of a TrkB or TrkC agonist composition or device described herein (e.g., a

composition or device comprising a growth factor) in conjunction with a cochlear implant or stem cell transplant has a trophic effect (e.g., promotes healthy growth of cells and/or healing of tissue in the area of an implant or transplant). As disclosed herein , a trophic effect is desirable during otic surgery or during intratympanic injection procedures. As disclosed herein , a trophic effect is desirable after installation of a medical device or after a cell transplant. In some of such embodiments, the TrkB or TrkC agonist compositions or devices described herein are administered via direct cochlear injection, through a chochleostomy or via deposition on the round window.

As disclosed herein , administration of an anti-inflammatory or immunosuppressant composition (e.g., a composition comprising an immunosuppresant such as a corticosteroid) reduces inflammation and/or infections associated with otic surgery, implantation of a medical device or a cell transplant. In some instances, perfusion of a surgical area with an auris sensory cell modulator formulation described herein reduces or eliminates post-surgical and/or post-implantation complications (e.g., inflammation, hair cell damage, neuronal degeneration, osteoneogenesis or the like). In some instances, perfusion of a surgical area with a formulation described herein reduces post-surgery or post-implantation recuperation time. As disclosed herein , a medical device is coated with a composition described herein prior to implantation in the ear.

In one aspect, the formulations described herein, and modes of administration thereof, are applicable to methods of direct perfusion of the inner ear compartments. Thus, the formulations described herein are useful in combination with otic interventions. As disclosed herein , an otic intervention is an implantation procedure (e.g., implantation of a hearing device in the cochlea). As disclosed herein , an otic intervention is a surgical procedure including, by way of non-limiting examples, cochleostomy, labyrinthotomy, mastoidectomy, stapedectomy, stapedotomy, endolymphatic sacculotomy, tympanostomy or the like. As disclosed herein , the inner ear compartments are perfused with a formulation described herein prior to otic intervention, during otic intervention, or after otic intervention, or a combination thereof.

As disclosed herein , when perfusion is carried out in combination with otic intervention, the TrkB or TrkC agonist compositions are immediate release compositions. In some of such embodiments, the immediate release formulations described herein are non-thickened compositions and are substantially free of extended release components (e.g., gelling components such as polyoxyethylene-polyoxypropylene copolymers). In some of such embodiments, the compositions contain less than 5% of the extended release components (e.g., gelling components such as polyoxyethylene-polyoxypropylene triblock copolymers) by weight of the formulation. In some of such embodiments, the compositions contain less than 2% of the extended release components (e.g., gelling components such as polyoxyethylene-polyoxypropylene triblock copolymers) by weight of the formulation. In some of such embodiments, the compositions contain less than 1% of the extended release components (e.g., gelling components such as polyoxyethylene-polyoxypropylene triblock copolymers) by weight of the formulation. In some of such embodiments, a composition described herein that is used for perfusion of a surgical area contains substantially no gelling component and is an immediate release composition.

In other embodiments, a TrkB or TrkC agonist composition described herein is administered after an otic intervention (e.g., after implantation of a medical device or a cell-based therapeutic). In some of such embodiments, a TrkB or TrkC agonist composition described herein that is administered after the otic intervention is an intermediate release or extended release composition and contains gelling components as described herein.

**General Methods of Sterilization**

The environment of the inner ear is an isolated environment. The endolymph and the perilymph are static fluids and are not in contiguous contact with the circulatory system. The blood - labyrinth - barrier (BLB), which includes a blood-endolymph barrier and a blood-perilymph barrier, consists of tight junctions between specialized epithelial cells in the labyrinth spaces (i.e., the vestibular and cochlear spaces). The presence of the BLB limits delivery of active agents (e.g., TrkB or TrkC agonists) to the isolated microenvironment of the inner ear. Auris hair cells are bathed in endolymphatic or perilymphatic fluids and cochlear recycling of potassium ions is important for hair cell function. When the inner ear is infected, there is an influx of leukocytes and/or immunoglobins (e.g. in response to a microbial infection) into the endolymph and/or the perilymph and the delicate ionic composition of inner ear fluids is upset by the influx of leukocytes and/or immunoglobins. In certain instances, a change in the ionic composition of inner ear fluids results in hearing loss, loss of balance and/or ossification of auditory structures. In certain instances, even trace amounts of pyrogens and/or microbes can trigger infections and related physiological changes in the isolated microenvironment of the inner ear.

Disclosed herein but not claimed are auris formulations that are manufactured with low bioburden or sterilized with stringent sterilty requirements and are suitable for administration to the middle and/or inner ear. As disclosed herein , the auris compatible compositions described herein are substantially free of pyrogens and/or microbes. Disclosed herein but not claimed herein are otic compositions comprising TrkB or TrkC agonists that ameliorate or lessen otic disorders described herein. Further provided herein are methods comprising the administration of said otic compositions. As disclosed herein, the compositions or devices are sterilized. Included within the embodiments disclosed herein are means and processes for sterilization of a pharmaceutical composition or device disclosed herein for use in humans.

The goal is to provide a safe pharmaceutical product, relatively free of infection causing micro-organisms. The U. S. Food and Drug Administration has provided regulatory guidance in the publication "Guidance for Industry: Sterile Drug Products Produced by Aseptic Processing" available at:
http://www.fda.gov/cder/guidance/5882fnl.htm.As used herein, sterilization means a process used to destroy or remove microorganisms that are present in a product or packaging. Any suitable method available for sterilization of objects and compositions is used. Available methods for the inactivation of microorganisms include, but are not limited to, the application of extreme heat, lethal chemicals, or gamma radiation. Disclosed herein, is a process for the preparation of an otic therapeutic formulation comprising subjecting the formulation to a sterilization method selected from chemical sterilization, radiation sterilization or filtration sterilization. The method used depends largely upon the nature of the device or composition to be sterilized. Detailed descriptions of many methods of sterilization are given in Chapter 40 of Remington: The Science and Practice of Pharmacy published by Lippincott, Williams & Wilkins. Chemical Sterilization

Chemical sterilization methods are an alternative for products that do not withstand the extremes of heat sterilization. In this method, a variety of gases and vapors with germicidal properties, such as ethylene oxide, chlorine dioxide, formaldehyde or ozone are used as the anti-apoptotic agents. The germicidal activity of ethylene oxide, for example, arises from its ability to serve as a reactive alkylating agent. Thus, the sterilization process requires the ethylene oxide vapors to make direct contact with the product to be sterilized.

*Radiation Sterilization*

One advantage of radiation sterilization is the ability to sterilize many types of products without heat degradation or other damage. The radiation commonly employed is beta radiation or alternatively, gamma radiation from a $^{60}Co$ source. The penetrating ability of gamma radiation allows its use in the sterilization of many product types, including solutions, compositions and heterogeneous mixtures. The germicidal effects of irradiation arise from the interaction of gamma radiation with biological macromolecules. This interaction generates charged species and free radicals. Subsequent chemical reactions, such as rearrangements and cross-linking processes, result in the loss of normal function for these biological macromolecules. The formulations described herein are also optionally sterilized using beta irradiation. The formulations described herein, comprising non-natural TrkB or TrkC agonists are in the form of solution, and the solutions are sterilized using radiation sterilization methods.

*Filtration*

Filtration sterilization is a method used to remove but not destroy microorganisms from solutions. Membrane filters are used to filter heat-sensitive solutions. Such filters are thin, strong, homogenous polymers of mixed cellulosic esters (MCE), polyvinylidene fluoride (PVF; also known as PVDF), or polytetrafluoroethylene (PTFE) and have pore sizes ranging from 0.1 to 0.22 $\mu$m. Solutions of various characteristics are optionally filtered using different filter membranes. For example, PVF and PTFE membranes are well suited to filtering organic solvents while aqueous solutions are filtered through PVF or MCE membranes. Filter apparatus are available for use on many scales ranging from the single point-of-use disposable filter attached to a syringe up to commercial scale filters for use in manufacturing plants. The membrane filters are sterilized by autoclave or chemical sterilization. Validation of membrane filtration systems is performed following standardized protocols (Microbiological Evaluation of Filters for Sterilizing Liquids, Vol 4, No. 3. Washington, D.C: Health Industry Manufacturers Association, 1981) and involve challenging the membrane filter with a known quantity (ca. $10^7/cm^2$) of unusually small microorganisms, such as Brevundimonas diminuta (ATCC 19146). As disclosed herein , the formulations described herein, comprising non-natural TrkB or TrkC agonists are in the form of solution, and the solutions are sterilized using filtration methods.

Pharmaceutical compositions are optionally sterilized by passing through membrane filters. Formulations comprising nanoparticles (U.S. Pat No. 6,139,870) or multilamellar vesicles (Richard et al., International Journal of Pharmaceutics (2006), 312(1-2):144-50) are amenable to sterilization by filtration through 0.22 $\mu$m filters without destroying their organized structure. As disclosed herein , the formulations described herein, comprising non-natural TrkB or TrkC agonists are in the form of multilamellar vesicles, and the multilamellar vesicles are sterilized using filtration methods.

The methods disclosed herein comprise sterilizing the formulation (or components thereof) by means of filtration sterilization. As disclosed herein, auris-acceptable otic therapeutic agent formulation comprises a particle wherein the particle formulation is suitable for filtration sterilization. In a further embodiment said particle formulation comprises particles of less than 300 nm in size, of less than 200 nm in size, of less than 100 nm in size. In another embodiment the auris-acceptable formulation comprises a particle formulation wherein the sterility of the particle is ensured by sterile filtration of the precursor component solutions. In another embodiment the auris-acceptable formulation comprises a particle formulation wherein the sterility of the particle formulation is ensured by low temperature sterile filtration. In a further embodiment, low temperature sterile filtration is carried out at a temperature between 0 and 30 °C, between 0 and 20 °C, between 0 and 10 °C, between 10 and 20 °C, or between 20 and 30 °C.

Disclosed herein but not claimed is a process for the preparation of an auris-acceptable particle formulation comprising: filtering the aqueous solution containing the particle formulation at low temperature through a sterilization filter; lyophilizing the sterile solution; and reconstituting the particle formulation with sterile water prior to administration. A formulation described herein is manufactured as a suspension in a single vial formulation containing the micronized active pharmaceutical ingredient. A single vial formulation is prepared by aseptically mixing a sterile poloxamer solution with sterile micronized active ingredient (e.g., ketamine) and transferring the formulation to sterile pharmaceutical containers. As disclosed herein , a single vial containing a formulation described herein as a suspension is resuspended before dispensing and/or administration.

As disclosed herein but not claimed, , filtration and/or filling procedures are carried out at about 5°C below the gel temperature (Tgel) of a formulation described herein and with viscosity below a theoretical value of 100cP to allow for filtration in a reasonable time using a peristaltic pump.

As disclosed herein but not claimed the auris-acceptable otic therapeutic agent formulation comprises a nanoparticle formulation wherein the nanoparticle formulation is suitable for filtration sterilization. As disclosed herein the nanoparticle formulation comprises nanoparticles of less than 300 nm in size, of less than 200 nm in size, or of less than 100 nm in size. As disclosed herein, the auris-acceptable formulation comprises a microsphere formulation wherein the sterility of the microsphere is ensured by sterile filtration of the precursor organic solution and aqueous solutions. A disclosed herein, the auris-acceptable formulation comprises a thermoreversible gel formulation wherein the sterility of the gel formulation is ensured by low temperature sterile filtration. In a further embodiment, the low temperature sterile filtration occurs at a temperature between 0 and 30 °C, or between 0 and 20 °C, or between 0 and 10 °C, or between 10 and 20 °C, or between 20 and 30 °C. Disclosed herein but not claimed, is a process for the preparation of an auris-acceptable thermoreversible gel formulation comprising: filtering the aqueous solution containing the thermoreversible gel components at low temperature through a sterilization filter; lyophilizing the sterile solution; and reconstituting the thermoreversible gel formulation with sterile water prior to administration.

A disclosed herein, the active ingredients are dissolved in a suitable vehicle (e.g. a buffer) and sterilized separately (e.g. by heat treatment, filtration, gamma radiation). In some instances, the active ingredients are sterilized separately in a dry state. In some instances, the active ingredients are sterilized as a suspension or as a colloidal suspension. The remaining excipients (e.g., fluid gel components present in auris formulations) are sterilized in a separate step by a suitable method (e.g. filtration and/or irradiation of a cooled mixture of excipients); the two solutions that are separately sterilized are then mixed aseptically to provide a final auris formulation. In some instances, the final aseptic mixing is performed just prior to administration of a formulation described herein.

In some instances, conventionally used methods of sterilization (e.g., heat treatment (e.g., in an autoclave), gamma irradiation, filtration) lead to irreversible degradation of polymeric components (e.g., thermosetting, gelling or mucoadhesive polymer components) and/or the active agent in the formulation. In some instances, sterilization of an auris formulation by filtration through membranes (e.g., 0.2 μM membranes) is not possible if the formulation comprises thixotropic polymers that gel during the process of filtration.

Accordingly, provided herein are methods for sterilization of auris formulations that prevent degradation of polymeric components (e.g., thermosetting and/or gelling and/or mucoadhesive polymer components) and/or the TrkB or TrkC agonist during the process of sterilization. As disclosed herein , degradation of the TrkB or TrkC agonist (e.g., antibody agonists described herein) is reduced or eliminated through the use of specific pH ranges for buffer components and specific proportions of gelling agents in the formulations. As disclosed herein , the choice of an appropriate gellling agent and/or thermosetting polymer allows for sterilization of formulations described herein by filtration. As disclosed herein , the use of an appropriate thermosetting polymer and an appropriate copolymer (e.g., a gellling agent) in combination with a specific pH range for the formulation allows for high temperature sterilization of formulations described with substantially no degradation of the therapeutic agent or the polymeric excipients. An advantage of the methods of sterilization provided herein is that, in certain instances, the formulations are subjected to terminal sterilization via autoclaving without any loss of the active agent and/or excipients and/or polymeric components during the sterilization step and are rendered substantially free of microbes and/or pyrogens.

*Microorganisms*

Provided herein are auris-acceptable compositions or devices that ameliorate or lessen otic disorders described herein. Further provided herein are methods comprising the administration of said otic compositions. As disclosed herein , the compositions or devices are substantially free of microorganisms. Acceptable bioburden or sterility levels are based on applicable standards that define therapeutically acceptable compositions, including but not limited to United States Pharmacopeia Chapters <1111> et seq. For example, acceptable sterility (e.g., bioburden) levels include about 10 colony forming units (cfu) per gram of formulation, about 50 cfu per gram of formulation, about 100 cfu per gram of formulation, about 500 cfu per gram of formulation or about 1000 cfu per gram of formulation. As disclosed herein , acceptable bioburden levels or sterility for formulations include less than 10 cfu/mL, less than 50 cfu/mL, less than 500 cfu/mL or

less than 1000 cfu/mL microbial agents. In addition, acceptable bioburden levels or sterility include the exclusion of specified objectionable microbiological agents. By way of example, specified objectionable microbiological agents include but are not limited to Escherichia coli (E. coli), Salmonella sp., Pseudomonas aeruginosa (P. aeruginosa) and/or other specific microbial agents.

Sterility of the auris-acceptable otic therapeutic agent formulation is confirmed through a sterility assurance program in accordance with United States Pharmacopeia Chapters <61>, <62> and <71>. A key component of the sterility assurance quality control, quality assurance and validation process is the method of sterility testing. Sterility testing, by way of example only, is performed by two methods. The first is direct inoculation wherein a sample of the composition to be tested is added to growth medium and incubated for a period of time up to 21 days. Turbidity of the growth medium indicates contamination. Drawbacks to this method include the small sampling size of bulk materials which reduces sensitivity, and detection of microorganism growth based on a visual observation. An alternative method is membrane filtration sterility testing. In this method, a volume of product is passed through a small membrane filter paper. The filter paper is then placed into media to promote the growth of microorganisms. This method has the advantage of greater sensitivity as the entire bulk product is sampled. The commercially available Millipore Steritest sterility testing system is optionally used for determinations by membrane filtration sterility testing. For the filtration testing of creams or ointments Steritest filter system No. TLHVSL210 are used. For the filtration testing of emulsions or viscous products Steritest filter system No. TLAREM210 or TDAREM210 are used. For the filtration testing of pre-filled syringes Steritest filter system No. TTHASY210 are used. For the filtration testing of material dispensed as an aerosol or foam Steritest filter system No. TTHVA210 are used. For the filtration testing of soluble powders in ampoules or vials Steritest filter system No. TTHADA210 or TTHADV210 are used.

Testing for E. coli and Salmonella includes the use of lactose broths incubated at 30 - 35 °C for 24-72 hours, incubation in MacConkey and/or EMB agars for 18-24 hours, and/or the use of Rappaport medium. Testing for the detection of P. aeruginosa includes the use of NAC agar. United States Pharmacopeia Chapter <62> further enumerates testing procedures for specified objectionable microorganisms.

A disclosed herein, any controlled release formulation described herein has less than about 60 colony forming units (CFU), less than about 50 colony forming units, less than about 40 colony forming units, or less than about 30 colony forming units of microbial agents per gram of formulation. A disclosed herein, the otic formulations described herein are formulated to be isotonic with the endolymph and/or the perilymph.

*Endotoxins*

Provided herein are otic compositions that ameliorate or lessen otic disorders described herein. Further provided herein are methods comprising the administration of said otic compositions. As disclosed herein , the compositions or devices are substantially free of endotoxins. An additional aspect of the sterilization process is the removal of by-products from the killing of microorganisms (hereinafter, "Product"). The process of depyrogenation removes pyrogens from the sample. Pyrogens are endotoxins or exotoxins which induce an immune response. An example of an endotoxin is the lipopolysaccharide (LPS) molecule found in the cell wall of gram-negative bacteria. While sterilization procedures such as autoclaving or treatment with ethylene oxide kill the bacteria, the LPS residue induces a proinflammatory immune response, such as septic shock. Because the molecular size of endotoxins can vary widely, the presence of endotoxins is expressed in "endotoxin units" (EU). One EU is equivalent to 100 picograms of E. coli LPS. Humans can develop a response to as little as 5 EU/kg of body weight. The bioburden (e.g., microbial limit) and/or sterility (e.g., endotoxin level) is expressed in any units as recognized in the art. A disclosed herein, otic compositions described herein contain lower endotoxin levels (e.g. < 4 EU/kg of body weight of a subject) when compared to conventionally acceptable endotoxin levels (e.g., 5 EU/kg of body weight of a subject). As disclosed herein , the auris-acceptable otic therapeutic agent formulation has less than about 5 EU/kg of body weight of a subject. In other embodiments, the auris-acceptable otic therapeutic agent formulation has less than about 4 EU/kg of body weight of a subject. In additional embodiments, the auris-acceptable otic therapeutic agent formulation has less than about 3 EU/kg of body weight of a subject. In additional embodiments, the auris-acceptable otic therapeutic agent formulation has less than about 2 EU/kg of body weight of a subject.

As disclosed herein , the auris-acceptable otic therapeutic agent formulation or device has less than about 5 EU/kg of formulation. In other embodiments, the auris-acceptable otic therapeutic agent formulation has less than about 4 EU/kg of formulation. In additional embodiments, the auris-acceptable otic therapeutic agent formulation has less than about 3 EU/kg of formulation. As disclosed herein , the auris-acceptable otic therapeutic agent formulation has less than about 5 EU/kg Product. In other embodiments, the auris-acceptable otic therapeutic agent formulation has less than about 1 EU/kg Product. In additional embodiments, the auris-acceptable otic therapeutic agent formulation has less than about 0.2 EU/kg Product. As disclosed herein , the auris-acceptable otic therapeutic agent formulation has less than about 5 EU/g of unit or Product. In other embodiments, the auris-acceptable otic therapeutic agent formulation has less than about 4 EU/ g of unit or Product. In additional embodiments, the auris-acceptable otic therapeutic agent formulation

has less than about 3 EU/g of unit or Product. As disclosed herein , the auris-acceptable otic therapeutic agent formulation has less than about 5 EU/mg of unit or Product. In other embodiments, the auris-acceptable otic therapeutic agent formulation has less than about 4 EU/ mg of unit or Product. In additional embodiments, the auris-acceptable otic therapeutic agent formulation has less than about 3 EU/mg of unit or Product. A disclosed herein, otic compositions described herein contain from about 1 to about 5 EU/mL of formulation. A disclosed herein, otic compositions described herein contain from about 2 to about 5 EU/mL of formulation, from about 3 to about 5 EU/mL of formulation, or from about 4 to about 5 EU/mL of formulation.

A disclosed herein, otic compositions or devices described herein contain lower endotoxin levels (e.g. < 0.5 EU/mL of formulation) when compared to conventionally acceptable endotoxin levels (e.g., 0.5 EU/mL of formulation). As disclosed herein , the auris-acceptable otic therapeutic agent formulation or device has less than about 0.5 EU/mL of formulation. In other embodiments, the auris-acceptable otic therapeutic agent formulation has less than about 0.4 EU/mL of formulation. In additional embodiments, the auris-acceptable otic therapeutic agent formulation has less than about 0.2 EU/mL of formulation.

Pyrogen detection, by way of example only, is performed by several methods. Suitable tests for sterility include tests described in United States Pharmacopoeia (USP) <71> Sterility Tests (23rd edition, 1995). The rabbit pyrogen test and the Limulus amebocyte lysate test are both specified in the United States Pharmacopeia Chapters <85> and <151> (USP23/NF 18, Biological Tests, The United States Pharmacopeial Convention, Rockville, MD, 1995). Alternative pyrogen assays have been developed based upon the monocyte activation-cytokine assay. Uniform cell lines suitable for quality control applications have been developed and have demonstrated the ability to detect pyrogenicity in samples that have passed the rabbit pyrogen test and the Limulus amebocyte lysate test (Taktak et al, J. Pharm. Pharmacol. (1990), 43:578-82). In an additional embodiment, the auris-acceptable otic therapeutic agent formulation is subject to depyrogenation. In a further embodiment, the process for the manufacture of the auris-acceptable otic therapeutic agent formulation comprises testing the formulation for pyrogenicity. A disclosed herein, the formulations described herein are substantially free of pyrogens.

**pH and Practical Osmolarity**

As used herein, "practical osmolarity" means the osmolarity of a formulation that is measured by including the active agent and all excipients except the gelling and/or the thickening agent (e.g., polyoxyethylene-polyooxypropylene copolymers, carboxymethylcellulose or the like). The practical osmolarity of a formulation described herein is measured by any suitable method, e.g., a freezing point depression method as described in Viegas et. al., Int. J. Pharm., 1998, 160, 157-162. In some instances, the practical osmolarity of a composition described herein is measured by vapor pressure osmometry (e.g., vapor pressure depresssion method) that allows for determination of the osmolarity of a composition at higher temperatures. In some instances, vapor pressure depression method allows for determination of the osmolarity of a formulation comprising a gelling agent (e.g., a thermoreversible polymer) at a higher temperature wherein the gelling agent is in the form of a gel. The practical osmolality of an otic formulation described herein is from about 100 mOsm/kg to about 1000 mOsm/kg, from about 200 mOsm/kg to about 800 mOsm/kg, from about 250 mOsm/kg to about 500 mOsm/kg, or from about 250 mOsm/kg to about 320 mOsm/kg, or from about 250 mOsm/kg to about 350 mOsm/kg or from about 280 mOsm/kg to about 320 mOsm/kg. As disclosed herein , the formulations described herein have a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 250 mOsm/L to about 320 mOsm/L, or about 280 mOsm/L to about 320 mOsm/L.

As disclosed herein , the osmolarity at a target site of action (e.g., the perilymph) is about the same as the delivered osmolarity (i.e., osmolarity of materials that cross or penetrate the round window membrane) of any formulation described herein. As disclosed herein , the formulations described herein have a delieverable osmolarity of about 150 mOsm/L to about 500 mOsm/L, about 250 mOsm/L to about 500 mOsm/L, about 250 mOsm/L to about 350 mOsm/L, about 280 mOsm/L to about 370 mOsm/L or about 250 mOsm/L to about 320 mOsm/L.

The main cation present in the endolymph is potassium. In addition the endolymph has a high concentration of positively charged amino acids. The main cation present in the perilymph is sodium. In certain instances, the ionic composition of the endolymph and perilymph regulate the electrochemical impulses of hair cells. In certain instances, any change in the ionic balance of the endolymph or perilymph results in a loss of hearing due to changes in the conduction of electrochemical impulses along otic hair cells. As disclosed herein , a composition disclosed herein does not disrupt the ionic balance of the perilymph. As disclosed herein , a composition disclosed herein has an ionic balance that is the same as or substantially the same as the perilymph. As disclosed herein , a composition disclosed herein does not disrupt the ionic balance of the endolymph. As disclosed herein , a composition disclosed herein has an ionic balance that is the same as or substantially the same as the endolymph. As disclosed herein , an otic formulation described herein is formulated to provide an ionic balance that is compatible with inner ear fluids (e.g., endolymph and/or perilymph).

The endolymph and the perilymph have a pH that is close to the physiological pH of blood. The endolymph

has a pH range of about 7.2-7.9; the perilymph has a pH range of about 7.2 - 7.4. The *in situ* pH of the proximal endolymph is about 7.4 while the pH of distal endolymph is about 7.9.

As disclosed herein , the pH of a composition described herein is adjusted (e.g., by use of a buffer) to an endolymph-compatible pH range of about 5.5 to 9.0. In specific embodiments, the pH of a composition described herein is adjusted to a perilymph-suitable pH range of about 5.5 to about 9.0. As disclosed herein , the pH of a composition described herein is adjusted to a perilymph-suitable range of about 5.5 to about 8.0, about 6 to about 8.0 or about 6.6 to about 8.0. As disclosed herein , the pH of a composition described herein is adjusted to a perilymph-suitable pH range of about 7.0 - 7.6.

As disclosed herein , useful formulations also include one or more pH adjusting agents or buffering agents. Suitable pH adjusting agents or buffers include, but are not limited to acetate, bicarbonate, ammonium chloride, citrate, phosphate, pharmaceutically acceptable salts thereof and combinations or mixtures thereof.

As disclosed herein , when one or more buffers are utilized in the formulations of the present disclosure, they are combined, e.g., with a pharmaceutically acceptable vehicle and are present in the final formulation, e.g., in an amount ranging from about 0.1% to about 20%, from about 0.5% to about 10%. A disclosed herein of the present disclosure, the amount of buffer included in the gel formulations are an amount such that the pH of the gel formulation does not interfere with the body’s natural buffering system.

As disclosed herein , diluents are also used to stabilize compounds because they can provide a more stable environment. Salts dissolved in buffered solutions (which also can provide pH control or maintenance) are utilized as diluents in the art, including, but not limited to a phosphate buffered saline solution.

As disclosed herein , any gel formulation described herein has a pH that allows for sterilization (e.g, by filtration or aseptic mixing or heat treatment and/or autoclaving (e.g., terminal sterilization) of a gel formulation without degradation of the pharmaceutical agent (e.g., TrkB or TrkC agonist) or the polymers comprising the gel. In order to reduce hydrolysis and/or degradation of the otic agent and/or the gel polymer during sterilization, the buffer pH is designed to maintain pH of the formulation in the 7-8 range during the process of sterilization (e.g., high temperature autoclaving).

In specific embodiments, any gel formulation described herein has a pH that allows for terminal sterilization (e.g, by heat treatment and/or autoclaving) of a gel formulation without degradation of the pharmaceutical agent (e.g., TrkB or TrkC agonist) or the polymers comprising the gel. For example, in order to reduce hydrolysis and/or degradation of the otic agent and/or the gel polymer during autoclaving, the buffer pH is designed to maintain pH of the formulation in the 7-8 range at elevated temperatures. Any appropriate buffer is used depending on the otic agent used in the formulation. In some instances, since pKa of TRIS decreases as temperature increases at approximately -0.03/°C and pKa of PBS increases as temperature increases at approximately 0.003/°C, autoclaving at 250°F (121°C) results in a significant downward pH shift (i.e. more acidic) in the TRIS buffer whereas a relatively much less upward pH shift in the PBS buffer and therefore much increased hydrolysis and/or degradation of an otic agent in TRIS than in PBS. Degradation of an otic agent is reduced by the use of an appropriate combination of a buffer and polymeric additives (e.g. CMC) as described herein.

As disclosed herein , a formulation pH of between about 5.0 and about 9.0, between about 5.5 and about 8.5, between about 6.0 and about 7.6, between about 7 and about 7.8, between about 7.0 and about 7.6, between about 7.2 and 7.6, or between about 7.2 and about 7.4 is suitable for sterilization (e.g, by filtration or aseptic mixing or heat treatment and/or autoclaving (e.g., terminal sterilization)) of auris formulations described herein. In specific embodiments a formulation pH of about 6.0, about 6.5, about 7.0, about 7.1, about 7.2, about 7.3, about 7.4, about 7.5, or about 7.6 is suitable for sterilization (e.g, by filtration or aseptic mixing or heat treatment and/or autoclaving (e.g., terminal sterilization)) of any composition described herein.

As disclosed herein , the formulations have a pH as described herein, and include a thickening agent (e.g, a viscosity enhancing agent) such as, by way of non-limiting example, a cellulose based thickening agent described herein. In some instances, the addition of a secondary polymer (e.g., a thickening agent) and a pH of formulation as described herein, allows for sterilization of a formulation described herein without any substantial degradation of the otic agent and/or the polymer components in the otic formulation. As disclosed herein , the ratio of a thermoreversible poloxamer to a thickening agent in a formulation that has a pH as described herein, is about 40:1, about 35:1, about 30:1, about 25:1, about 20:1, about 15:1 about 10:1,or about 5:1. For example, A disclosed herein, a sustained and/or extended release formulation described herein comprises a combination of poloxamer 407 (pluronic F127) and carboxymethylcellulose (CMC) in a ratio of about 40:1, about 35:1, about 30:1, about 25:1, about 20:1, about 15:1, about 10:1 or about 5:1.

As disclosed herein , the amount of thermoreversible polymer in any formulation described herein is about 10%, about 15%, about 20%, about 25%, about 30%, about 35% or about 40% of the total weight of the formulation. As disclosed herein , the amount of thermoreversible polymer in any formulation described herein is about 10%, about 11%, about 12%, about 13%, about 14%, about 15%, about 16%, about 17%, about 18%, about 19%, about 20%, about 21%, about 22%, about 23%, about 24% or about 25% of the total weight of the formulation. As disclosed herein , the amount of thermoreversible polymer (e.g., pluronic F127) in any formulation described herein is about 7.5% of the total weight of the formulation. As disclosed herein , the amount of thermoreversible polymer (e.g., pluronic F127) in any formulation

described herein is about 10% of the total weight of the formulation. As disclosed herein , the amount of thermoreversible polymer (e.g., pluronic F127) in any formulation described herein is about 11% of the total weight of the formulation. As disclosed herein , the amount of thermoreversible polymer (e.g., pluronic F127) in any formulation described herein is about 12% of the total weight of the formulation. As disclosed herein , the amount of thermoreversible polymer (e.g., pluronic F127) in any formulation described herein is about 13% of the total weight of the formulation. As disclosed herein , the amount of thermoreversible polymer (e.g., pluronic F127) in any formulation described herein is about 14% of the total weight of the formulation. As disclosed herein , the amount of thermoreversible polymer (e.g., pluronic F127) in any formulation described herein is about 15% of the total weight of the formulation. As disclosed herein , the amount of thermoreversible polymer (e.g., pluronic F127) in any formulation described herein is about 16% of the total weight of the formulation. As disclosed herein , the amount of thermoreversible polymer (e.g., pluronic F127) in any formulation described herein is about 17% of the total weight of the formulation. As disclosed herein , the amount of thermoreversible polymer (e.g., pluronic F127) in any formulation described herein is about 18% of the total weight of the formulation. As disclosed herein , the amount of thermoreversible polymer (e.g., pluronic F127) in any formulation described herein is about 19% of the total weight of the formulation. As disclosed herein , the amount of thermoreversible polymer (e.g., pluronic F127) in any formulation described herein is about 20% of the total weight of the formulation. As disclosed herein , the amount of thermoreversible polymer (e.g., pluronic F127) in any formulation described herein is about 21% of the total weight of the formulation. As disclosed herein , the amount of thermoreversible polymer (e.g., pluronic F127) in any formulation described herein is about 23% of the total weight of the formulation. As disclosed herein , the amount of thermoreversible polymer (e.g., pluronic F127) in any formulation described herein is about 25% of the total weight of the formulation.

As disclosed herein , the amount of thickening agent (e.g., a gelling agent) in any formulation described herein is about 0.1%, about 0.2%, about 0.3%, about 0.4%, about 0.5%, about 0.6%, about 0.7%, about 0.8%, about 0.9%, about 1%, about 5%, about 10%, or about 15% of the total weight of the formulation. As disclosed herein , the amount of thickening agent (e.g., a gelling agent) in any formulation described herein is about 0.1%, 0.5%, about 1%, about 1.5%, about 2%, about 2.5%, about 3%, about 3.5%, about 4%, about 4.5%, or about 5% of the total weight of the formulation.

As disclosed herein , the pharmaceutical formulations described herein are stable with respect to pH over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 1 month, at least about 2 months, at least about 3 months, at least about 4 months, at least about 5 months, or at least about 6 months. In other embodiments, the formulations described herein are stable with respect to pH over a period of at least about 1 week. Also described herein are formulations that are stable with respect to pH over a period of at least about 1 month.

*Tonicity Agents*

In general, the endolymph has a higher osmolality than the perilymph. For example, the endolymph has an osmolality of about 304 mOsm/kg $H_2O$ while the perilymph has an osmolality of about 294 mOsm/kg $H_2O$. A disclosed herein, tonicity agents are added to the formulations described herein in an amount as to provide a practical osmolality of an otic formulation of about 100 mOsm/kg to about 1000 mOsm/kg, from about 200 mOsm/kg to about 800 mOsm/kg, from about 250 mOsm/kg to about 500 mOsm/kg, or from about 250 mOsm/kg to about 350 mOsm/kg or from about 280 mOsm/kg to about 320 mOsm/kg. As disclosed herein , the formulations described herein have a practical osmolarity of about 100 mOsm/L to about 1000 mOsm/L, about 200 mOsm/L to about 800 mOsm/L, about 250 mOsm/L to about 500 mOsm/L, about 250 mOsm/L to about 350 mOsm/L, about 280 mOsm/L to about 320 mOsm/L or about 250 mOsm/L to about 320 mOsm/L.

As disclosed herein , the deliverable osmolarity of any formulation described herein is designed to be isotonic with the targeted otic structure (e.g., endolymph, perilymph or the like). In specific embodiments, auris compositions described herein are formulated to provide a delivered perilymph-suitable osmolarity at the target site of action of about 250 to about 320 mOsm/L; and preferably about 270 to about 320 mOsm/L. In specific embodiments, auris compositions described herein are formulated to provide a delivered perilymph-suitable osmolality at the target site of action of about 250 to about 320 mOsm/kg $H_2O$; or an osmolality of about 270 to about 320 mOsm/kg $H_2O$. In specific embodiments, the deliverable osmolarity/osmolality of the formulations (i.e., the osmolarity/osmolality of the formulation in the absence of gelling or thickening agents (e.g., thermoreversible gel polymers) is adjusted, for example, by the use of appropriate salt concentrations (e.g., concentration of potassium or sodium salts) or the use of tonicity agents which renders the formulations endolymph-compatible and/or perilymph-compatible (i.e. isotonic with the endolymph and/or perilymph) upon delivery at the target site . The osmolarity of a formulation comprising a thermoreversible gel polymer is an unreliable measure due to the association of varying amounts of water with the monomeric units of the polymer. The practical

osmolarity of a formulation (i.e., osmolarity in the absence of a gelling or thickening agent (e.g. a thermoreversible gel polymer) is a reliable measure and is measured by any suitable method (e.g., freezing point depression method, vapor depression method). In some instances, the formulations described herein provide a deliverable osmolarity (e.g., at a target site (e.g., perilymph) that causes minimal disturbance to the environment of the inner ear and causes minimum discomfort (e.g., vertigo and/or nausea) to a mammal upon administration.

As disclosed herein , any formulation described herein is isotonic with the perilymph and/or endolymph. Isotonic formulations are provided by the addition of a tonicity agent. Suitable tonicity agents include, but are not limited to any pharmaceutically acceptable sugar, salt or any combinations or mixtures thereof, such as, but not limited to dextrose, glycerin, mannitol, sorbitol, sodium chloride, and other electrolytes.

Useful auris compositions include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

As disclosed herein , the formulations described herein have a pH and/or practical osmolarity as described herein, and have a concentration of active pharmaceutical ingredient between about 1 μM and about 10 μM, between about 1 mM and about 100 mM, between about 0.1 mM and about 100 mM, between about 0.1 mM and about 100 nM. As disclosed herein , the formulations described herein have a pH and/or practical osmolarity as described herein, and have a concentration of active pharmaceutical ingredient between about 0.001% - about 60%, between about 0.01% - about 20%, between about 0.01% - about 10%, between about 0.01% - about 7.5%, between about 0.01% - 6%, between about 0.01 - 5%, between about 0.1 - about 10%, or between about 0.1 - about 6% of the active ingredient by weight of the formulation. As disclosed herein , the formulations described herein have a pH and/or practical osmolarity as described herein, and have a concentration of active pharmaceutical ingredient between about 0.1 mg/mL and about 100 mg/mL, between about 1 mg/mL and about 100 mg/mL, between about 1 mg/mL and about 80 mg/mL, between about 1 mg/mL and about 60 mg/mL, between about 1 mg/mL and about 50 mg/mL, between about 1 mg/mL and about 50 mg/mL, between about 1 mg/mL and about 20 mg/mL, between about 1 mg/mL to about 10 mg/mL,-between about 1 mg/mL to about 5 mg/mL, or between about 0.5 mg/mL and about 5 mg/mL of the active agent by volume of the formulation. As disclosed herein , the formulations described herein have a pH and/or practical osmolarity as described herein, and have a concentration of active pharmaceutical ingredient between about 1 μg/mL and about 500 μg/mL, between about 1 μg/mL and about 250 μg/mL, between about 1 μg and about 100 μg/mL, between about 1 μg/mL and about 50 μg/mL, or between about 1 μg/mL and about 20 μg/mL of the active agent by volume of the formulation.

As disclosed herein , the active pharmaceutical ingredient comprises a TrkB or TrkC agonist. In some cases, the formulations described herein have a pH and/or practical osmolarity as described herein, and have a concentration of a TrkB or TrkC agonist between about 1 μM and about 10 μM, between about 1 mM and about 100 mM, between about 0.1 mM and about 100 mM, between about 0.1 mM and about 100 nM. As disclosed herein , the formulations described herein have a pH and/or practical osmolarity as described herein, and have a concentration of a TrkB or TrkC agonist between about 0.001% - about 60%, between about 0.01% - about 20%, between about 0.01% - about 10%., between about 0.01% - about 7.5%, between about 0.01% - 6%, between about 0.01 - 5%, between about 0.1 - about 10%, or between about 0.1 - about 6% of the active ingredient by weight of the formulation. As disclosed herein , the formulations described herein have a pH and/or practical osmolarity as described herein, and have a concentration of a TrkB or TrkC agonist between about 0.1 mg/mL and about 100 mg/mL, between about 1 mg/mL and about 100 mg/mL, between about 1 mg/mL and about 80 mg/mL, between about 1 mg/mL and about 60 mg/mL, between about 1 mg/mL and about 50 mg/mL, between about 1 mg/mL and about 50 mg/mL, between about 1 mg/mL and about 20 mg/mL, between about 1 mg/mL to about 10 mg/mL,-between about 1 mg/mL to about 5 mg/mL, or between about 0.5 mg/mL and about 5 mg/mL of the TrkB or TrkC agonist by volume of the formulation. As disclosed herein , the formulations described herein have a pH and/or practical osmolarity as described herein, and have a concentration of a TrkB or TrkC agonist between about 1 μg/mL and about 500 μg/mL, between about 1 μg/mL and about 250 μg/mL, between about 1 μg and about 100 μg/mL, between about 1 μg/mL and about 50 μg/mL, or between about 1 μg/mL and about 20 μg/mL of the TrkB or TrkC agonist by volume of the formulation.

## Tunable release characteristics

The release of non-natural TrkB or TrkC agonists from any formulation, composition or device described herein is optionally tunable to the desired release characteristics. As disclosed herein , a composition described herein is a solution that is substantially free of gelling components. In such instances, the composition provides essentially immediate release of the TrkB or TrkC agonists. In some of such embodiments, the composition is useful in perfusion of otic structures, e.g., during surgery.

In some of such embodiments, the composition provides release of the non-natural TrkB or TrkC agonist from about 2 days to about 4 days.

As disclosed herein , a composition described herein comprising a non-natural TrkB or TrkC agonist, further comprises a gelling agent (e.g., poloxamer 407) and provides release of the non-natural TrkB or TrkC agonist over a period of from about 1 day to about 3 days. As disclosed herein , a composition described herein comprising a non-natural TrkB or TrkC agonist, further comprises a gelling agent (e.g., poloxamer 407) and provides release of the non-natural TrkB or TrkC agonist over a period of from about 1 day to about 5 days. As disclosed herein , a composition described herein comprising a non-natural TrkB or TrkC agonist, further comprises a gelling agent (e.g., poloxamer 407) and provides release of the non-natural TrkB or TrkC agonist over a period of from about 2 days to about 7 days.

As disclosed herein , a composition described herein comprising a non-natural TrkB or TrkC agonist, further comprises about 14-17% of a gelling agent (e.g., poloxamer 407), and provides extended sustained release over a period of from about 1 week to about 3 weeks. As disclosed herein , a composition described herein comprising a non-natural TrkB or TrkC agonist, further comprises about 18-21% of a gelling agent (e.g., poloxamer 407) and, provides extended sustained release over a period of from about 3 weeks to about 6 weeks.

As disclosed herein , the viscosity of any formulation described herein comprising a non-natural TrkB or TrkC agonist, is designed to provide a suitable rate of release from an auris compatible gel. As disclosed herein , the concentration of a thickening agent (e.g., gelling components such as polyoxyethylene-polyoxypropylene copolymers) allows for a tunable mean dissolution time (MDT). The MDT is inversely proportional to the release rate of an active agent from a composition or device described herein. Experimentally, the released non-natural TrkB or TrkC agonist is optionally fitted to the Korsmeyer-Peppas equation

$$\frac{Q}{Q_\alpha} = kt^n + b$$

where Q is the amount of otic agent released at time t, $Q_\alpha$ is the overall released amount of otic agent, k is a release constant of the nth order, *n* is a dimensionless number related to the dissolution mechanism and b is the axis intercept, characterizing the initial burst release mechanism wherein n=1 characterizes an erosion controlled mechanism. The mean dissolution time (MDT) is the sum of different periods of time the drug molecules stay in the matrix before release, divided by the total number of molecules and is optionally calculated by:

$$MDT = \frac{nk^{-1/n}}{n+1}$$

For example, a linear relationship between the mean dissolution time (MDT) of a composition or device and the concentration of the gelling agent (e.g., poloxamer) indicates that the non-natural TrkB or TrkC agonist is released due to the erosion of the polymer gel (e.g., poloxamer) and not via diffusion. In another example, a non-linear relationship indicates release of otic agent via a combination of diffusion and/or polymer gel degradation. In another example, a faster gel elimination time course of a composition or device (a faster release of the non-natural TrkB or TrkC agonist) indicates lower mean dissolution time (MDT). The concentration of gelling components and/or active agent in a composition are tested to determine suitable parameters for MDT. As disclosed herein , injection volumes are also tested to determine suitable parameters for preclinical and clinical studies. The gel strength and concentration of the non-natural TrkB or TrkC agonist affects release kinetics of the non-natural TrkB or TrkC agonist from the composition. At low poloxamer concentration, elimination rate is accelerated (MDT is lower). An increase in the non-natural TrkB or TrkC agonist concentration in the composition or device prolongs residence time and/or MDT of the non-natural TrkB or TrkC agonist in the ear.

As disclosed herein , the MDT for poloxamer from a composition or device described herein is at least 6 hours. As disclosed herein , the MDT for poloxamer from a composition or device described herein is at least 10 hours.

As disclosed herein , the MDT for a TrkB or TrkC agonist from a composition or device described herein is from about 30 hours to about 48 hours. As disclosed herein , the MDT for a TrkB or TrkC agonist from a composition or device described herein is from about 30 hours to about 96 hours. As disclosed herein , the MDT for a TrkB or TrkC agonist from a composition or device described herein is from about 30 hours to about 1 week. As disclosed herein , the MDT for a TrkB or TrkC agonist from a composition or device described herein is from about 1 week to about 6 weeks.

A disclosed herein, any controlled release otic composition described herein increases the exposure of a TrkB or TrkC agonist and increases the Area Under the Curve (AUC) in otic fluids (e.g., endolymph and/or perilymph) by about 30%, about 40%, about 50%, about 60%, about 70%, about 80%, about 90%, about 100%, or higher than 100%, compared to an otic composition that is not a controlled release otic composition. A disclosed herein, any controlled release otic composition described herein increases the exposure time of a TrkB or TrkC agonist and decreases the $C_{max}$ in otic fluids (e.g., endolymph and/or perilymph) by about 40%, about 30%, about 20%, or about 10%, compared

to a formulation that is not a controlled release otic composition. A disclosed herein, any controlled release otic composition described herein alters (e.g. reduces) the ratio of $C_{max}$ to $C_{min}$ compared to a formulation that is not a controlled release otic composition. A disclosed herein, any controlled release otic composition described herein increases the exposure of a TrkB or TrkC agonist and increases the length of time that the concentration of the TrkB or TrkC agonist is above $C_{min}$ by about 30%, about 40%, about 50%, about 60%, about 70%, about 80% or about 90% compared to a formulation that is not a controlled release otic composition. A disclosed herein, the increase in exposure of a TrkB or TrkC agonist and the increase in the length of time that the concentration of the TrkB or TrkC agonist is above $C_{min}$ by a controlled release otic composition described herein is greater than 100% compared to a formulation that is not a controlled release otic composition. In certain instances, controlled release otic compositions described herein delay the time to $C_{max}$. In certain instances, the controlled steady release of a drug prolongs the time the concentration of the TrkB or TrkC agonist will stay above the $C_{min}$. As disclosed herein , otic compositions described herein prolong the residence time of a TrkB or TrkC agonist in the inner ear and provide a stable drug exposure profile. In some instances, an increase in concentration of a TrkB or TrkC agonist in the otic composition saturates the clearance process and allows for a more rapid and stable steady state to be reached.

In certain instances, once exposure to a TrkB or TrkC agonist (e.g., concentration in the endolymph or perilymph) reaches steady state, the concentration of the TrkB or TrkC agonist in the endolymph or perilymph stays at or about the therapeutic dose for an extended period of time (e.g., one day, 2 days, 3 days, 4 days, 5 days, 6 days, or 1 week, 3 weeks, 6 weeks, 2 months). As disclosed herein , the steady state concentration of a TrkB or TrkC agonist released from a controlled release otic composition described herein is about 20 to about 50 times the steady state concentration of a TrkB or TrkC agonist released from a formulation that is not a controlled release otic composition.

**Pharmaceutical Formulations**

Provided herein are otic pharmaceutical compositions or devices that include at least one TrkB or TrkC agonist and a pharmaceutically acceptable diluent(s), excipient(s), or carrier(s). As disclosed herein , the pharmaceutical compositions include other medicinal or pharmaceutical agents, carriers, adjuvants, such as preserving, stabilizing, wetting or emulsifying agents, solution promoters, salts for regulating the osmotic pressure, and/or buffers. As disclosed herein , the otic composition or device comprises (i) a non-natural TrkB or TrkC agonist (ii) a gelling and viscosity enhancing agent, (iii) a pH adjusting agent, and (iv) sterile water.

In other embodiments, the otic pharmaceutical compositions also contain other therapeutic substances.

As disclosed herein , the otic pharmaceutical compositions or devices described herein include a dye to help enhance the visualization of the gel when applied. As disclosed herein , dyes that are compatible with the auris-acceptable compositions or devices described herein include Evans blue (e.g., 0.5% of the total weight of an otic formulation), Methylene blue (e.g., 1% of the total weight of an otic formulation), Isosulfan blue (e.g., 1% of the total weight of an otic formulation), Trypan blue (e.g., 0.15% of the total weight of an otic formulation), and/or indocyanine green (e.g., 25mg/vial). Other common dyes, e.g, FD&C red 40, FD&C red 3, FD&C yellow 5, FD&C yellow 6, FD&C blue 1, FD&C blue2, FD&C green 3, fluorescence dyes (e.g., Fluorescein isothiocyanate, rhodamine, Alexa Fluors, DyLight Fluors) and/or dyes that are visualizable in conjunction with non-invasive imaging techniques such as MRI, CAT scans, PET scans or the like. Gadolinium-based MRI dyes, iodine-base dyes, barium-based dyes or the like are also contemplated for use with any otic composition described herein. Other dyes that are compatible with any formulation or composition described herein are listed in the Sigma-Aldrich catalog under dyes.

As disclosed herein , mechanical or imaging devices are used to monitor or survey the hearing, balance or other auris disorder. For example, magnetic resonance imaging (MRI) devices are specifically contemplated within the scope of the embodiments, wherein the MRI devices (for example, 3 Tesla MRI devices) are capable of evaluating Meniere Disease progression, and subsequent treatment with the pharmaceutical formulations disclosed herein. Gadolinium-based dyes, iodine-base dyes, barium-based dyes or the like are also contemplated for use with any auris-compatible composition or device described herein and/or with any mechanical or imaging devices described herein. A disclosed herein, gadolinium hydrate is used in combination with MRI and/or any pharmaceutical composition or device described herein to evaluate disease severity (e.g., size of endolymphatic hydrops), formulation penetration into the inner ear, and/or therapeutic effectiveness of the pharmaceutical formulations/devices in the otic diseases described herein (e.g., Meniere's disease).

Any otic pharmaceutical composition or device described herein is administered by locating the composition or device in contact with the crista fenestrae cochlea, the round window, the tympanic cavity, the tympanic membrane, the auris media or the auris externa.

In one specific embodiment of the auris-acceptable controlled release TrkB agonist pharmaceutical formulations described herein, the TrkB agonist is provided in a gel matrix, also referred to herein as "auris acceptable gel formulations," "auris interna-acceptable gel formulations," "auris media-acceptable gel formulations," "auris externa-acceptable gel formulations", "auris gel formulations" or variations thereof. All of the components of the gel formulation must be compatible with the targeted auris structure. Further, the gel formulations provide controlled release of the TrkB to the desired site within the targeted auris structure; As disclosed herein , the gel formulation also has an immediate or rapid release component for delivery of the TrkB or TrkC agonist to the desired target site. In other embodiments, the gel formulation has a sustained release component for delivery of the TrkB or TrkC agonist. As disclosed herein , the auris gel formulations are biodegradeable. As disclosed herein, the auris gel formulations include a mucoadhesive excipient to allow adhesion to the external mucous layer of the round window membrane. In yet other embodiments, the auris gel formulations include a penetration enhancer excipient; As disclosed herein , the auris gel formulation contains a viscosity enhancing agent sufficient to provide a viscosity of between about 500 and 1,000,000 centipoise, between about 750 and 1,000,000 centipoise; between about 1000 and 1,000,000 centipoise; between about 1000 and 400,000 centipoise; between about 2000 and 100,000 centipoise; between about 3000 and 50,000 centipoise; between about 4000 and 25,000 centipoise; between about 5000 and 20,000 centipoise; or between about 6000 and 15,000 centipoise. As disclosed herein , the auris gel formulation contains a viscosity enhancing agent sufficient to provide a viscosity of between about 50,0000 and 1,000,000 centipoise.

As disclosed herein , the otic pharmaceutical compositions or devices described herein are low viscosity compositions or devices at body temperature. As disclosed herein , low viscosity compositions or devices contain from about 1% to about 10% of a viscosity enhancing agent (e.g., gelling components such as polyoxyethylene-polyoxypropylene copolymers). As disclosed herein , low viscosity compositions or devices contain from about 2% to about 10% of a viscosity enhancing agent (e.g., gelling components such as polyoxyethylene-polyoxypropylene copolymers). As disclosed herein , low viscosity compositions or devices contain from about 5% to about 10% of a viscosity enhancing agent (e.g., gelling components such as polyoxyethylene-polyoxypropylene copolymers). As disclosed herein , low viscosity compositions or devices are substantially free of a viscosity enhancing agent (e.g., gelling components such as polyoxyethylene-polyoxypropylene copolymers). As disclosed herein , a low viscosity TrkB or TrkC agonist composition or device described herein provides an apparent viscosity of from about 100 cP to about 10,000 cP. As disclosed herein , a low viscosity TrkB or TrkC agonist composition or device described herein provides an apparent viscosity of from about 500 cP to about 10,000 cP. As disclosed herein , a low viscosity TrkB or TrkC agonist composition or device described herein provides an apparent viscosity of from about 1000 cP to about 10,000 cP. A disclosed herein, a low viscosity TrkB or TrkC agonist composition or device is administered in combination with an external otic intervention, e.g., a surgical procedure including but not limited to middle ear surgery, inner ear surgery, typanostomy, cochleostomy, labyrinthotomy, mastoidectomy, stapedectomy, stapedotomy, endolymphatic sacculotomy or the like. As disclosed herein, a low viscosity TrkB or TrkC agonist composition or device is administered during an otic intervention. As disclosed herein, a low viscosity TrkB or TrkC agonist composition or device is administered before the otic intervention.

As disclosed herein , the otic pharmaceutical compositions or devices described herein are high viscosity compositions or devices at body temperature. As disclosed herein , high viscosity compositions or devices contain from about 10% to about 25% of a viscosity enhancing agent (e.g., gelling components such as polyoxyethylene-polyoxypropylene copolymers). As disclosed herein , high viscosity compositions or devices contain from about 14% to about 22% of a viscosity enhancing agent (e.g., gelling components such as polyoxyethylene-polyoxypropylene copolymers). As disclosed herein , high viscosity compositions or devices contain from about 15% to about 21% of a viscosity enhancing agent (e.g., gelling components such as polyoxyethylene-polyoxypropylene copolymers). As disclosed herein , a high viscosity TrkB or TrkC agonist composition or device described herein provides an apparent viscosity of from about 100,000 cP to about 1,000,000 cP. As disclosed herein , a high viscosity TrkB or TrkC agonist composition or device described herein provides an apparent viscosity of from about 150,000 cP to about 500,000 cP. As disclosed herein , a high viscosity TrkB or TrkC agonist composition or device described herein provides an apparent viscosity of from about 250,000 cP to about 500,000 cP. In some of such embodiments, a high viscosity composition or device is a liquid at room temperature and gels at about between room temperature and body temperature (including an individual with a serious fever, e.g., up to about 42 °C). As disclosed herein , an otic high viscosity composition or device comprising a TrkB or TrkC agonist is administered as monotherapy for treatment of an otic disease or condition described herein. As disclosed herein , an otic high viscosity composition or device comprising a TrkB or TrkC agonist is administered in combination with an external otic intervention, e.g., a surgical procedure including but not limited to middle ear surgery, inner ear surgery, typanostomy, cochleostomy, labyrinthotomy, mastoidectomy, stapedectomy, stapedotomy, endolymphatic sacculotomy or the like. As disclosed herein, a high viscosity otic composition or device comprising a TrkB or TrkC agonist is administered after the otic intervention. In other such embodiments, a high viscosity TrkB or TrkC agonist composition or device is administered before the otic intervention.

In other embodiments, the otic pharmaceutical formulations described herein further provide an auris-acceptable hydrogel; in yet other embodiments, the otic pharmaceutical formulations provide an auris-acceptable microsphere or microparticle; in still other embodiments, the otic pharmaceutical formulations provide an auris-acceptable liposome. As disclosed herein , the otic pharmaceutical formulations provide an auris-acceptable foam; in yet other embodiments, the otic pharmaceutical formulations provide an auris-acceptable paint; in still further embodiments, otic pharmaceutical formulations provide an auris-acceptable in situ forming spongy material. As disclosed herein , the otic pharmaceutical

formulations provide an auris-acceptable solvent release gel. As disclosed herein , the otic pharmaceutical formulations provide an actinic radiation curable gel. Further embodiments include a thermoreversible gel in the otic pharmaceutical formulation, such that upon preparation of the gel at room temperature or below, the formulation is a fluid, but upon application of the gel into or near the auris interna and/or auris media target site, including the tympanic cavity, round window membrane or the crista fenestrae cochleae, the otic-pharmaceutical formulation stiffens or hardens into a gel-like substance.

In further or alternative embodiments, the otic gel formulations are capable of being administered on or near the round window membrane via intratympanic injection. In other embodiments, the otic gel formulations are administered on or near the round window or the crista fenestrae cochleae through entry via a post-auricular incision and surgical manipulation into or near the round window or the crista fenestrae cochleae area. Alternatively, the otic gel formulation is applied via syringe and needle, wherein the needle is inserted through the tympanic membrane and guided to the area of the round window or crista fenestrae cochleae. The otic gel formulations are then deposited on or near the round window or crista fenestrae cochleae for localized treatment of autoimmune otic disorders. In other embodiments, the otic gel formulations are applied via microcathethers implanted into the patient, and in yet further embodiments the formulations are administered via a pump device onto or near the round window membrane. In still further embodiments, the otic gel formulations are applied at or near the round window membrane via a microinjection device. In yet other embodiments, the otic gel formulations are applied in the tympanic cavity. As disclosed herein , the otic gel formulations are applied on the tympanic membrane. In still other embodiments, the otic gel formulations are applied onto or in the auditory canal.

### Controlled Release Formulations

In general, controlled release drug formulations impart control over the release of drug with respect to site of release and time of release within the body. As discussed herein, controlled release refers to immediate release, delayed release, sustained release, extended release, variable release, pulsatile release and bi-modal release. Many advantages are offered by controlled release. First, controlled release of a pharmaceutical agent allows less frequent dosing and thus minimizes repeated treatment. Second, controlled release treatment results in more efficient drug utilization and less of the compound remains as a residue. Third, controlled release offers the possibility of localized drug delivery by placement of a delivery device or formulation at the site of disease. Still further, controlled release offers the opportunity to administer and release two or more different drugs, each having a unique release profile, or to release the same drug at different rates or for different durations, by means of a single dosage unit.

Accordingly, one aspect of the embodiments disclosed herein is to provide a controlled release TrkB or TrkC agonist auris-acceptable composition or device for the treatment of autoimmune disorders and/or inflammatory disorders. The controlled release aspect of the compositions and/or formulations and/or devices disclosed herein is imparted through a variety of agents, including but not limited to excipients, agents or materials that are acceptable for use in the auris interna or other otic structure. By way of example only, such excipients, agents or materials include an auris-acceptable polymer, an auris-acceptable viscosity enhancing agent, an auris-acceptable gel, an auris-acceptable paint, an auris-acceptable foam, an auris-acceptable xerogel, an auris-acceptable microsphere or microparticle, an auris-acceptable hydrogel, an auris-acceptable in situ forming spongy material, an auris-acceptable actinic radiation curable gel, an auris-acceptable solvent release gel, an auris-acceptable liposome, an auris-acceptable nanocapsule or nanosphere, an auris-acceptable thermoreversible gel, or combinations thereof.

### Auris-Acceptable Gels

Gels, sometimes referred to as jellies, have been defined in various ways. For example, the United States Pharmacopoeia defines gels as semisolid systems consisting of either suspensions made up of small inorganic particles or large organic molecules interpenetrated by a liquid. Gels include a single-phase or a two-phase system. A single-phase gel consists of organic macromolecules distributed uniformly throughout a liquid in such a manner that no apparent boundaries exist between the dispersed macromolecules and the liquid. Some single-phase gels are prepared from synthetic macromolecules (e.g., carbomer) or from natural gums, (e.g., tragacanth). As disclosed herein , single-phase gels are generally aqueous, but will also be made using alcohols and oils. Two-phase gels consist of a network of small discrete particles.

Gels can also be classified as being hydrophobic or hydrophilic. A disclosed herein, the base of a hydrophobic gel consists of a liquid paraffin with polyethylene or fatty oils gelled with colloidal silica, or aluminum or zinc soaps. In contrast, the base of hydrophilic gels usually consists of water, glycerol, or propylene glycol gelled with a suitable gelling agent (e.g., tragacanth, starch, cellulose derivatives, carboxyvinylpolymers, and magnesium-aluminum silicates). As disclosed herein, the rheology of the compositions or devices disclosed herein is pseudo plastic, plastic, thixotropic, or dilatant.

As disclosed herein, the enhanced viscosity auris-acceptable formulation described herein is not a liquid at room temperature. As disclosed herein, the enhanced viscosity formulation is characterized by a phase transition between room temperature and body temperature (including an individual with a serious fever, e.g., up to about 42 °C). As disclosed herein , the phase transition occurs at 1 °C below body temperature, at 2 °C below body temperature, at 3 °C below body temperature, at 4 °C below body temperature, at 6 °C below body temperature, at 8 °C below body temperature, or at 10 °C below body temperature. As disclosed herein , the phase transition occurs at about 15 °C below body temperature, at about 20 °C below body temperature or at about 25 °C below body temperature. As disclosed herein, the gelation temperature (Tgel) of a formulation described herein is about 20 °C, about 25 °C, or about 30 °C. As disclosed herein, the gelation temperature (Tgel) of a formulation described herein is about 35 °C, or about 40 °C. As disclosed herein , administration of any formulation described herein at about body temperature reduces or inhibits vertigo associated with intratympanic administration of otic formulations. Included within the definition of body temperature is the body temperature of a healthy individual, or an unhealthy individual, including an individual with a fever (up to ~42 °C). As disclosed herein , the pharmaceutical compositions or devices described herein are liquids at about room temperature and are administered at or about room temperature, reducing or ameliorating side effects such as, for example, vertigo.

Polymers composed of polyoxypropylene and polyoxyethylene form thermoreversible gels when incorporated into aqueous solutions. These polymers have the ability to change from the liquid state to the gel state at temperatures close to body temperature, therefore allowing useful formulations that are applied to the targeted auris structure(s). The liquid state-to-gel state phase transition is dependent on the polymer concentration and the ingredients in the solution.

Poloxamer 407 (PF-127) is a nonionic surfactant composed of polyoxyethylene-polyoxypropylene copolymers. Other poloxamers include 188 (F-68 grade), 237 (F-87 grade), 338 (F-108 grade). Aqueous solutions of poloxamers are stable in the presence of acids, alkalis, and metal ions. PF-127 is a commercially available polyoxyethylene-polyoxypropylene triblock copolymer of general formula E106 P70 E106, with an average molar mass of 13,000. The polymer can be further purified by suitable methods that will enhance gelation properties of the polymer. It contains approximately 70% ethylene oxide, which accounts for its hydrophilicity. It is one of the series of poloxamer ABA block copolymers, whose members share the chemical formula shown below.

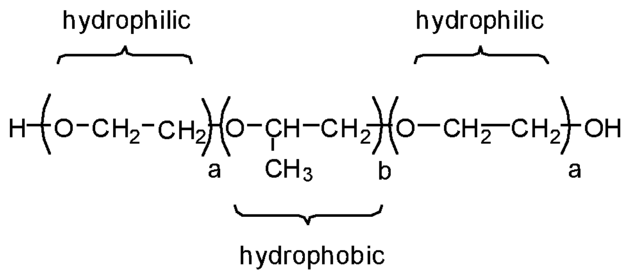


PF-127 is of particular interest since concentrated solutions (>20% w/w) of the copolymer are transformed from low viscosity transparent solutions to solid gels on heating to body temperature. This phenomenon, therefore, suggests that when placed in contact with the body, the gel preparation will form a semi-solid structure and a sustained release depot. Furthermore, PF-127 has good solubilizing capacity, low toxicity and is, therefore, considered a good medium for drug delivery systems.

In an alternative embodiment, the thermogel is a PEG-PLGA-PEG triblock copolymer (Jeong etal, Nature (1997), 388:860-2; Jeong etal, J. Control. Release (2000), 63:155-63; Jeong etal, Adv. Drug Delivery Rev. (2002), 54:37-51). The polymer exhibits sol-gel behavior over a concentration of about 5% w/w to about 40% w/w. Depending on the properties desired, the lactide/glycolide molar ratio in the PLGA copolymer ranges from about 1:1 to about 20:1. The resulting coploymers are soluble in water and form a free-flowing liquid at room temperature, but form a hydrogel at body temperature. A commercially available PEG-PLGA-PEG triblock copolymer is RESOMER RGP t50106 manufactured by Boehringer Ingelheim. This material is composed of a PGLA copolymer of 50:50 poly(DL-lactide-co-glycolide) and is 10% w/w of PEG and has a molecular weight of about 6000.

ReGel® is a tradename of MacroMed Incorporated for a class of low molecular weight, biodegradable block copolymers having reverse thermal gelation properties as described in U.S. Pat. Nos. 6,004,573, 6,117949, 6,201,072, and 6,287,588. It also includes biodegradable polymeric drug carriers disclosed in pending U.S. patent application Ser. Nos. 09/906,041 (U.S. Pat No. 6,589,549 B2), 09/559,799 (U.S. Patent No. 7,018,645 B1) and 10/919,603 (U.S. Patent Publication No. 2006/0034889 A1). The biodegradable drug carrier comprises ABA-type or BAB-type triblock copolymers or mixtures thereof, wherein the A-blocks are relatively hydrophobic and comprise biodegradable polyesters or poly(orthoester)s, and the B-blocks are relatively hydrophilic and comprise polyethylene glycol (PEG), said copolymers having a hydrophobic content of between 50.1 to 83% by weight and a hydrophilic content of between 17 to 49.9% by weight, and an overall block copolymer molecular weight of between 2000 and 8000 Daltons. The drug carriers exhibit water

solubility at temperatures below normal mammalian body temperatures and undergo reversible thermal gelation to then exist as a gel at temperatures equal to physiological mammalian body temperatures. The biodegradable, hydrophobic A polymer block comprises a polyester or poly(ortho ester), in which the polyester is synthesized from monomers selected from the group consisting of D,L-lactide, D-lactide, L-lactide, D,L-lactic acid, D-lactic acid, L-lactic acid, glycolide, glycolic acid, ε-caprolactone, ε-hydroxyhexanoic acid, γ-butyrolactone, γ-hydroxybutyric acid, δ-valerolactone, δ-hydroxyvaleric acid, hydroxybutyric acids, malic acid, and copolymers thereof and having an average molecular weight of between about 600 and 3000 Daltons. The hydrophilic B-block segment is preferably polyethylene glycol (PEG) having an average molecular weight of between about 500 and 2200 Daltons.

Additional biodegradable thermoplastic polyesters include AtriGel® (provided by Atrix Laboratories, Inc.) and/or those disclosed, e.g., in U.S. Patent Nos. 5,324,519; 4,938,763; 5,702,716; 5,744,153; and 5,990,194; wherein the suitable biodegradable thermoplastic polyester is disclosed as a thermoplastic polymer. Examples of suitable biodegradable thermoplastic polyesters include polylactides, polyglycolides, polycaprolactones, copolymers thereof, terpolymers thereof, and any combinations thereof. As disclosed herein, the suitable biodegradable thermoplastic polyester is a polylactide, a polyglycolide, a copolymer thereof, a terpolymer thereof, or a combination thereof. As disclosed herein , the biodegradable thermoplastic polyester is 50/50 poly(DL-lactide-co-glycolide) having a carboxy terminal group; is present in about 30 wt. % to about 40 wt. % of the composition; and has an average molecular weight of about 23,000 to about 45,000. Alternatively, in another embodiment, the biodegradable thermoplastic polyester is 75/25 poly (DL-lactide-co-glycolide) without a carboxy terminal group; is present in about 40 wt. % to about 50 wt. % of the composition; and has an average molecular weight of about 15,000 to about 24,000. As disclosed herein, the terminal groups of the poly(DL-lactide-co-glycolide) are either hydroxyl, carboxyl, or ester depending upon the method of polymerization. Polycondensation of lactic or glycolic acid provides a polymer with terminal hydroxyl and carboxyl groups. Ring-opening polymerization of the cyclic lactide or glycolide monomers with water, lactic acid, or glycolic acid provides polymers with the same terminal groups. However, ring-opening of the cyclic monomers with a monofunctional alcohol such as methanol, ethanol, or 1-dodecanol provides a polymer with one hydroxyl group and one ester terminal groups. Ring-opening polymerization of the cyclic monomers with a diol such as 1,6-hexanediol or polyethylene glycol provides a polymer with only hydroxyl terminal groups.

Since the polymer systems of thermoreversible gels dissolve more completely at reduced temperatures, methods of solubilization include adding the required amount of polymer to the amount of water to be used at reduced temperatures. Generally after wetting the polymer by shaking, the mixture is capped and placed in a cold chamber or in a thermostatic container at about 0-10 °C in order to dissolve the polymer. The mixture is stirred or shaken to bring about a more rapid dissolution of the thermoreversible gel polymer. The TrkB or TrkC agonist and various additives such as buffers, salts, and preservatives are subsequently added and dissolved. In some instances the TrkB or TrkC agonist and/or other pharmaceutically active agent is suspended if it is insoluble in water. The pH is modulated by the addition of appropriate buffering agents. round window membrane mucoadhesive characteristics are optionally imparted to a thermoreversible gel by incorporation of round window membrane mucoadhesive carbomers, such as Carbopol® 934P, to the composition (Majithiya etal, AAPS PharmSciTech (2006), 7(3), p. E1; EP0551626).

As disclosed herein are auris-acceptable pharmaceutical gel formulations which do not require the use of an added viscosity enhancing agent. Such gel formulations incorporate at least one pharmaceutically acceptable buffer. In one aspect is a gel formulation comprising a TrkB or TrkC agonist and a pharmaceutically acceptable buffer. As disclosed herein, the pharmaceutically acceptable excipient or carrier is a gelling agent.

As disclosed herein, useful TrkB or TrkC agonist auris-acceptable pharmaceutical formulations also include one or more pH adjusting agents or buffering agents to provide an endolymph or perilymph suitable pH. Suitable pH adjusting agents or buffers include, but are not limited to acetate, bicarbonate, ammonium chloride, citrate, phosphate, pharmaceutically acceptable salts thereof and combinations or mixtures thereof. Such pH adjusting agents and buffers are included in an amount required to maintain pH of the composition between a pH of about 5 and about 9, As disclosed herein a pH between about 6.5 to about 7.5, and in yet another disclosure herein at a pH of about 6.5, 6.6, 6.7, 6.8, 6.9, 7.0, 7.1, 7.2, 7.3, 7.4, 7.5, 7.6, 7.7, 7.8, 7.9, or 8.0. As disclosed herein, when one or more buffers are utilized in the formulations of the present disclosure, they are combined, e.g., with a pharmaceutically acceptable vehicle and are present in the final formulation, e.g., in an amount ranging from about 0.1% to about 20%, from about 0.5% to about 10%. As disclosed hereinof the present disclosure, the amount of buffer included in the gel formulations are an amount such that the pH of the gel formulation does not interfere with the auris media or auris interna's natural buffering system, or does not interfere with the natural pH of the endolymph or perilymph: depending on where in the cochlea the TrkB or TrkC agonist formulation is targeted. As disclosed herein , from about 10 μM to about 200 mM concentration of a buffer is present in the gel formulation. As disclosed herein, from about a 5 mM to about a 200 mM concentration of a buffer is present. As disclosed herein, from about a 20 mM to about a 100 mM concentration of a buffer is present. As disclosed herein is a buffer such as acetate or citrate at slightly acidic pH. As disclosed herein the buffer is a sodium acetate buffer having a pH of about 4.5 to about 6.5. As disclosed herein the buffer is a sodium citrate buffer having a pH of about 5.0 to about 8.0, or about 5.5 to about 7.0.

As disclosed herein, the buffer used is tris(hydroxymethyl)aminomethane, bicarbonate, carbonate or phosphate at slightly basic pH. As disclosed herein , the buffer is a sodium bicarbonate buffer having a pH of about 6.5 to about 8.5, or about 7.0 to about 8.0. As disclosed herein, the buffer is a sodium phosphate dibasic buffer having a pH of about 6.0 to about 9.0.

Also described herein are controlled release formulations or devices comprising a TrkB or TrkC agonist and a viscosity enhancing agent. Suitable viscosity-enhancing agents include by way of example only, gelling agents and suspending agents. As disclosed herein , the enhanced viscosity formulation does not include a buffer. As disclosed herein, the enhanced viscosity formulation includes a pharmaceutically acceptable buffer. Sodium chloride or other tonicity agents are optionally used to adjust tonicity, if necessary.

By way of example only, the auris-acceptable viscosity agent includes hydroxypropyl methylcellulose, hydroxyethyl cellulose, polyvinylpyrrolidone, carboxymethyl cellulose, polyvinyl alcohol, sodium chondroitin sulfate, sodium hyaluronate. Other viscosity enhancing agents compatible with the targeted auris structure include, but are not limited to, acacia (gum arabic), agar, aluminum magnesium silicate, sodium alginate, sodium stearate, bladderwrack, bentonite, carbomer, carrageenan, Carbopol, xanthan, cellulose, microcrystalline cellulose (MCC), ceratonia, chitin, carboxymethylated chitosan, chondrus, dextrose, furcellaran, gelatin, Ghatti gum, guar gum, hectorite, lactose, sucrose, maltodextrin, mannitol, sorbitol, honey, maize starch, wheat starch, rice starch, potato starch, gelatin, sterculia gum, xanthum gum, gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethylcellulose (HPMC), sodium carboxymethyl-cellulose (CMC), silicon dioxide, polyvinylpyrrolidone (PVP: povidone), Splenda® (dextrose, maltodextrin and sucralose) or combinations thereof. As disclosed herein, the viscosity-enhancing excipient is a combination of MCC and CMC. As disclosed herein, the viscosity-enhancing agent is a combination of carboxymethylated chitosan, or chitin, and alginate. The combination of chitin and alginate with the TrkB or TrkC agonists disclosed herein acts as a controlled release formulation, restricting the diffusion of the TrkB or TrkC agonists from the formulation. Moreover, the combination of carboxymethylated chitosan and alginate is optionally used to assist in increasing the permeability of the TrkB or TrkC agonists through the round window membrane.

As disclosed herein is an enhanced viscosity formulation, comprising from about 0.1 mM and about 100 mM of an TrkB or TrkC agonist, a pharmaceutically acceptable viscosity agent, and water for injection, the concentration of the viscosity agent in the water being sufficient to provide an enhanced viscosity formulation with a final viscosity from about 100 to about 100,000 cP. As disclosed herein, the viscosity of the gel is in the range from about 100 to about 50,000 cP, about 100 cP to about 1,000 cP, about 500 cP to about 1500 cP, about 1000 cP to about 3000 cP, about 2000 cP to about 8,000 cP, about 4,000 cP to about 50,000 cP, about 10,000 cP to about 500,000 cP, about 15,000 cP to about 1,000,000 cP. As disclosed herein, when an even more viscous medium is desired, the biocompatible gel comprises at least about 35%, at least about 45%, at least about 55%, at least about 65%, at least about 70%, at least about 75%, or even at least about 80% or so by weight of the TrkB or TrkC agonist. In highly concentrated samples, the biocompatible enhanced viscosity formulation comprises at least about 25%, at least about 35%, at least about 45%, at least about 55%, at least about 65%, at least about 75%, at least about 85%, at least about 90% or at least about 95% or more by weight of the TrkB or TrkC agonist.

As disclosed herein , the viscosity of the gel formulations presented herein are measured by any means described. For example, As disclosed herein , an LVDV-II+CP Cone Plate Viscometer and a Cone Spindle CPE-40 is used to calculate the viscosity of the gel formulation described herein. As disclosed herein, a Brookfield (spindle and cup) viscometer is used to calculate the viscosity of the gel formulation described herein. As disclosed herein , the viscosity ranges referred to herein are measured at room temperature. As disclosed herein the viscosity ranges referred to herein are measured at body temperature (e.g., at the average body temperature of a healthy human).

As disclosed herein , the pharmaceutically acceptable enhanced viscosity auris-acceptable formulation comprises at least one TrkB or TrkC agonist and at least one gelling agent. Suitable gelling agents for use in preparation of the gel formulation include, but are not limited to, celluloses, cellulose derivatives, cellulose ethers (e.g., carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (e.g., alginic acid), silicates, starch, tragacanth, carboxyvinyl polymers, carrageenan, paraffin, petrolatum and any combinations or mixtures thereof. As disclosed herein, hydroxypropylmethylcellulose (Methocel®) is utilized as the gelling agent. As disclosed herein, the viscosity enhancing agents described herein are also utilized as the gelling agent for the gel formulations presented herein.

As disclosed herein , the TrkB or TrkC agonists disclosed herein are dispensed as an auris-acceptable paint. As used herein, paints (also known as film formers) are solutions comprised of a solvent, a monomer or polymer, an active agent, and optionally one or more pharmaceutically-acceptable excipients. After application to a tissue, the solvent evaporates leaving behind a thin coating comprised of the monomers or polymers, and the TrkB or TrkC agonist. The coating protects the TrkB or TrkC agonists and maintains them in an immobilized state at the site of application. This

decreases the amount of TrkB or TrkC agonists which may be lost and correspondingly increases the amount delivered to the subject. By way of non-limiting example, paints include collodions (e.g. Flexible Collodion, USP), and solutions comprising saccharide siloxane copolymers and a cross-linking agent. Collodions are ethyl ether/ethanol solutions containing pyroxylin (a nitrocellulose). After application, the ethyl ether/ethanol solution evaporates leaving behind a thin film of pyroxylin. In solutions comprising saccharide siloxane copolymers, the saccharide siloxane copolymers form the coating after evaporation of the solvent initiates the cross-linking of the saccharide siloxane copolymers. For additional disclosures regarding paints, see *Remington: The Science and Practice of Pharmacy* which is hereby incorporated with respect to this subject matter. The paints contemplated for use herein, are flexible such that they do not interfere with the propagation of pressure waves through the ear. Further, the paints may be applied as a liquid (i.e. solution, suspension, or emulsion), a semisolid (i.e. a gel, foam, paste, or jelly) or an aerosol.

As disclosed herein , the TrkB or TrkC agonists disclosed herein are dispensed as a controlled-release foam. Examples of suitable foamable carriers for use in the compositions disclosed herein include, but are not limited to, alginate and derivatives thereof, carboxymethylcellulose and derivatives thereof, collagen, polysaccharides, including, for example, dextran, dextran derivatives, pectin, starch, modified starches such as starches having additional carboxyl and/or carboxamide groups and/or having hydrophilic side-chains, cellulose and derivatives thereof, agar and derivatives thereof, such as agar stabilized with polyacrylamide, polyethylene oxides, glycol methacrylates, gelatin, gums such as xanthum, guar, karaya, gellan, arabic, tragacanth and locust bean gum, or combinations thereof. Also suitable are the salts of the aforementioned carriers, for example, sodium alginate. The formulation optionally further comprises a foaming agent, which promotes the formation of the foam, including a surfactant or external propellant. Examples of suitable foaming agents include cetrimide, lecithin, soaps, silicones and the like. Commercially available surfactants such as Tween® are also suitable.

As disclosed herein , other gel formulations are useful depending upon the particular TrkB or TrkC agonists, other pharmaceutical agent or excipients/additives used, and as such are considered to fall within the scope of the present disclosure. For example, other commercially-available glycerin-based gels, glycerin-derived compounds, conjugated, or crosslinked gels, matrices, hydrogels, and polymers, as well as gelatins and their derivatives, alginates, and alginate-based gels, and even various native and synthetic hydrogel and hydrogel-derived compounds are all expected to be useful in the TrkB or TrkC agonist formulations described herein. As disclosed herein , auris-acceptable gels include, but are not limited to, alginate hydrogels SAF®-Gel (ConvaTec, Princeton, N.J.), Duoderm® Hydroactive Gel (ConvaTec), Nu-gel ®(Johnson & Johnson Medical, Arlington, Tex.); Carrasyn®(V) Acemannan Hydrogel (Carrington Laboratories, Inc., Irving, Tex.); glycerin gels Elta® Hydrogel (Swiss-American Products, Inc., Dallas, Tex.) and K-Y® Sterile (Johnson & Johnson). As disclosed herein, biodegradable biocompatible gels also represent compounds present in auris-acceptable formulations disclosed and described herein.

In some formulations developed for administration to a mammal, and for compositions formulated for human administration, the auris-acceptable gel comprises substantially all of the weight of the composition. As disclosed herein, the auris-acceptable gel comprises as much as about 98% or about 99% of the composition by weight. This is desirable when a substantially non-fluid, or substantially viscous formulation is needed. As disclosed herein, when slightly less viscous, or slightly more fluid auris-acceptable pharmaceutical gel formulations are desired, the biocompatible gel portion of the formulation comprises at least about 50% by weight, at least about 60% by weight, at least about 70% by weight, or even at least about 80% or 90% by weight of the compound. All intermediate integers within these ranges are contemplated to fall within the scope of this disclosure, and as disclosed herein, even more fluid (and consequently less viscous) auris-acceptable gel compositions are formulated, such as for example, those in which the gel or matrix component of the mixture comprises not more than about 50% by weight, not more than about 40% by weight, not more than about 30% by weight, or even those than comprise not more than about 15% or about 20% by weight of the composition.

### Auris-Acceptable Suspending Agents

As disclosed herein , at least one TrkB or TrkC agonist is included in a pharmaceutically acceptable enhanced viscosity formulation wherein the formulation further comprises at least one suspending agent, wherein the suspending agent assists in imparting controlled release characteristics to the formulation. As disclosed herein , suspending agents also serve to increase the viscosity of the auris-acceptable TrkB or TrkC agonist formulations and compositions.

Suspending agents include, by way of example only, compounds such as polyvinylpyrrolidone, e.g., polyvinylpyrrolidone K12, polyvinylpyrrolidone K17, polyvinylpyrrolidone K25, or polyvinylpyrrolidone K30, vinyl pyrrolidone/vinyl acetate copolymer (S630), sodium carboxymethylcellulose, methylcellulose, hydroxypropylmethylcellulose (hypromellose), hydroxymethylcellulose acetate stearate, polysorbate-80, hydroxyethylcellulose, sodium alginate, gums, such as, e.g., gum tragacanth and gum acacia, guar gum, xanthans, including xanthan gum, sugars, cellulosics, such as, e.g., sodium carboxymethylcellulose, methylcellulose, sodium carboxymethylcellulose, hydroxypropylmethylcellulose, hydroxyethylcellulose, polysorbate-80, sodium alginate, polyethoxylated sorbitan monolaurate, polyethoxylated

sorbitan monolaurate, povidone and the like. As disclosed herein , useful aqueous suspensions also contain one or more polymers as suspending agents. Useful polymers include water-soluble polymers such as cellulosic polymers, e.g., hydroxypropyl methylcellulose, and water-insoluble polymers such as cross-linked carboxyl-containing polymers.

As disclosed herein , the present disclosure provides auris-acceptable gel compositions comprising a therapeutically effective amount of a TrkB or TrkC agonist in a hydroxyethyl cellulose gel. Hydroxyethyl cellulose (HEC) is obtained as a dry powder which is reconstituted in water or an aqueous buffer solution to give the desired viscosity (generally about 200 cps to about 30,000 cps, corresponding to about 0.2 to about 10% HEC). As disclosed herein the concentration of HEC is between about 1% and about 15%, about 1% and about 2%, or about 1.5% to about 2%.

As disclosed herein, the auris-acceptable formulations, including gel formulations and viscosity-enhanced formulations, further include excipients, other medicinal or pharmaceutical agents, carriers, adjuvants, such as preserving, stabilizing, wetting or emulsifying agents, solution promoters, salts, solubilizers, an antifoaming agent, an antioxidant, a dispersing agent, a wetting agent, a surfactant, and combinations thereof.

**Auris-Acceptable Actinic Radiation Curable Gel**

As disclosed herein, the gel is an actinic radiation curable gel, such that following administration to or near the targeted auris structure, use of actinic radiation (or light, including UV light, visible light, or infrared light) the desired gel properties are formed. By way of example only, fiber optics are used to provide the actinic radiation so as to form the desired gel properties. As disclosed herein , the fiber optics and the gel administration device form a single unit. As disclosed herein, the fiber optics and the gel administration device are provided separately.

**Auris-Acceptable Solvent Release Gel**

As disclosed herein , the gel is a solvent release gel such that the desired gel properties are formed after administration to or near the targeted auris structure, that is, as the solvent in the injected gel formulation diffuses out the gel, a gel having the desired gel properties is formed. For example, a formulation that comprises sucrose acetate isobutyrate, a pharmaceutically acceptable solvent, one or more additives, and the TrkB or TrkC agonist is administered at or near the round window membrane: diffusion of the solvent out of the injected formulation provides a depot having the desired gel properties. For example, use of a water soluble solvent provides a high viscosity depot when the solvent diffuses rapidly out of the injected formulation. On the other hand, use of a hydrophobic solvent (e.g., benzyl benzoate) provides a less viscous depot. One example of an auris-acceptable solvent release gel formulation is the SABER™ Delivery System marketed by DURECT Corporation.

**Auris-Acceptable In Situ Forming Spongy Material**

As disclosed hereinis the use of a spongy material, formed in situ in the auris interna or auris media. As disclosed herein , the spongy material is formed from hyaluronic acid or its derivatives. The spongy material is impregnated with a TrkB or TrkC agonist and placed within the auris media so as to provide controlled release of the TrkB or TrkC agonist within the auris media, or in contact with the round window membrane so as to provide controlled release of the TrkB or TrkC agonist into the auris interna. As disclosed herein , the spongy material is biodegradable.

**Round window membrane Mucoadhesives**

As disclosed hereinis the addition of a round window membrane mucoadhesive with the TrkB or TrkC agonist formulations and compositions and devices disclosed herein. The term 'mucoadhesion' is used for materials that bind to the mucin layer of a biological membrane, such as the external membrane of the 3-layered round window membrane. To serve as round window membrane mucoadhesive polymers, the polymers possess some general physiochemical features such as predominantly anionic hydrophilicity with numerous hydrogen bond forming groups, suitable surface property for wetting mucus/mucosal tissue surfaces or sufficient flexibility to penetrate the mucus network.

Round window membrane mucoadhesive agents that are used with the auris-acceptable formulations include, but are not limited to, at least one soluble polyvinylpyrrolidone polymer (PVP); a water-swellable, but water-insoluble, fibrous, cross-linked carboxy-functional polymer; a crosslinked poly(acrylic acid) (e.g. Carbopol® 947P); a carbomer homopolymer; a carbomer copolymer; a hydrophilic polysaccharide gum, maltodextrin, a cross-linked alignate gum gel, a water-dispersible polycarboxylated vinyl polymer, at least two particulate components selected from the group consisting of titanium dioxide, silicon dioxide, and clay, or a mixture thereof. The round window membrane mucoadhesive agent is optionally used in combination with an auris-acceptable viscosity increasing excipient, or used alone to increase the interaction of the composition with the mucosal layer target otic component. In one non-limiting example, the mucoadhesive agent is maltodextrin. As disclosed herein , the mucoadhesive agent is an alginate gum. When used, the

round window membrane mucoadhesive character imparted to the composition is at a level that is sufficient to deliver an effective amount of the TrkB or TrkC agonist composition to, for example, the mucosal layer of round window membrane or the crista fenestrae cochleae in an amount that coats the mucosal membrane, and thereafter deliver the composition to the affected areas, including by way of example only, the vestibular and/or cochlear structures of the auris interna. When used, the mucoadhesive characteristics of the compositions provided herein are determined, and using this information (along with the other teachings provided herein), the appropriate amounts are determined. One method for determining sufficient mucoadhesiveness includes monitoring changes in the interaction of the composition with a mucosal layer, including but not limited to measuring changes in residence or retention time of the composition in the absence and presence of the mucoadhesive excipient.

Mucoadhesive agents have been described, for example, in U.S. Patent Nos. 6,638,521, 6,562,363, 6,509,028, 6,348,502, 6,319,513, 6,306,789, 5,814,330, and 4,900,552.

In another non-limiting example, a mucoadhesive agent is, for example, at least two particulate components selected from titanium dioxide, silicon dioxide, and clay, wherein the composition is not further diluted with any liquid prior to administration and the level of silicon dioxide, if present, is from about 3% to about 15%, by weight of the composition. Silicon dioxide, if present, includes fumed silicon dioxide, precipitated silicon dioxide, coacervated silicon dioxide, gel silicon dioxide, and mixtures thereof. Clay, if present, includes kaolin minerals, serpentine minerals, smectites, illite or a mixture thereof. For example, clay includes laponite, bentonite, hectorite, saponite, montmorillonites or a mixture thereof.

In one non-limiting example, the round window membrane mucoadhesive agent is maltodextrin. Maltodextrin is a carbohydrate produced by the hydrolysis of starch that is optionally derived from corn, potato, wheat or other plant products. Maltodextrin is optionally used either alone or in combination with other round window membrane mucoadhesive agents to impart mucoadhesive characteristics on the compositions disclosed herein. As disclosed herein , a combination of maltodextrin and a carbopol polymer are used to increase the round window membrane mucoadhesive characteristics of the compositions or devices disclosed herein.

As disclosed herein, the round window membrane mucoadhesive agent is an alkyl-glycoside and/or a saccharide alkyl ester. As used herein, an "alkyl-glycoside" means a compound comprising any hydrophilic saccharide (e.g. sucrose, maltose, or glucose) linked to a hydrophobic alkyl. As disclosed herein , the round window membrane mucoadhesive agent is an alkyl-glycoside wherein the alkyl-glycoside comprises a sugar linked to a hydrophobic alkyl (e.g., an alkyl comprising about 6 to about 25 carbon atoms) by an amide linkage, an amine linkage, a carbamate linkage, an ether linkage, a thioether linkage, an ester linkage, a thioester linkage, a glycosidic linkage, a thioglycosidic linkage, and/or a ureide linkage. As disclosed herein , the round window membrane mucoadhesive agent is a hexyl-, heptyl-, octyl-, nonyl-, decyl-, undecyl-, dodecyl-, tridecyl-, tetradecyl, pentadecyl-, hexadecyl-, heptadecyl-, and octadecyl α- or β-D-maltoside; hexyl-, heptyl-, octyl-, nonyl-, decyl-, undecyl-, dodecyl-, tridecyl-, tetradecyl, pentadecyl-, hexadecyl-, heptadecyl-, and octadecyl α- or β-D-glucoside; hexyl-, heptyl-, octyl-, nonyl-, decyl-, undecyl-, dodecyl-, tridecyl-, tetradecyl, pentadecyl-, hexadecyl-, heptadecyl-, and octadecyl α- or β-D-sucroside; hexyl-, heptyl-, octyl-, dodecyl-, tridecyl-, and tetradecyl-β-D-thiomaltoside; dodecyl maltoside; heptyl- or octyl-1-thio-α- or β-D- glucopyranoside; alkyl thiosucroses; alkyl maltotriosides; long chain aliphatic carbonic acid amides of sucrose β-amino-alkyl ethers; derivatives of palatinose or isomaltamine linked by an amide linkage to an alkyl chain and derivatives of isomaltamine linked by urea to an alkyl chain; long chain aliphatic carbonic acid ureides of sucrose β-amino- alkyl ethers and long chain aliphatic carbonic acid amides of sucrose β- amino-alkyl ethers. As disclosed herein , the round window membrane mucoadhesive agent is an alkyl-glycoside wherein the alkyl glycoside is maltose, sucrose, glucose, or a combination thereof linked by a glycosidic linkage to an alkyl chain of 9-16 carbon atoms (e.g., nonyl-, decyl-, dodecyl- and tetradecyl sucroside; nonyl-, decyl-, dodecyl- and tetradecyl glucoside; and nonyl-, decyl-, dodecyl- and tetradecyl maltoside). As disclosed herein , the round window membrane mucoadhesive agent is an alkyl-glycoside wherein the alkyl glycoside is dodecylmaltoside, tridecylmaltoside, and tetradecylmaltoside.

As disclosed herein , the round window membrane mucoadhesive agent is an alkyl-glycoside wherein the alkyl-glycoside is a disaccharide with at least one glucose. As disclosed herein , the auris acceptable penetration enhancer is a surfactant comprising α-D-glucopyranosyl-β-glycopyranoside, n-Dodecyl-4-O-α- D-glucopyranosyl-β-glycopyranoside, and/or n-tetradecyl-4-O-α- D-glucopyranosyl-β-glycopyranoside. As disclosed herein , the round window membrane mucoadhesive agent is an alkyl-glycoside wherein the alkyl-glycoside has a critical miscelle concentration (CMC) of less than about 1mM in pure water or in aqueous solutions. As disclosed herein , the round window membrane mucoadhesive agent is an alkyl-glycoside wherein an oxygen atom within the alkyl-glycoside is substituted with a sulfur atom. As disclosed herein , the round window membrane mucoadhesive agent is an alkyl-glycoside wherein the alkylglycoside is the β anomer. As disclosed herein , the round window membrane mucoadhesive agent is an alkyl-glycoside wherein the alkylglycoside comprises 90%, 91%, 92%, 93%, 94%, 95%, 96%, 97%, 98%, 99%, 99.1%, 99.5%, or 99.9% of the β anomer.

**Auris-Acceptable Controlled Release Particles**

TrkB or TrkC agonists and/or other pharmaceutical agents disclosed herein are optionally incorporated within controlled release particles, lipid complexes, liposomes, nanoparticles, microparticles, microspheres, coacervates, nanocapsules or other agents which enhance or facilitate the localized delivery of the TrkB or TrkC agonists. As disclosed herein , a single enhanced viscosity formulation is used, in which at least one TrkB or TrkC agonist is present, while in other disclosures herein, a pharmaceutical formulation that comprises a mixture of two or more distinct enhanced viscosity formulations is used, in which at least TrkB or TrkC agonist is present. As disclosed herein , combinations of sols, gels and/or biocompatible matrices is also employed to provide desirable characteristics of the controlled release TrkB or TrkC agonist compositions or formulations. As disclosed herein, the controlled release TrkB or TrkC agonist formulations or compositions are cross-linked by one or more agents to alter or improve the properties of the composition.

Examples of microspheres relevant to the pharmaceutical formulations disclosed herein include: Luzzi, L. A., J. Pharm. Psy. 59:1367 (1970); U.S. Pat. No. 4,530,840; Lewis, D. H., "Controlled Release of Bioactive Agents from Lactides/Glycolide Polymers" in Biodegradable Polymers as Drug Delivery Systems, Chasin, M. and Langer, R., eds., Marcel Decker (1990); U.S. Pat. No. 4,675,189; Beck et al., "Poly(lactic acid) and Poly(lactic acid-co-glycolic acid) Contraceptive Delivery Systems," in Long Acting Steroid Contraception, Mishell, D. R., ed., Raven Press (1983); U.S. Pat. No. 4,758,435; U.S. Pat. No. 3,773,919; U.S. Pat. No. 4,474,572. Examples of protein therapeutics formulated as microspheres include: U.S. Pat. No. 6,458,387; U.S. Pat. No. 6,268,053; U.S. Pat. No. 6,090,925; U.S. Pat. No. 5,981,719; and U.S. Pat. No. 5,578,709.

Microspheres usually have a spherical shape, although irregularly-shaped microparticles are possible. Microspheres may vary in size, ranging from submicron to 1000 micron diameters. Microspheres suitable for use with the auris-acceptable formulations disclosed herein are submicron to 250 micron diameter microspheres, allowing administration by injection with a standard gauge needle. The auris-acceptable microspheres are prepared by any method which produces microspheres in a size range acceptable for use in an injectable composition. Injection is optionally accomplished with standard gauge needles used for administering liquid compositions.

Suitable examples of polymeric matrix materials for use in the auris-acceptable controlled release particles herein include poly(glycolic acid), poly-d,l-lactic acid, poly-l-lactic acid, copolymers of the foregoing, poly(aliphatic carboxylic acids), copolyoxalates, polycaprolactone, polydioxonene, poly(orthocarbonates), poly(acetals), poly(lactic acid-caprolactone), polyorthoesters, poly(glycolic acid-caprolactone), polydioxonene, polyanhydrides, polyphosphazines, and natural polymers including albumin, casein, and some waxes, such as, glycerol mono- and distearate, and the like. Various commercially available poly (lactide-co-glycolide) materials (PLGA) are optionally used in the method disclosed herein. For example, poly (d,l-lactic-co-glycolic acid) is commercially available from Boehringer-Ingelheim as RESOMER RG 503 H. This product has a mole percent composition of 50% lactide and 50% glycolide. These copolymers are available in a wide range of molecular weights and ratios of lactic acid to glycolic acid. One disclosure includes the use of the polymer poly(d,l-lactide-co-glycolide). The molar ratio of lactide to glycolide in such a copolymer includes the range of from about 95:5 to about 50:50.

The molecular weight of the polymeric matrix material is of some importance. The molecular weight should be high enough so that it forms satisfactory polymer coatings, i.e., the polymer should be a good film former. Usually, a satisfactory molecular weight is in the range of 5,000 to 500,000 daltons. The molecular weight of a polymer is also important from the point of view that molecular weight influences the biodegradation rate of the polymer. For a diffusional mechanism of drug release, the polymer should remain intact until all of the drug is released from the microparticles and then degrade. The TrkB or TrkC agonist is also released from the microparticles as the polymeric excipient bioerodes. By an appropriate selection of polymeric materials a microsphere formulation is made such that the resulting microspheres exhibit both diffusional release and biodegradation release properties. This is useful in affording multiphasic release patterns.

A variety of methods are known by which compounds are encapsulated in microspheres. In these methods, the TrkB or TrkC agonist is generally dispersed or emulsified, using stirrers, agitators, or other dynamic mixing techniques, in a solvent containing a wall-forming material. Solvent is then removed from the microspheres, and thereafter the microsphere product is obtained.

As disclosed herein , controlled release TrkB or TrkC agonist formulations are made through the incorporation of the TrkB or TrkC agonists and/or other pharmaceutical agents into ethylene-vinyl acetate copolymer matrices. (*See* U.S. Patent No. 6,083,534, incorporated herein for such disclosure). As disclosed herein, TrkB or TrkC agonists are incorporated into poly (lactic-glycolic acid) or poly-L-lactic acid microspheres. *Id.* In yet another disclosure, the TrkB or TrkC agonists are encapsulated into alginate microspheres. (*See* U.S. Patent No. 6,036,978, incorporated herein for such disclosure). Biocompatible methacrylate-based polymers to encapsulate the TrkB or TrkC agonist compounds or compositions are optionally used in the formulations and methods disclosed herein. A wide range of methacrylate-based polymer systems are commercially available, such as the EUDRAGIT polymers marketed by Evonik. One useful aspect of methacrylate polymers is that the properties of the formulation are varied by incorporating various copolymers. For

example, poly(acrylic acid-co-methylmethacrylate) microparticles exhibit enhanced mucoadhesion properties as the carboxylic acid groups in the poly(acrylic acid) form hydrogen bonds with mucin (Park etal, Pharm. Res. (1987) 4(6):457-464). Variation of the ratio between acrylic acid and methylmethacrylate monomers serves to modulate the properties of the co-polymer. Methacrylate-based microparticles have also been used in protein therapeutic formulations (Naha et al, Journal of Microencapsulation 04 February, 2008 (online publication)). As disclosed herein , the enhanced viscosity auris-acceptable formulations described herein comprises TrkB or TrkC agonist microspheres wherein the microspheres are formed from a methacrylate polymer or copolymer. As disclosed herein, the enhanced viscosity formulation described herein comprises TrkB or TrkC agonist microspheres wherein the microspheres are mucoadhesive. Other controlled release systems, including incorporation or deposit of polymeric materials or matrices onto solid or hollow spheres containing TrkB or TrkC agonists, are discosed herein The types of controlled release systems available without significantly losing activity of the TrkB or TrkC agonists are determined using the teachings, examples, and principles disclosed herein

An example of a conventional microencapsulation process for pharmaceutical preparations is shown in U.S. Pat. No. 3,737,337. The TrkB or TrkC agonist substances to be encapsulated or embedded are dissolved or dispersed in the organic solution of the polymer (phase A), using conventional mixers, including (in the preparation of dispersion) vibrators and high-speed stirrers, etc. The dispersion of phase (A), containing the core material in solution or in suspension, is carried out in the aqueous phase (B), again using conventional mixers, such as high-speed mixers, vibration mixers, or even spray nozzles, in which case the particle size of the microspheres will be determined not only by the concentration of phase (A), but also by the emulsate or microsphere size. With conventional techniques for the microencapsulation of TrkB or TrkC agonists, the microspheres form when the solvent containing an active agent and a polymer is emulsified or dispersed in an immiscible solution by stirring, agitating, vibrating, or some other dynamic mixing technique, often for a relatively long period of time.

Methods for the construction of microspheres are also described in U.S. Pat. No. 4,389,330, and U.S. Pat. No. 4,530,840.. The desired TrkB or TrkC agonist is dissolved or dispersed in an appropriate solvent. To the agent-containing medium is added the polymeric matrix material in an amount relative to the active ingredient which gives a product of the desired loading of TrkB or TrkC agonist. Optionally, all of the ingredients of the TrkB or TrkC agonist microsphere product can be blended in the solvent medium together. Suitable solvents for the agonist and the polymeric matrix material include organic solvents such as acetone, halogenated hydrocarbons such as chloroform, methylene chloride and the like, aromatic hydrocarbon compounds, halogenated aromatic hydrocarbon compounds, cyclic ethers, alcohols, ethyl acetate and the like.

The mixture of ingredients in the solvent is emulsified in a continuous-phase processing medium; the continuous-phase medium being such that a dispersion of microdroplets containing the indicated ingredients is formed in the continuous-phase medium. Naturally, the continuous-phase processing medium and the organic solvent must be immiscible, and includes water although nonaqueous media such as xylene and toluene and synthetic oils and natural oils are optionally used. Optionally, a surfactant is added to the continuous-phase processing medium to prevent the microparticles from agglomerating and to control the size of the solvent microdroplets in the emulsion. A preferred surfactant-dispersing medium combination is a 1 to 10 wt. % poly (vinyl alcohol) in water mixture. The dispersion is formed by mechanical agitation of the mixed materials. An emulsion is optionally formed by adding small drops of the TrkB or TrkC agonist-wall forming material solution to the continuous phase processing medium. The temperature during the formation of the emulsion is not especially critical but influences the size and quality of the microspheres and the solubility of the drug in the continuous phase. It is desirable to have as little of the TrkB or TrkC agonist in the continuous phase as possible. Moreover, depending on the solvent and continuous-phase processing medium employed, the temperature must not be too low or the solvent and processing medium will solidify or the processing medium will become too viscous for practical purposes, or too high that the processing medium will evaporate, or that the liquid processing medium will not be maintained. Moreover, the temperature of the medium cannot be so high that the stability of the particular agent being incorporated in the microspheres is adversely affected. Accordingly, the dispersion process is conducted at any temperature which maintains stable operating conditions, which preferred temperature being about 15 °C to 60 °C, depending upon the drug and excipient selected.

The dispersion which is formed is a stable emulsion and from this dispersion the organic solvent immiscible fluid is optionally partially removed in the first step of the solvent removal process. The solvent is removed by techniques such as heating, the application of a reduced pressure or a combination of both. The temperature employed to evaporate solvent from the microdroplets is not critical, but should not be that high that it degrades the TrkB or TrkC agonist employed in the preparation of a given microparticle, nor should it be so high as to evaporate solvent at such a rapid rate to cause defects in the wall forming material. Generally, from 5 to 75%, of the solvent is removed in the first solvent removal step.

After the first stage, the dispersed microparticles in the solvent immiscible fluid medium are isolated from the fluid medium by any convenient means of separation. Thus, for example, the fluid is decanted from the microsphere or the microsphere suspension is filtered. Still other, various combinations of separation techniques are used if desired.

Following the isolation of the microspheres from the continuous-phase processing medium, the remainder of the solvent in the microspheres is removed by extraction. In this step, the microspheres are suspended in the same continuous-phase processing medium used in step one, with or without surfactant, or in another liquid. The extraction medium removes the solvent from the microspheres and yet does not dissolve the microspheres. During the extraction, the extraction medium with dissolved solvent is optionally removed and replaced with fresh extraction medium. This is best done on a continual basis. The rate of extraction medium replenishment of a given process is a variable which is determined at the time the process is performed and, therefore, no precise limits for the rate must be predetermined. After the majority of the solvent has been removed from the microspheres, the microspheres are dried by exposure to air or by other conventional drying techniques such as vacuum drying, drying over a desiccant, or the like. This process is very efficient in encapsulating the TrkB or TrkC agonist since core loadings of up to 80 wt. %, preferably up to 60 wt. % are obtained.

Alternatively, controlled release microspheres containing TrkB or TrkC agonist is prepared through the use of static mixers. Static or motionless mixers consist of a conduit or tube in which is received a number of static mixing agents. Static mixers provide homogeneous mixing in a relatively short length of conduit, and in a relatively short period of time. With static mixers, the fluid moves through the mixer, rather than some part of the mixer, such as a blade, moving through the fluid.

A static mixer is optionally used to create an emulsion. When using a static mixer to form an emulsion, several factors determine emulsion particle size, including the density and viscosity of the various solutions or phases to be mixed, volume ratio of the phases, interfacial tension between the phases, static mixer parameters (conduit diameter; length of mixing element; number of mixing elements), and linear velocity through the static mixer. Temperature is a variable because it affects density, viscosity, and interfacial tension. The controlling variables are linear velocity, sheer rate, and pressure drop per unit length of static mixer.

In order to create microspheres containing TrkB or TrkC agonist using a static mixer process, an organic phase and an aqueous phase are combined. The organic and aqueous phases are largely or substantially immiscible, with the aqueous phase constituting the continuous phase of the emulsion. The organic phase includes TrkB or TrkC agonist as well as a wall-forming polymer or polymeric matrix material. The organic phase is prepared by dissolving a TrkB or TrkC agonist in an organic or other suitable solvent, or by forming a dispersion or an emulsion containing the TrkB or TrkC agonist. The organic phase and the aqueous phase are pumped so that the two phases flow simultaneously through a static mixer, thereby forming an emulsion which comprises microspheres containing the TrkB or TrkC agonist encapsulated in the polymeric matrix material. The organic and aqueous phases are pumped through the static mixer into a large volume of quench liquid to extract or remove the organic solvent. Organic solvent is optionally removed from the microspheres while they are washing or being stirred in the quench liquid. After the microspheres are washed in a quench liquid, they are isolated, as through a sieve, and dried.

As disclosed herein , microspheres are prepared using a static mixer. The process is not limited to the solvent extraction technique discussed above, but is used with other encapsulation techniques. For example, the process is optionally used with a phase separation encapsulation technique. To do so, an organic phase is prepared that comprises a TrkB or TrkC agonist suspended or dispersed in a polymer solution. The non-solvent second phase is free from solvents for the polymer and active agent. A preferred non-solvent second phase is silicone oil. The organic phase and the non-solvent phase are pumped through a static mixer into a non-solvent quench liquid, such as heptane. The semi-solid particles are quenched for complete hardening and washing. The process of microencapsulation includes spray drying, solvent evaporation, a combination of evaporation and extraction, and melt extrusion.

As disclosed herein, the microencapsulation process involves the use of a static mixer with a single solvent. This process is described in detail in U.S. application Ser. No. 08/338,805. An alternative process involves the use of a static mixer with cosolvents. In this process, biodegradable microspheres comprising a biodegradable polymeric binder and a TrkB or TrkC agonist are prepared, which comprises a blend of at least two substantially non-toxic solvents, free of halogenated hydrocarbons to dissolve both the agent and the polymer. The solvent blend containing the dissolved agent and polymer is dispersed in an aqueous solution to form droplets. The resulting emulsion is then added to an aqueous extraction medium preferably containing at least one of the solvents of the blend, whereby the rate of extraction of each solvent is controlled, whereupon the biodegradable microspheres containing the TrkB or TrkC agonist are formed. This process has the advantage that less extraction medium is required because the solubility of one solvent in water is substantially independent of the other and solvent selection is increased, especially with solvents that are particularly difficult to extract.

Nanoparticles are also contemplated for use with the TrkB or TrkC agonists disclosed herein. Nanoparticles are material structures of about 100 nm or less in size. One use of nanoparticles in pharmaceutical formulations is the formation of suspensions as the interaction of the particle surface with solvent is strong enough to overcome differences in density. Nanoparticle suspensions are sterilized as the nanoparticles are small enough to be subjected to sterilizing filtration (see, e.g., U.S. Patent No. 6,139,870). Nanoparticles comprise at least one hydrophobic, water-insoluble and water-indispersible polymer or copolymer emulsified in a solution or aqueous dispersion of surfactants, phospholipids

or fatty acids. The TrkB or TrkC agonist is optionally introduced with the polymer or the copolymer into the nanoparticles.

Lipid nanocapsules as controlled release structures, as well for penetrating the round window membrane and reaching auris interna and/or auris media targets, is also contemplated herein. Lipid nanocapsules are optionally formed by emulsifying capric and caprylic acid triglycerides (Labrafac WL 1349; avg. mw 512), soybean lecithin (LIPOID® S75-3; 69% phosphatidylcholine and other phospholipids), surfactant (for example, Solutol HS15), a mixture of polyethylene glycol 660 hydroxystearate and free polyethylene glycol 660; NaCl and water. The mixture is stirred at room temperature to obtain an oil emulsion in water. After progressive heating at a rate of 4 °C/min under magnetic stirring, a short interval of transparency should occur close to 70 °C, and the inverted phase (water droplets in oil) obtained at 85 °C. Three cycles of cooling and heating is then applied between 85 °C and 60 °C at the rate of 4 °C/min, and a fast dilution in cold water at a temperature close to 0 °C to produce a suspension of nanocapsules. To encapsulate the TrkB or TrkC agonist, the agonist is optionally added just prior to the dilution with cold water.

TrkB or TrkC agonists are also inserted into the lipid nanocapsules by incubation for 90 minutes with an aqueous micellar solution of the TrkB or TrkC agonist. The suspension is then vortexed every 15 minutes, and then quenched in an ice bath for 1 minute.

Suitable auris-acceptable surfactants are, by way of example, cholic acid or taurocholic acid salts. Taurocholic acid, the conjugate formed from cholic acid and taurine, is a fully metabolizable sulfonic acid surfactant. An analog of taurocholic acid, tauroursodeoxycholic acid (TUDCA), is a naturally occurring bile acid and is a conjugate of taurine and ursodeoxycholic acid (UDCA). Other naturally occurring anionic (e.g., galactocerebroside sulfate), neutral (e.g., lactosylceramide) or zwitterionic surfactants (e.g., sphingomyelin, phosphatidyl choline, palmitoyl carnitine) are optionally used to prepare nanoparticles.

The auris-acceptable phospholipids are chosen, by way of example, from natural, synthetic or semi-synthetic phospholipids; lecithins (phosphatidylcholine) such as, for example, purified egg or soya lecithins (lecithin E100, lecithin E80 and phospholipons, for example phospholipon 90), phosphatidylethanolamine, phosphatidylserine, phosphatidylinositol, phosphatidylglycerol, dipalmitoylphosphatidylcholine, dipalmitoylglycerophosphatidylcholine, dimyristoylphosphatidylcholine, distearoylphosphatidylcholine and phosphatidic acid or mixtures thereof are used more particularly.

Fatty acids for use with the auris-acceptable formulations are chosen from, by way of example, lauric acid, mysristic acid, palmitic acid, stearic acid, isostearic acid, arachidic acid, behenic acid, oleic acid, myristoleic acid, palmitoleic acid, linoleic acid, alpha-linoleic acid, arachidonic acid, eicosapentaenoic acid, erucic acid, docosahexaenoic acid, and the like.

Suitable auris-acceptable surfactants are selected from known organic and inorganic pharmaceutical excipients. Such excipients include various polymers, low molecular weight oligomers, natural products, and surfactants. Preferred surface modifiers include nonionic and ionic surfactants. Two or more surface modifiers are used in combination.

Representative examples of auris-acceptable surfactants include cetyl pyridinium chloride, gelatin, casein, lecithin (phosphatides), dextran, glycerol, gum acacia, cholesterol, tragacanth, stearic acid, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters; dodecyl trimethyl ammonium bromide, polyoxyethylenestearates, colloidal silicon dioxide, phosphates, sodium dodecylsulfate, carboxymethylcellulose calcium, hydroxypropyl cellulose (HPC, HPC-SL, and HPC-L), hydroxypropyl methylcellulose (HPMC), carboxymethylcellulose sodium, methylcellulose, hydroxyethylcellulose, hydroxypropylcellulose, hydroxypropylmethyl-cellulose phthalate, noncrystalline cellulose, magnesium aluminum silicate, triethanolamine, polyvinyl alcohol (PVA), polyvinylpyrrolidone (PVP), 4-(1,1,3,3-tetaamethylbutyl)-phenol polymer with ethylene oxide and formaldehyde (also known as tyloxapol, superione, and triton), poloxamers, poloxamnines, a charged phospholipid such as dimyristoyl phophatidyl glycerol, dioctylsulfosuccinate (DOSS); Tetronic® 1508, dialkylesters of sodium sulfosuccinic acid, Duponol P, Tritons X-200, Crodestas F-110, p-isononylphenoxypoly-(glycidol), Crodestas SL-40 (Croda, Inc.); and SA9OHCO, which is $C_{18}$ $H_{37}$ $CH_2$ $(CON(CH_3)$-$CH_2$ $(CHOH)_4$ $(CH_2$ $OH)_2$ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucarmide; n-octyl-β-D-glucopyranoside; octyl β-D-thioglucopyranoside; and the like. Most of these surfactants are known pharmaceutical excipients and are described in detail in the Handbook of Pharmaceutical Excipients, published jointly by the American Pharmaceutical Association and The Pharmaceutical Society of Great Britain (The Pharmaceutical Press, 1986),.

The hydrophobic, water-insoluble and water-indispersible polymer or copolymer may be chosen from biocompatible and biodegradable polymers, for example lactic or glycolic acid polymers and copolymers thereof, or polylactic/polyethylene (or polypropylene) oxide copolymers, preferably with molecular weights of between 1000 and 200,000, polyhydroxybutyric acid polymers, polylactones of fatty acids containing at least 12 carbon atoms, or polyanhydrides.

The nanoparticles may be obtained by coacervation, or by the technique of evaporation of solvent, from an aqueous dispersion or solution of phospholipids and of an oleic acid salt into which is added an immiscible organic phase comprising the active principle and the hydrophobic, water-insoluble and water-indispersible polymer or copolymer. The

mixture is pre-emulsified and then subjected to homogenization and evaporation of the organic solvent to obtain an aqueous suspension of very small-sized nanoparticles.

A variety of methods are optionally employed to fabricate the TrkB or TrkC agonist nanoparticles are disclosed herein but not claimed. These methods include vaporization methods, such as free jet expansion, laser vaporization, spark erosion, electro explosion and chemical vapor deposition; physical methods involving mechanical attrition (e.g., "pearlmilling" technology, Elan Nanosystems), super critical CO2 and interfacial deposition following solvent displacement. As disclosed herein , the solvent displacement method is used. The size of nanoparticles produced by this method is sensitive to the concentration of polymer in the organic solvent; the rate of mixing; and to the surfactant employed in the process. Continuous flow mixers provide the necessary turbulence to ensure small particle size. One type of continuous flow mixing device that is optionally used to prepare nanoparticles has been described (Hansen et al J Phys Chem 92, 2189-96, 1988). As disclosed hereinultrasonic devices, flow through homogenizers or supercritical CO2 devices may be used to prepare nanoparticles.

If suitable nanoparticle homogeneity is not obtained on direct synthesis, then size-exclusion chromatography is used to produce highly uniform drug-containing particles that are freed of other components involved in their fabrication. Size-exclusion chromatography (SEC) techniques, such as gel- filtration chromatography, is used to separate particle-bound TrkB or TrkC agonist or other pharmaceutical compound from free TrkB or TrkC agonist or other pharmaceutical compound, or to select a suitable size range of TrkB or TrkC agonist -containing nanoparticles. Various SEC media, such as Superdex 200, Superose 6, Sephacryl 1000 are commercially available and are employed for the size-based fractionation of such mixtures. Additionally, nanoparticles are optionally purified by centrifugation, membrane filtration and by use of other molecular sieving devices, crosslinked gels/materials and membranes.

**Auris-Acceptable Cyclodextrin and Other Stabilizing Formulations**

As discosed herein, the auris-acceptable formulations alternatively comprises a cyclodextrin. Cyclodextrins are cyclic oligosaccharides containing 6, 7, or 8 glucopyranose units, referred to as α-cyclodextrin, β-cyclodextrin, or γ-cyclodextrin respectively. Cyclodextrins have a hydrophilic exterior, which enhances water-soluble, and a hydrophobic interior which forms a cavity. In an aqueous environment, hydrophobic portions of other molecules often enter the hydrophobic cavity of cyclodextrin to form inclusion compounds. Additionally, cyclodextrins are also capable of other types of nonbonding interactions with molecules that are not inside the hydrophobic cavity. Cyclodextrins have three free hydroxyl groups for each glucopyranose unit, or 18 hydroxyl groups on α-cyclodextrin, 21 hydroxyl groups on β-cyclodextrin, and 24 hydroxyl groups on γ-cyclodextrin. One or more of these hydroxyl groups can be reacted with any of a number of reagents to form a large variety of cyclodextrin derivatives, including hydroxypropyl ethers, sulfonates, and sulfoalkylethers. Shown below is the structure of β-cyclodextrin and the hydroxypropyl-β-cyclodextrin (HPβCD).

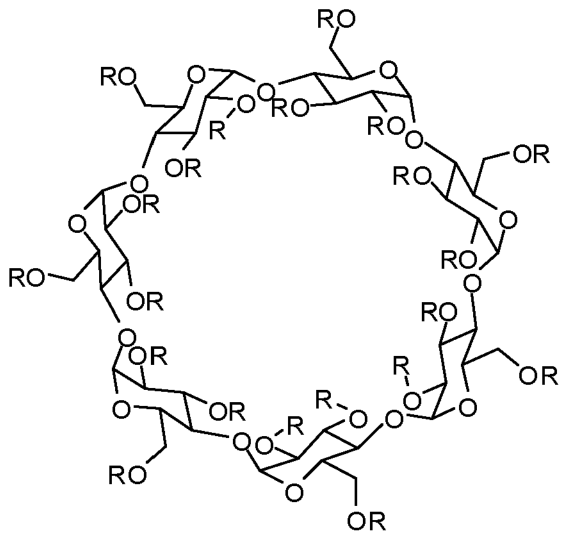

R = H
?-cyclodextrin

R = $CH_2CH(OH)CH_3$
hydroxypropyl ?-cyclodextrin

As disclosed herein , the use of cyclodextrins in the pharmaceutical compositions described herein improves the solubility of the drug. Inclusion compounds are involved in many cases of enhanced solubility; however other interactions between cyclodextrins and insoluble compounds also improves solubility. Hydroxypropyl-β-cyclodextrin (HPβCD) is commercially available as a pyrogen free product. It is a nonhygroscopic white powder that readily dissolves in water. HPβCD is thermally stable and does not degrade at neutral pH. Thus, cyclodextrins improve the solubility of a therapeutic agent in a composition or formulation. Accordingly, As disclosed herein , cyclodextrins are included to increase the

solubility of the auris-acceptable TrkB or TrkC agonists within the formulations described herein. Cyclodextrins in addition serve as controlled release excipients within the formulations described herein.

By way of example only, cyclodextrin derivatives for use include α-cyclodextrin, β-cyclodextrin, γ-cyclodextrin, hydroxyethyl β-cyclodextrin, hydroxypropyl γ-cyclodextrin, sulfated β-cyclodextrin, sulfated α-cyclodextrin, sulfobutyl ether β-cyclodextrin.

The concentration of the cyclodextrin used in the compositions and methods disclosed herein varies according to the physiochemical properties, pharmacokinetic properties, side effect or adverse events, formulation considerations, or other factors associated with the therapeutically active agent, or a salt or prodrug thereof, or with the properties of other excipients in the composition. Thus, in certain circumstances, the concentration or amount of cyclodextrin used in accordance with the compositions and methods disclosed herein will vary, depending on the need. When used, the amount of cyclodextrins needed to increase solubility of the TrkB or TrK agonist and/or function as a controlled release excipient in any of the formulations described herein is selected using the principles, examples, and teachings described herein.

Other stabilizers that are useful in the auris-acceptable formulations disclosed herein include, for example, fatty acids, fatty alcohols, alcohols, long chain fatty acid esters, long chain ethers, hydrophilic derivatives of fatty acids, polyvinyl pyrrolidones, polyvinyl ethers, polyvinyl alcohols, hydrocarbons, hydrophobic polymers, moisture-absorbing polymers, and combinations thereof. As disclosed herein , amide analogues of stabilizers are also used. As disclosed herein, the chosen stabilizer changes the hydrophobicity of the formulation (e.g., oleic acid, waxes), or improves the mixing of various components in the formulation (e.g., ethanol), controls the moisture level in the formula (e.g., PVP or polyvinyl pyrrolidone), controls the mobility of the phase (substances with melting points higher than room temperature such as long chain fatty acids, alcohols, esters, ethers, amides etc. or mixtures thereof; waxes), and/or improves the compatibility of the formula with encapsulating materials (e.g., oleic acid or wax). As disclosed herein some of these stabilizers are used as solvents/co-solvents (e.g., ethanol). As disclosed herein are, stabilizers which are present in sufficient amounts to inhibit the degradation of the TrkB or TrkC agonist. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (l) pentosan polysulfate and other heparinoids, (m) divalent cations such as magnesium and zinc; or (n) combinations thereof.

Additional useful TrkB or TrkC agonist auris-acceptable formulations include one or more anti-aggregation additives to enhance stability of TrkB or TrkC agonistformulations by reducing the rate of protein aggregation. The anti-aggregation additive selected depends upon the nature of the conditions to which the TrkB or TrkC agonist, for example TrkB or TrkC agonist antibodies are exposed. For example, certain formulations undergoing agitation and thermal stress require a different anti-aggregation additive than a formulation undergoing lyophilization and reconstitution. Useful anti-aggregation additives include, by way of example only, urea, guanidinium chloride, simple amino acids such as glycine or arginine, sugars, polyalcohols, polysorbates, polymers such as polyethylene glycol and dextrans, alkyl saccharides, such as alkyl glycoside, and surfactants.

Other useful formulations optionally include one or more auris-acceptable antioxidants to enhance chemical stability where required. Suitable antioxidants include, by way of example only, ascorbic acid, methionine, sodium thiosulfate and sodium metabisulfite. As disclosed herein , antioxidants are selected from metal chelating agents, thiol containing compounds and other general stabilizing agents.

Still other useful compositions include one or more auris-acceptable surfactants to enhance physical stability or for other purposes. Suitable nonionic surfactants include, but are not limited to, polyoxyethylene fatty acid glycerides and vegetable oils, e.g., polyoxyethylene (60) hydrogenated castor oil; and polyoxyethylene alkylethers and alkylphenyl ethers, e.g., octoxynol 10, octoxynol 40.

As disclosed herein , the auris-acceptable pharmaceutical formulations described herein are stable with respect to compound degradation over a period of any of at least about 1 day, at least about 2 days, at least about 3 days, at least about 4 days, at least about 5 days, at least about 6 days, at least about 1 week, at least about 2 weeks, at least about 3 weeks, at least about 4 weeks, at least about 5 weeks, at least about 6 weeks, at least about 7 weeks, at least about 8 weeks, at least about 3 months, at least about 4 months, at least about 5 months, or at least about 6 months. The formulations described herein are stable with respect to compound degradation over a period of at least about 1 week. Also described herein are formulations that are stable with respect to compound degradation over a period of at least about 1 month.

disclosed but not claimed is that an additional surfactant (co-surfactant) and/or buffering agent is combined with one or more of the pharmaceutically acceptable vehicles previously described herein so that the surfactant and/or buffering agent maintains the product at an optimal pH for stability. Suitable co-surfactants include, but are not limited to: a) natural and synthetic lipophilic agents, e.g., phospholipids, cholesterol, and cholesterol fatty acid esters and derivatives thereof; b) nonionic surfactants, which include for example, polyoxyethylene fatty alcohol esters, sorbitan

fatty acid esters (Spans), polyoxyethylene sorbitan fatty acid esters (e.g., polyoxyethylene (20) sorbitan monooleate (Tween 80), polyoxyethylene (20) sorbitan monostearate (Tween 60), polyoxyethylene (20) sorbitan monolaurate (Tween 20) and other Tweens, sorbitan esters, glycerol esters, e.g., Myrj and glycerol triacetate (triacetin), polyethylene glycols, cetyl alcohol, cetostearyl alcohol, stearyl alcohol, polysorbate 80, poloxamers, poloxamines, polyoxyethylene castor oil derivatives (e.g., Cremophor® RH40, Cremphor A25, Cremphor A20, Cremophor® EL) and other Cremophors, sulfosuccinates, alkyl sulphates (SLS); PEG glyceryl fatty acid esters such as PEG-8 glyceryl caprylate/caprate (Labrasol), PEG-4 glyceryl caprylate/caprate (Labrafac Hydro WL 1219), PEG-32 glyceryl laurate (Gelucire 444/14), PEG-6 glyceryl mono oleate (Labrafil M 1944 CS), PEG-6 glyceryl linoleate (Labrafil M 2125 CS); propylene glycol mono- and di-fatty acid esters, such as propylene glycol laurate, propylene glycol caprylate/caprate; Brij® 700, ascorbyl-6-palmitate, stearylamine, sodium lauryl sulfate, polyoxethyleneglycerol triiricinoleate, and any combinations or mixtures thereof; c) anionic surfactants include, but are not limited to, calcium carboxymethylcellulose, sodium carboxymethylcellulose, sodium sulfosuccinate, dioctyl, sodium alginate, alkyl polyoxyethylene sulfates, sodium lauryl sulfate, triethanolamine stearate, potassium laurate, bile salts, and any combinations or mixtures thereof; and d) cationic surfactants such as cetyltrimethylammonium bromide, and lauryldimethylbenzyl-ammonium chloride.

As disclosed herein, when one or more co-surfactants are utilized in the auris-acceptable formulations of the present disclosure, they are combined, e.g., with a pharmaceutically acceptable vehicle and is present in the final formulation, e.g., in an amount ranging from about 0.1% to about 20%, from about 0.5% to about 10%.

As disclosed herein, the surfactant has an HLB value of 0 to 20. As disclosed herein, the surfactant has an HLB value of 0 to 3, of 4 to 6, of 7 to 9, of 8 to 18, of 13 to 15, of 10 to 18.

As disclosed herein, diluents are also used to stabilize the TrkB or TrkC agonist or other pharmaceutical compounds because they provide a more stable environment. Salts dissolved in buffered solutions (which also can provide pH control or maintenance) are utilized as diluents, including, but not limited to a phosphate buffered saline solution. As disclosed herein, the gel formulation is isotonic with the endolymph or the perilymph: depending on the portion of the cochlea that the TrkB or TrkC agonist formulation is targeted. Isotonic formulations are provided by the addition of a tonicity agent. Suitable tonicity agents include, but are not limited to any pharmaceutically acceptable sugar, salt or any combinations or mixtures thereof, such as, but not limited to dextrose and sodium chloride. As disclosed herein , the tonicity agents are present in an amount from about 100 mOsm/kg to about 500 mOsm/kg. As disclosed herein , the tonicity agent is present in an amount from about 200 mOsm/kg to about 400 mOsm/kg, from about 280 mOsm/kg to about 320 mOsm/kg. The amount of tonicity agents will depend on the target structure of the pharmaceutical formulation, as described herein.

Useful tonicity compositions also include one or more salts in an amount required to bring osmolality of the composition into an acceptable range for the perilymph or the endolymph. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

As disclosed herein , the auris-acceptable gel formulations disclosed herein alternatively or additionally contains preservatives to prevent microbial growth. Suitable auris-acceptable preservatives for use in the enhanced viscosity formulations described herein include, but are not limited to benzoic acid, boric acid, p-hydroxybenzoates, alcohols, quarternary compounds, stabilized chlorine dioxide, mercurials, such as merfen and thiomersal, mixtures of the foregoing and the like.

As disclosed herein, the preservative is, by way of example only, an antimicrobial agent, within the auris-acceptable formulations presented herein. As disclosed herein , the formulation includes a preservative such as by way of example only, methyl paraben, sodium bisulfite, sodium thiosulfate, ascorbate, chorobutanol, thimerosal, parabens, benzyl alcohol, phenylethanol and others. As disclosed herein, the methyl paraben is at a concentration of about 0.05% to about 1.0%, about 0.1% to about 0.2%. As disclosed herein, the gel is prepared by mixing water, methylparaben, hydroxyethylcellulose and sodium citrate. As disclosed herein, the gel is prepared by mixing water, methylparaben, hydroxyethylcellulose and sodium acetate. As disclosed herein, the mixture is sterilized by autoclaving at 120 °C for about 20 minutes, and tested for pH, methylparaben concentration and viscosity before mixing with the appropriate amount of the TrkB or TrkC agonist disclosed herein.

Suitable auris-acceptable water soluble preservatives which are employed in the drug delivery vehicle include sodium bisulfite, sodium thiosulfate, ascorbate, chorobutanol, thimerosal, parabens, benzyl alcohol, Butylated hydroxytoluene (BHT), phenylethanol and others. These agents are present, generally, in amounts of about 0.001% to about 5% by weight and, preferably, in the amount of about 0.01 to about 2% by weight. As disclosed herein , auris-compatible formulations described herein are free of preservatives.

**Round window membrane Penetration Enhancers**

As disclosed herein, the formulation further comprises one or more round window membrane penetration

enhancers. Penetration across the round window membrane is enhanced by the presence of round window membrane penetration enhancers. Round window membrane penetration enhancers are chemical entities that facilitate transport of coadministered substances across the round window membrane. Round window membrane penetration enhancers are grouped according to chemical structure. Surfactants, both ionic and non-ionic, such as sodium lauryl sulfate, sodium laurate, polyoxyethylene-20-cetyl ether, laureth-9, sodium dodecylsulfate, dioctyl sodium sulfosuccinate, polyoxyethylene-9-lauryl ether (PLE), Tween® 80, nonylphenoxypolyethylene (NP-POE), polysorbates and the like, function as round window membrane penetration enhancers. Bile salts (such as sodium glycocholate, sodium deoxycholate, sodium taurocholate, sodium taurodihydrofusidate, sodium glycodihydrofusidate and the like), fatty acids and derivatives (such as oleic acid, caprylic acid, mono- and di-glycerides, lauric acids, acylcholines, caprylic acids, acylcarnitines, sodium caprates and the like), chelating agents (such as EDTA, citric acid, salicylates and the like), sulfoxides (such as dimethyl sulfoxide (DMSO), decylmethyl sulfoxide and the like), and alcohols (such as ethanol, isopropanol, glycerol, propanediol and the like) also function as round window membrane penetration enhancers.

As disclosed herein , the auris acceptable penetration enhancer is a surfactant comprising an alkyl-glycoside wherein the alkyl glycoside is tetradecyl- β-D-maltoside. As disclosed herein , the auris acceptable penetration enhancer is a surfactant comprising an alkyl-glycoside wherein the alkyl glycoside is dodecyl-maltoside. In certain instances, the penetration enhancing agent is a hyaluronidase. In certain instances, a hyaluronidase is a human or bovine hyaluronidase. In some instances, a hyaluronidase is a human hyaluronidase (e.g., hyaluronidase found in human sperm, PH20 (Halozyme), Hyelenex® (Baxter International, Inc.)). In some instances, a hyaluronidase is a bovine hyaluronidase (e.g., bovine testicular hyaluronidase, Amphadase® (Amphastar Pharmaceuticals), Hydase® (PrimaPharm, Inc). In some instances, a hyluronidase is an ovine hyaluronidase, Vitrase® (ISTA Pharmaceuticals). In certain instances, a hyaluronidase described herein is a recombinant hyaluronidase. In some instances, a hyaluronidase described herein is a humanized recombinant hyaluronidase. In some instances, a hyaluronidase described herein is a pegylated hyaluronidase (e.g., PEGPH20 (Halozyme)). In addition, the peptide-like penetration enhancers described in U.S. Patent Nos. 7,151,191, 6,221,367 and 5,714,167.These penetration enhancers are amino-acid and peptide derviatives and enable drug absorption by passive transcellular diffusion without affecting the integrity of membranes or intercellular tight junctions.

**Round window membrane Permeable Liposomes**

Liposomes or lipid particles may also be employed to encapsulate the TrkB or TrkC agonist formulations or compositions. Phospholipids that are gently dispersed in an aqueous medium form multilayer vesicles with areas of entrapped aqueous media separating the lipid layers. Sonication, or turbulent agitation, of these multilayer vesicles results in the formation of single layer vesicles, commonly referred to as liposomes, with sizes of about 10-1000 nm. These liposomes have many advantages as TrkB or TrkC agonists or other pharmaceutical agent carriers. They are biologically inert, biodegradable, non-toxic and non-antigenic. Liposomes are formed in various sizes and with varying compositions and surface properties. Additionally, they are able to entrap a wide variety of agents and release the agent at the site of liposome collapse.

Suitable phospholipids for use in auris-acceptable liposomes here are, for example, phosphatidyl cholines, ethanolamines and serines, sphingomyelins, cardiolipins, plasmalogens, phosphatidic acids and cerebrosides, in particular those which are soluble together with the TrkB or TrkC agonists herein in non-toxic, pharmaceutically acceptable organic solvents. Preferred phospholipids are, for example, phosphatidyl choline, phosphatidyl ethanolmine, phosphatidyl serine, phosphatidyl inositol, lysophosphatidyl choline, phosphatidyl glycerol and the like, and mixtures thereof especially lecithin, e.g. soya lecithin. The amount of phospholipid used in the present formulation range from about 10 to about 30%, preferably from about 15 to about 25% and in particular is about 20%.

Lipophilic additives may be employed advantageously to modify selectively the characteristics of the liposomes. Examples of such additives include by way of example only, stearylamine, phosphatidic acid, tocopherol, cholesterol, cholesterol hemisuccinate and lanolin extracts. The amount of lipophilic additive used range from 0.5 to 8%, preferably from 1.5 to 4% and in particular is about 2%. Generally, the ratio of the amount of lipophilic additive to the amount of phospholipid ranges from about 1:8 to about 1:12 and in particular is about 1:10. Said phospholipid, lipophilic additive and the TrkB or TrkC agonist and other pharmaceutical compounds are employed in conjunction with a non-toxic, pharmaceutically acceptable organic solvent system which dissolve said ingredients. Said solvent system not only must dissolve the TrkB or TrkC agonist completely, but it also has to allow the formulation of stable single bilayered liposomes. The solvent system comprises dimethylisosorbide and tetraglycol (glycofurol, tetrahydrofurfuryl alcohol polyethylene glycol ether) in an amount of about 8 to about 30%. In said solvent system, the ratio of the amount of dimethylisosorbide to the amount of tetraglycol range from about 2:1 to about 1:3, in particular from about 1:1 to about 1:2.5 and preferably is about 1:2. The amount of tetraglycol in the final composition thus vary from 5 to 20%, in particular from 5 to 15% and preferably is approximately 10%. The amount of dimethylisosorbide in the final composition thus range from 3 to 10%, in particular from 3 to 7% and preferably is approximately 5%.

The term "organic component" as used hereinafter refers to mixtures comprising said phospholipid, lipophilic

additives and organic solvents. The TrkB or TrkC agonist may be dissolved in the organic component, or other means to maintain full activity of the agent. The amount of TrkB or TrkC agonist in the final formulation may range from 0.1 to 5.0%. In addition, other ingredients such as anti-oxidants may be added to the organic component. Examples include tocopherol, butylated hydroxyanisole, butylated hydroxytoluene, ascorbyl palmitate, ascorbyl oleate and the like.

Liposomal formulations are alternatively prepared, for TrkB or TrkC agonist or other pharmaceutical agents that are moderately heat-resistant, by (a) heating the phospholipid and the organic solvent system to about 60-80 °C in a vessel, dissolving the active ingredient, then adding any additional formulating agents, and stirring the mixture until complete dissolution is obtained; (b) heating the aqueous solution to 90-95 °C in a second vessel and dissolving the preservatives therein, allowing the mixture to cool and then adding the remainder of the auxiliary formulating agents and the remainder of the water, and stirring the mixture until complete dissolution is obtained; thus preparing the aqueous component; (c) transferring the organic phase directly into the aqueous component, while homogenizing the combination with a high performance mixing apparatus, for example, a high-shear mixer; and (d) adding a viscosity enhancing agent to the resulting mixture while further homogenizing. The aqueous component is optionally placed in a suitable vessel which is equipped with a homogenizer and homogenization is effected by creating turbulence during the injection of the organic component. Any mixing means or homogenizer which exerts high shear forces on the mixture may be employed. Generally, a mixer capable of speeds from about 1,500 to 20,000 rpm, in particular from about 3,000 to about 6,000 rpm may be employed. Suitable viscosity enhancing agents for use in process step (d) are for example, xanthan gum, hydroxypropyl cellulose, hydroxypropyl methylcellulose or mixtures thereof. The amount of viscosity enhancing agent depends on the nature and the concentration of the other ingredients and in general ranges from about 0.5 to 2.0%, or approximately 1.5%. In order to prevent degradation of the materials used during the preparation of the liposomal formulation, it is advantageous to purge all solutions with an inert gas such as nitrogen or argon, and to conduct all steps under an inert atmosphere. Liposomes prepared by the above described method usually contain most of the active ingredient bound in the lipid bilayer and separation of the liposomes from unencapsulated material is not required.

As disclosed herein, the auris-acceptable formulations, including gel formulations and viscosity-enhanced formulations, further include excipients, other medicinal or pharmaceutical agents, carriers, adjuvants, such as preserving, stabilizing, wetting or emulsifying agents, solution promoters, salts, solubilizers, an antifoaming agent, an antioxidant, a dispersing agent, a wetting agent, a surfactant, and combinations thereof.

Suitable carriers for use in an auris-acceptable formulation described herein include, but are not limited to, any pharmaceutically acceptable solvent compatible with the targeted auris structure's physiological environment. As disclosed herein, the base is a combination of a pharmaceutically acceptable surfactant and solvent.

As disclosed herein , other excipients include, sodium stearyl fumarate, diethanolamine cetyl sulfate, isostearate, polyethoxylated castor oil, nonoxyl 10, octoxynol 9, sodium lauryl sulfate, sorbitan esters (sorbitan monolaurate, sorbitan monooleate, sorbitan monopalmitate, sorbitan monostearate, sorbitan sesquioleate, sorbitan trioleate, sorbitan tristearate, sorbitan laurate, sorbitan oleate, sorbitan palmitate, sorbitan stearate, sorbitan dioleate, sorbitan sesqui-isostearate, sorbitan sesquistearate, sorbitan tri-isostearate), lecithin pharmaceutical acceptable salts thereof and combinations or mixtures thereof.

As disclosed herein, the carrier is a polysorbate. Polysorbates are nonionic surfactants of sorbitan esters. Polysorbates useful in the present disclosure include, but are not limited to polysorbate 20, polysorbate 40, polysorbate 60, polysorbate 80 (Tween 80) and any combinations or mixtures thereof. As disclosed herein , polysorbate 80 is utilized as the pharmaceutically acceptable carrier.

As disclosed herein , water-soluble glycerin-based auris-acceptable enhanced viscosity formulations utilized in the preparation of pharmaceutical delivery vehicles comprise at least one TrkB or TrkC agonist containing at least about 0.1% of the water-soluble glycerin compound or more. As disclosed herein , the percentage of TrkB or TrkC agonist is varied between about 1% and about 95%, between about 5% and about 80%, between about 10% and about 60% or more of the weight or volume of the total pharmaceutical formulation. As disclosed herein , the amount of the compound(s) in each therapeutically useful TrkB or TrkC agonist formulation is prepared in such a way that a suitable dosage will be obtained in any given unit dose of the compound. Factors such as solubility, bioavailability, biological half-life, route of administration, product shelf life, as well as other pharmacological considerations are contemplated herein.

If desired, the auris-acceptable pharmaceutical gels also contain cosolvents, preservatives, cosolvents, ionic strength and osmolality adjustors and other excipeints in addition to buffering agents. Suitable auris-acceptable water soluble buffering agents are alkali or alkaline earth metal carbonates, phosphates, bicarbonates, citrates, borates, acetates, succinates and the like, such as sodium phosphate, citrate, borate, acetate, bicarbonate, carbonate and tromethamine (TRIS). These agents are present in amounts sufficient to maintain the pH of the system at 7.4±0.2 and preferably, 7.4. As such, the buffering agent is as much as 5% on a weight basis of the total composition.

Cosolvents are used to enhance TrkB or TrkC agonist solubility, however, some TrkB or TrkC agonist or other pharmaceutical compounds are insoluble. These are often suspended in the polymer vehicle with the aid of suitable suspending or viscosity enhancing agents.

Moreover, some pharmaceutical excipients, diluents or carriers are potentially ototoxic. For example, benzalkonium chloride, a common preservative, is ototoxic and therefore potentially harmful if introduced into the vestibular or cochlear structures. In formulating a controlled release TrkB or TrkC agonist formulation, it is advised to avoid or combine the appropriate excipients, diluents or carriers to lessen or eliminate potential ototoxic components from the formulation, or to decrease the amount of such excipients, diluents or carriers. Optionally, a controlled release TrkB or TrkC agonist formulation includes otoprotective agents, such as antioxidants, alpha lipoic acid, calicum, fosfomycin or iron chelators, to counteract potential ototoxic effects that may arise from the use of specific therapeutic agents or excipients, diluents or carriers.

Therapeutically acceptable otic formulations:

| Example Formulation | Example Characteristics |
|---|---|
| Chitosan glycerophosphate (CGP) | • tunable degradation of matrix in vitro<br>• tunable TACE inhibitor release in vitro: e.g., ~50 % of drug released after 24 hrs<br>• biodegradable<br>• compatible with drug delivery to the inner ear<br>• suitable for macromolecules and hydrophobic drugs |
| PEG-PLGA-PEG triblock polymers | • tunable high stability: e.g., maintains mechanical integrity > 1 month in vitro<br>• tunable fast release of hydrophilic drugs: e.g., ~ 50 % of drug released after 24 hrs, and remainder released over 5 days<br>• tunable slow release of hydrophobic drugs: e.g., ~ 80 % released after 8 weeks<br>• biodegradable<br>• subcutaneous injection of solution: e.g., gel forms within seconds and is intact after 1 month |
| PEO-PPO-PEO triblock copolymers (e.g., Pluronic or Poloxameres) (e.g., F127) | • Tunable sol-gel transition temperature: e.g., decreases with increasing F127 concentration |
| Chitosan glycerophosphate with drug-loaded liposomes | • CGP formulation tolerates liposomes: e.g., up to 15 uM/ml liposomes.<br>• liposomes tunably reduce drug release time (e.g., up to 2 weeks in vitro).<br>• increase in liposome diameter optionally reduces drug release kinetics (e.g., liposome size between 100 and 300 nm)<br>• release parameters are controlled by changing composition of liposomes |

The formulations disclosed herein alternatively encompass an otoprotectant agent in addition to the at least one TrkB or TrkC agonist and/or excipients, including but not limited to such agents as antioxidants, alpha lipoic acid, calcium, fosfomycin or iron chelators, to counteract potential ototoxic effects that may arise from the use of specific therapeutic agents or excipients, diluents or carriers.

**Modes of Treatment**

**Dosing Methods and Schedules**

Drugs delivered to the inner ear have been administered systemically via oral, intravenous or intramuscular routes. However, systemic administration for pathologies local to the inner ear increases the likelihood of systemic toxicities and adverse side effects and creates a non-productive distribution of drug in which high levels of drug are found in the serum and correspondingly lower levels are found at the inner ear.

Intratympanic injection of therapeutic agents is the technique of injecting a therapeutic agent behind the tympanic membrane into the middle and/or inner ear. As disclosed herein , the TrkB or TrkC agonist formulations described herein are administered directly onto the round window membrane via transtympanic injection. As disclosed herein, the TrkB or TrkC agonist auris-acceptable formulations described herein are administered onto the round window membrane via

a non-transtympanic approach to the inner ear. As disclosed herein, the TrkB or TrkC agonist auris-acceptable formulation described herein is administered onto the round window membrane via a surgical approach to the round window membrane comprising modification of the crista fenestrae cochleae.

As disclosed herein the delivery system is a syringe and needle apparatus that is capable of piercing the tympanic membrane and directly accessing the round window membrane or crista fenestrae cochleae of the auris interna. As disclosed herein , the needle on the syringe is wider than a 18 gauge needle. As disclosed herein, the needle gauge is from 18 gauge to 31 gauge. As disclosed herein, the needle gauge is from 25 gauge to 30 gauge. Depending upon the thickness or viscosity of the TrkB or TrkC agonist compositions or formulations, the gauge level of the syringe or hypodermic needle may be varied accordingly. As disclosed herein, the internal diameter of the needle can be increased by reducing the wall thickness of the needle (commonly referred as thin wall or extra thin wall needles) to reduce the possibility of needle clogging while maintaining an adequate needle gauge.

As disclosed herein, the needle is a hypodermic needle used for instant delivery of the gel formulation. The hypodermic needle may be a single use needle or a disposable needle. As disclosed herein , a syringe may be used for delivery of the pharmaceutically acceptable gel-based TrkB or TrkC agonist-containing compositions as disclosed herein wherein the syringe has a press-fit (Luer) or twist-on (Luer-lock) fitting. As disclosed herein , the syringe is a hypodermic syringe. As disclosed herein, the syringe is made of plastic or glass. As disclosed herein, the hypodermic syringe is a single use syringe. As disclosed herein, the glass syringe is capable of being sterilized. As disclosed herein, the sterilization occurs through an autoclave. As disclosed herein, the syringe comprises a cylindrical syringe body wherein the gel formulation is stored before use. As disclosed herein, the syringe comprises a cylindrical syringe body wherein the TrkB or TrkC agonist pharmaceutically acceptable gel-based compositions as disclosed herein is stored before use which conveniently allows for mixing with a suitable pharmaceutically acceptable buffer. As disclosed herein, the syringe may contain other excipients, stabilizers, suspending agents, diluents or a combination thereof to stabilize or otherwise stably store the TrkB or TrkC agonist or other pharmaceutical compounds contained therein.

As disclosed herein , the syringe comprises a cylindrical syringe body wherein the body is compartmentalized in that each compartment is able to store at least one component of the auris-acceptable TrkB or TrkC agonist gel formulation. As disclosed herein, the syringe having a compartmentalized body allows for mixing of the components prior to injection into the auris media or auris interna. As disclosed herein, the delivery system comprises multiple syringes, each syringe of the multiple syringes contains at least one component of the gel formulation such that each component is pre-mixed prior to injection or is mixed subsequent to injection. As disclosed herein, the syringes disclosed herein comprise at least one reservoir wherein the at least one reservoir comprises an TrkB or TrkC agonist, or a pharmaceutically acceptable buffer, or a viscosity enhancing agent, such as a gelling agent or a combination thereof. Commercially available injection devices are optionally employed in their simplest form as ready-to-use plastic syringes with a syringe barrel, needle assembly with a needle, plunger with a plunger rod, and holding flange, to perform an intratympanic injection.

As disclosed herein , the delivery device is an apparatus designed for administration of therapeutic agents to the middle and/or inner ear. By way of example only: GYRUS Medical Gmbh offers micro-otoscopes for visualization of and drug delivery to the round window niche; Arenberg has described a medical treatment device to deliver fluids to inner ear structures in U.S. Patent Nos. 5,421,818; 5,474,529; and 5,476,446. U.S. Patent Application No. 08/874,208 (US Pat. No. 6045528), describes a surgical method for implanting a fluid transfer conduit to deliver therapeutic agents to the inner ear. U.S. Patent Application Publication 2007/0167918, , further describes a combined otic aspirator and medication dispenser for intratympanic fluid sampling and medicament application.

The auris-acceptable compositions or formulations containing the TrkB or TrkC agonists described herein are administered for prophylactic and/or therapeutic treatments. In therapeutic applications, the TrkB or TrkC agonist compositions are administered to a patient already suffering from an autoimmune disease, condition or disorder, in an amount sufficient to cure or at least partially arrest the symptoms of the disease, disorder or condition. Amounts effective for this use will depend on the severity and course of the disease, disorder or condition, previous therapy, the patient's health status and response to the drugs, and the judgment of the treating physician.

**Frequency of Administration**

As disclosed herein , a composition disclosed herein is administered to an individual in need thereof once. As disclosed herein , a composition disclosed herein is administered to an individual in need thereof more than once. As disclosed herein , a first administration of a composition disclosed herein is followed by a second administration of a composition disclosed herein. As disclosed herein , a first administration of a composition disclosed herein is followed by a second and third administration of a composition disclosed herein. As disclosed herein , a first administration of a composition disclosed herein is followed by a second, third, and fourth administration of a composition disclosed herein. As disclosed herein , a first administration of a composition disclosed herein is followed by a second, third, fourth, and fifth administration of a composition disclosed herein. As disclosed herein , a first administration of a composition disclosed

herein is followed by a drug holiday.

The number of times a composition is administered to an individual in need thereof depends on the discretion of a medical professional, the disorder, the severity of the disorder, and the individuals's response to the formulation. As disclosed herein , a composition disclosed herein is administered once to an individual in need thereof with a mild acute condition. As disclosed herein , a composition disclosed herein is administered more than once to an individual in need thereof with a moderate or severe acute condition. In the case wherein the patient's condition does not improve, upon the doctor's discretion the administration of an auris sensory cell modulator may be administered chronically, that is, for an extended period of time, including throughout the duration of the patient's life in order to ameliorate or otherwise control or limit the symptoms of the patient's disease or condition.

In the case wherein the patient's condition does not improve, upon the doctor's discretion the administration of the TrkB or TrkC agonist compounds may be administered chronically, that is, for an extended period of time, including throughout the duration of the patient's life in order to ameliorate or otherwise control or limit the symptoms of the patient's disease or condition.

In the case wherein the patient's status does improve, upon the doctor's discretion the administration of the TrkB or TrkC agonist is given continuously; alternatively, the dose of drug being administered is temporarily reduced or temporarily suspended for a certain length of time (*i.e.*, a "drug holiday"). The length of the drug holiday varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, and 365 days. The dose reduction during a drug holiday may be from 10%-100%, including by way of example only 10%, 15%, 20%, 25%, 30%, 35%, 40%, 45%, 50%, 55%, 60%, 65%, 70%, 75%, 80%, 85%, 90%, 95%, and 100%.

Once improvement of the patient's otic conditions has occurred, a maintenance TrkB or TrkC agonist dose is administered if necessary. Subsequently, the dosage or the frequency of administration, or both, is optionally reduced, as a function of the symptoms, to a level at which the improved disease, disorder or condition is retained. In certain disclosures, patients require intermittent treatment on a long-term basis upon any recurrence of symptoms.

The amount of TrkB or TrkC agonist that will correspond to such an amount will vary depending upon factors such as the particular compound, disease condition and its severity, according to the particular circumstances surrounding the case, including, e.g., the specific TrkB or TrkC agonist being administered, the route of administration, the autoimmune condition being treated, the target area being treated, and the subject or host being treated. In general, however, doses employed for adult human treatment will typically be in the range of 0.02-50 mg per administration, preferably 1-15 mg per administration. The desired dose is presented in a single dose or as divided doses administered simultaneously (or over a short period of time) or at appropriate intervals.

As disclosed herein , the initial administration is a particular TrkB or TrkC agonist and the subsequent administration a different formulation or TrkB or TrkC agonist.

**Pharmacokinetics of Controlled Release Formulations**

The formulations disclosed herein additionally provides an immediate release of a TrkB or TrkC agonist from the composition, or within 1 minute, or within 5 minutes, or within 10 minutes, or within 15 minutes, or within 30 minutes, or within 60 minutes or within 90 minutes. As disclosed herein, a therapeutically effective amount of at least one TrkB or TrkC agonist is released from the composition immediately, or within 1 minute, or within 5 minutes, or within 10 minutes, or within 15 minutes, or within 30 minutes, or within 60 minutes or within 90 minutes. As discosed herein the composition comprises an auris-pharmaceutically acceptable gel formulation providing immediate release of at least one TrkB or TrkC agonist. Additional aspects of the disclosure of the formulation also includes an agent that enhances the viscosity of the formulations included herein.

In other or further aspects, the formulation provides an extended release formulation of at least one TrkB or TrkC agonist. A disclosed herein, diffusion of at least one TrkB or TrkC agonist from the formulation occurs for a time period exceeding 5 minutes, or 15 minutes, or 30 minutes, or 1 hour, or 4 hours, or 6 hours, or 12 hours, or 18 hours, or 1 day, or 2 days, or 3 days, or 4 days, or 5 days, or 6 days, or 7 days, or 10 days, or 12 days, or 14 days, or 18 days, or 21 days, or 25 days, or 30 days, or 45 days, or 2 months or 3 months or 4 months or 5 months or 6 months or 9 months or 1 year. In other embodiments, a therapeutically effective amount of at least one TrkB or TrkC agonist is released from the formulation for a time period exceeding 5 minutes, or 15 minutes, or 30 minutes, or 1 hour, or 4 hours, or 6 hours, or 12 hours, or 18 hours, or 1 day, or 2 days, or 3 days, or 4 days, or 5 days, or 6 days, or 7 days, or 10 days, or 12 days, or 14 days, or 18 days, or 21 days, or 25 days, or 30 days, or 45 days, or 2 months or 3 months or 4 months or 5 months or 6 months or 9 months or 1 year.

In other aspects of the disclosure, the formulation provides both an immediate release and an extended release formulation of a TrkB or TrkC agonist. In yet other embodiments, the formulation contains a 0.25: 1 ratio, or a 0.5:1 ratio, or a 1:1 ratio, or a 1:2 ratio, or a 1:3, or a 1:4 ratio, or a 1:5 ratio, or a 1:7 ratio, or a 1:10 ratio, or a 1: 15 ratio, or a 1:20

ratio of immediate release and extended release formulations. In a further aspect the formulation provides an immediate release of a first TrkB or TrkC agonist and an extended release of a second TrkB or TrkC agonist or other therapeutic agent. In yet other aspects, the formulation provides an immediate release and extended release formulation of at least one TrkB or TrkC agonist, and at least one other therapeutic agent. As disclosed herein , the formulation provides a 0.25:1 ratio, or a 0.5:1 ratio, or a 1:1 ratio, or a 1:2 ratio, or a 1:3, or a 1:4 ratio, or a 1:5 ratio, or a 1:7 ratio, or a 1:10 ratio, or a 1: 15 ratio, or a 1:20 ratio of immediate release and extended release formulations of a first TrkB or TrkC agonist and second therapeutic agent, respectively.

In a specific aspect of the disclosure the formulation provides a therapeutically effective amount of at least one TrkB or TrkC agonist at the site of disease with essentially no systemic exposure. In an additional aspect the formulation provides a therapeutically effective amount of at least one TrkB or TrkC agonist at the site of disease with essentially no detectable systemic exposure. In other aspects, the formulation provides a therapeutically effective amount of at least one TrkB or TrkC agonist at the site of disease with little or no detectable systemic exposure.

The combination of immediate release, delayed release and/or extended release TrkB or TrkC agonist compositions or formulations may be combined with other pharmaceutical agents, as well as the excipients, diluents, stabilizers, tonicity agents and other components disclosed herein. As such, depending upon the TrkB or TrkC agonist used, the thickness or viscosity desired, or the mode of delivery chosen, alternative aspects of the embodiments disclosed herein are combined with the immediate release, delayed release and/or extended release embodiments accordingly.

A disclosed herein, the pharmacokinetics of the TrkB or TrkC agonist formulations described herein are determined by injecting the formulation on or near the round window membrane of a test animal (including by way of example, a guinea pig or a chinchilla). At a determined period of time (e.g., 6 hours, 12 hours, 1 day, 2 days, 3 days, 4 days, 5 days, 6 days, and 7 days for testing the pharmacokinetics of a formulation over a 1 week period), the test animal is euthanized and a 5 mL sample of the perilymph fluid is tested. The inner ear removed and tested for the presence of the TrkB or TrkC agonist. As needed, the level of TrkB or TrkC agonist is measured in other organs. In addition, the systemic level of the TrkB or TrkC agonist is measured by withdrawing a blood sample from the test animal. In order to determine whether the formulation impedes hearing, the hearing of the test animal is optionally tested.

Alternatively, an inner ear is provided (as removed from a test animal) and the migration of the TrkB or TrkC agonist is measured. As yet another alternative, an in vitro model of a round window membrane is provided and the migration of the TrkB or TrkC agonist is measured.

## Kits/Articles of Manufacture

The disclosure also provides kits for preventing, treating or ameliorating the symptoms of a disease or disorder in a mammal. Such kits generally will comprise one or more of the TrkB or TrkC agonist controlled-release compositions or devices disclosed herein, and instructions for using the kit. The disclosure also contemplates the use of one or more of the TrkB or TrkC agonist controlled-release compositions, in the manufacture of medicaments for treating, abating, reducing, or ameliorating the symptoms of a disease, dysfunction, or disorder in a mammal, such as a human that has, is suspected of having, or at risk for developing an inner ear disorder.

As disclosed herein , kits include a carrier, package, or container that is compartmentalized to receive one or more containers such as vials, tubes, and the like, each of the container(s) including one of the separate elements to be used in a method described herein. Suitable containers include, for example, bottles, vials, syringes, and test tubes. In other aspects, the containers are formed from a variety of materials such as glass or plastic.

The articles of manufacture provided herein contain packaging materials. Packaging materials for use in packaging pharmaceutical products are also presented herein. See, e.g., U.S. Patent Nos. 5,323,907, 5,052,558 and 5,033,252. Examples of pharmaceutical packaging materials include, but are not limited to, blister packs, bottles, tubes, inhalers, pumps, bags, vials, containers, syringes, bottles, and any packaging material suitable for a selected formulation and intended mode of administration and treatment. A wide array of TrkB or TrkC agonist formulations compositions provided herein are contemplated as are a variety of treatments for any disease, disorder, or condition that would benefit by controlled release administration of a TrkB or TrkC agonist to the inner ear.

As disclosed herein , a kit includes one or more additional containers, each with one or more of various materials (such as reagents, optionally in concentrated form, and/or devices) desirable from a commercial and user standpoint for use of a formulation described herein. Non-limiting examples of such materials include, but not limited to, buffers, diluents, filters, needles, syringes; carrier, package, container, vial and/or tube labels listing contents and/or instructions for use and package inserts with instructions for use. A set of instructions is optionally included. In a further aspect of the disclosure, a label is on or associated with the container. In yet a further aspect, a label is on a container when letters, numbers or other characters forming the label are attached, molded or etched into the container itself; a label is associated with a container when it is present within a receptacle or carrier that also holds the container, e.g., as a package insert. In other aspects, a label is used to indicate that the contents are to be used for a specific therapeutic application. In yet another aspect, a label also indicates directions for use of the contents, such as in the methods

described herein.

A disclosed herein, the pharmaceutical compositions are presented in a pack or dispenser device which contains one or more unit dosage forms containing a compound provided herein. In another aspect of the disclosure, the pack for example contains metal or plastic foil, such as a blister pack. In a further aspect, the pack or dispenser device is accompanied by instructions for administration. In yet a further aspect, the pack or dispenser is also accompanied with a notice associated with the container in form prescribed by a governmental agency regulating the manufacture, use, or sale of pharmaceuticals, which notice is reflective of approval by the agency of the form of the drug for human or veterinary administration. In another aspect, such notice, for example, is the labeling approved by the U.S. Food and Drug Administration for prescription drugs, or the approved product insert. In yet another aspect, compositions containing a compound provided herein formulated in a compatible pharmaceutical carrier are also prepared, placed in an appropriate container, and labeled for treatment of an indicated condition.

## EXAMPLES

### Example 1 - Preparation of a Thermoreversible Gel TrkB or TrkC Agonist Formulation (TrkC Agonist is for comparative purposes only and is not part of the invention)

**Table 4. Thermoreversible Gel TrkB or TrkC Agonist Formulation**

| Ingredient | Concentration in 1000mL aqueous solution |
|---|---|
| TrkB or TrkC agonist | 0.01-10 (wt%) |
| Polyoxyethylene-polypropylene triblock copolymer (e.g. Poloxamer 407) | 14 - 21 (wt%) |
| pH adjusting agent (e.g. HCl) | q.s. for pH= 5.5-8.0 |
| Sterile water | q.s. to 1000mL |

An exemplary batch of gel formulation containing, for example, 1.0% of a TrkB or TrkC agonist is prepared by first suspending Poloxamer 407 (BASF Corp.) in sterile water with a pH between 5.5-8.0. The Poloxamer 407 and pH adjusted sterile water are mixed under agitation overnight at 4 °C to ensure complete dissolution of the Poloxamer 407 in the pH adjusted sterile water. A solution of TrkB or TrkC agonist is added and the composition is mixed until a homogenous gel is produced. The mixture is maintained below room temperature until use.

### Example 2 - In vitro comparison of gelation temperature

The effect of Poloxamer 188 and TrkB or TrkC agonist on the gelation temperature and viscosity of Poloxamer 407 formulations is evaluated with the purpose of manipulating the gelation temperature.

A 25% Poloxamer 407 stock solution in PBS buffer. Poloxamer 188NF from BASF is used. An appropriate amount of TrkB or TrkC agonist is added to the solutions described in Table 4 to provide a 2% formulation of the TrkB or TrkC agonist.

A PBS buffer (pH 7.3) is prepared by dissolving 805.5 mg of sodium chloride (Fisher Scientific), 606 mg of sodium phosphate dibasic anhydrous (Fisher Scientific), 247 mg of sodium phosphate monobasic anhydrous (Fisher Scientific), then QS to 200g with sterile filtered DI water.

**Table 5. Preparation of Samples Containing Poloxamer 407/Poloxamer 188**

| Sample | 25%P407 Stock Solution (g) | Poloxamer 188 (mg) | PBS Buffer (g) |
|---|---|---|---|
| 16%P407/10%P188 | 3.207 | 501 | 1.3036 |
| 17%P407/10%P188 | 3.4089 | 500 | 1.1056 |
| 18%P407/10%P188 | 3.6156 | 502 | 0.9072 |
| 19%P407/10%P188 | 3.8183 | 500 | 0.7050 |
| 20%P407/10%P188 | 4.008 | 501 | 0.5032 |

(continued)

| Sample | 25%P407 Stock Solution (g) | Poloxamer 188 (mg) | PBS Buffer (g) |
|---|---|---|---|
| 20%P407/5%P188 | 4.01 | 256 | 0.770 |

Gellation temperature of the above formulations are measured using procedures described herein.

An equation is fitted to the data obtained and is utilized to estimate the gelation temperature of F127/F68 mixtures (for 17-20% F127 and 0-10% F68).

$$T_{gel} = -1.8\ (\%F127) + 1.3\ (\%F68) + 53$$

An equation is fitted to the data obtained and can be utilized to estimate the Mean Dissolution Time (hr) based on the gelation temperature of F127/F68 mixtures (for 17-25% F127 and 0-10% F68), using results obtained in examples above.

$$MDT = -0.2\ (T_{gel}) + 8$$

**Example 3 - Pharmacokinetics of BDNF and NT3 intratympanic injections**

Poloxamer 407 gel at 16% was prepared using the cold method. In brief, a 16% w/w stock solution of poloxamer 407 was prepared by slowly adding it to a cold buffer solution (10 mM PBS, pH 7.4). Sterilization was achieved by filtration. Human recombinant BDNF was suspended with an appropriate amount of poloxamer 407 solution to reach a concentration of 1.05 mg/ml BDNF. Human recombinant NT-3 was suspended with an appropriate amount of poloxamer 407 solution to reach a concentration of 1.05 mg/ml NT-3.

Female rats (Charles River) weighing 200-300 g of approximately 12-16 weeks of age served as subjects (N = 4 per group). Prior to any procedures, animals were anesthetized using a combination of xylazine (10 mg/kg) and ketamine (90 mg/kg) for up to an hour via the intraperitoneal route. If needed, an intraoperative booster was administered intraperitoneal representing a one-tenth of the original dose.

*Intratympanic injection* - Each animal was positioned so that the head was tilted at an angle to favor injection towards the round window niche. Briefly, under visualization with an operating microscope, 20 μL of the formulation was injected using a 25G (Gauge) 11/2 needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 $^0$C) heating pad until consciousness was regained at which time they were returned to the vivarium.

*Perilymph sampling procedure* - The skin behind the ear of anesthetized rats was shaved and disinfected with povidone-iodine. An incision was then made behind the ear, and muscles were carefully retracted from over the bulla. A hole was drilled through the bulla using a dental burr so that the middle ear was exposed and accessed. The cochlea and the round window membrane were visualized under a stereo surgical microscope. The basal turn of bulla was cleaned by using small cotton ball. A unique microhole was hand drilled through the bony shell of the cochlea (cochlear capsule) adjacent to the round window. A 2 μL volume of perilymph was then collected using a microcapillary inserted into the cochlear scala tympani. Perilymph samples were added to a vial containing 18 μL of acetonitrile/water (50/50, v/v), stored at -80 °C until analysis.

Concentrations of BDNF and NT-3 in perilymph and plasma samples were determined using commercially available ELISA kits. The limits of detection of human BDNF were 80 pg/mL. The limits of detection of human NT-3 were < 4 pg/mL.

Results shown in Figure 2 indicate that both BDNF and NT-3 were present in the perilymph for 7 days after a single intratympanic injection.

**Example 4 - Clinical Trial of a TrkB or TrkC agonist as a Treatment for Tinnitus (TrkC Agonist is for comparative purposes only and is not part of the invention)**

Active Ingredient: TrkB or TrkC agonist
Dosage: 10 ng delivered in 10 $\mu$L of a thermoreversible gel. Release of a TrkB or TrkC agonist is controlled release and occurs over thirty (30) days.
Route of Administration: Intratympanic injection
Treatment Duration: 12 weeks

Methodology

- Monocentric
- Prospective
- Randomized
- Double-blind
- Placebo-controlled
- Parallel group
- Adaptive

Inclusion Criteria

- Male and female subjects between the 18 and 64 years of age.
- Subjects experiencing subjective tinnitus.
- Duration of tinnitus is greater than 3 months.
- No treatment of tinnitus within 4 weeks.

Evaluation Criteria

- Efficacy (Primary)

    1. Total score of the Tinnitus Questionnaire

- Efficacy (Secondary)

    1. Audiometric measurements (mode, frequency, loudness of the tinnitus, pure tone audiogram, speech audiogram)
    2. Quality of Life questionnaire

- Safety

    1. Treatment groups were compared with respect to incidence rates of premature termination, treatment-emergent adverse events, laboratory abnormalities, and ECG abnormalities.

Study Design

Subjects are divided into three treatment groups. The first group is the safety sample. The second group is the intent-to-treat (ITT) sample. The third group is the valid for efficacy (VfE) group.
For each group, one half of subjects to be given a TrkB or TrkC agonist and the remainder to be given placebo.

Statistical Methods

The primary efficacy analysis is based on the total score of the Tinnitus Questionnaire in the ITT sample. The statistical analysis is based on an analysis of covariance (ANCOVA) with baseline as covariant and the last observation carried forward value as dependent variable. Factor is "treatment." The homogeneity of regression slopes is tested. The analysis is repeated for the VfE sample.

Audiometric measurements (mode, frequency, loudness of the tinnitus, pure tone audiogram, speech audiogram) as well as quality of life are also analyzed via the aforementioned model. The appropriateness of the model is not tested. P values are exploratory and are not adjusted for multiplicity.

**Example 5 - Clinical Trial of a TrkB or TrkC agonist as a Treatment for Noise Induced Hearing Loss (TrkC Agonist is for comparative purposes only and is not part of the invention)**

Active Ingredient: TrkB or TrkC agonist
Dosage: A composition comprising 4% by weight of a TrkB or TrkC agonist delivered in 10 μL dose of a thermoreversible gel. Release of the TrkB or TrkC agonist is controlled release and occurs over 3 weeks.
Route of Administration: Intratympanic injection
Treatment Duration: 12 weeks, one injection every 3 weeks

Methodology

- Monocentric
- Prospective
- Randomized
- Double-blind
- Placebo-controlled
- Parallel group
- Adaptive

Inclusion Criteria

- Male and female subjects between the 18 and 64 years of age.
- Acoustic trauma followed by hearing loss that is documented by audiogram and medical report with an inner ear hearing loss of at least 15 dB
- Acute tinnitus that has persisted for at least 3 months.
- No prior treatment of hearing loss within 4 weeks.

Evaluation Criteria

- Efficacy (Primary)

    1. Audiometric measurements (pure tone audiogram, speech audiogram)
    2. Quality of Life questionnaire

- Safety

    1. Treatment groups were compared with respect to incidence rates of premature termination, treatment-emergent adverse events, laboratory abnormalities, and ECG abnormalities.

Study Design

Subjects are divided into three treatment groups. The first group is the safety sample. The second group is the intent-to-treat (ITT) sample. The third group is the valid for efficacy (VfE) group.
For each group, one half of subjects to be given a TrkB or TrkC agonist and the remainder to be given placebo.

Statistical Methods

The primary efficacy analysis is based on the pure tone audiogram in the ITT sample. The statistical analysis is based on an analysis of covariance (ANCOVA) with baseline as covariant and the last observation carried forward value as dependent variable. Factor is "treatment." The homogeneity of regression slopes is tested. The analysis is repeated for the VfE sample.

**Example 6 - Clinical Trial of a TrkB or TrkC agonist as a treatment in combination with implantation of a cochlear hearing device (TrkC Agonist is for comparative purposes only and is not part of the invention)**

Active Ingredient: TrkB or TrkC agonist
Dosage: A composition comprising a TrkB or TrkC agonist, used as a pre-surgical irrigation solution and a post-surgical irrigation solution. Release of a TrkB or TrkC agonist is immediate release.

Study Design

Twenty patients will be enrolled in the study. Ten patients will be in the control group and ten patients will be in the treatment group.

Eligibility criteria

- Having severe to profound sensorineural hearing impairment in both ears
- Having a functioning auditory nerve
- Having lived at least a short amount of time without hearing (approximately 70+ decibel hearing loss, on average)
- Having good speech, language, and communication skills, or in the case of infants and young children, having a family willing to work toward speech and language skills with therapy
- Not benefitting enough from other kinds of hearing aids
- Having no medical reason to avoid surgery

Each patient will be subjected to chochloestomy and insertion of electrodes. The treatment group will be subjected to perfusion of the surgical area with the test composition prior to surgery and after surgery. The patients will be monitored for 6 weeks. Intracochlear trauma will be evaluated based on audiometric measurements, speech audiogram as well as quality of life. Occurrence of secondary infections and/or inflammation will be monitored.

**Example 7 - Clinical Trial of a TrkB or TrkC agonist in combination with Cisplatin (TrkC Agonist is for comparative purposes only and is not part of the invention)**

The purpose of this study is to determine if a composition comprising a TrkB or TrkC agonist administered in combination with cisplatin is safe and effective in preventing and/or treating chemotherapy induced hearing loss in patients.

Study Type: Interventional
Study Design: This will be a non-inferiority open label study to compare the current standard of care versus the use of extended release intratympanic compositions in combination with cisplatin. The study is designed to test whether administration of a sustained release composition in combination with cisplatin prevents and/or treats chemotherapy induced hearing loss.

Inclusion Criteria:

- Male and female subjects between the 18 and 64 years of age, hearing loss in one or both ears
- Confirmed diagnosis of advanced head and neck cancer or advanced lung cancer
- Patient may not have any disease or condition that would negatively affect the conduct of the study
- Analgesic use (other than acetaminophen) is not allowed

Exclusion Criteria:

- Age
- Subjects previously treated with chemotherapy, antibiotics, or diuretics known to cause hearing loss in the last 90 days
- History or presence of significant cardiovascular, pulmonary, hepatic, renal, hematologic, gastrointestinal, endocrine, immunologic, dermatologic, neurologic, otologic, or psychiatric disease
- Presence of alcoholism or drug abuse
- Participation in another investigational drug or device clinical trial within 30 days prior to the study
- Female subjects who are pregnant or lactating

Twenty patients will be divided into two groups. The first group of patients will receive an injection of an extended release composition comprising a TrkB or TrkC agonist in combination with cisplatin. The second group of patients will be given placebo in combination with cisplatin.

Patients are monitored with weekly follow up visits for one month. Any differences in treatment outcomes between the two groups are recorded.

Primary Outcome Measures: Reduction and/or cessation of cisplatin induced hearing loss or the severity of cisplatin induced hearing loss; and number of participants with adverse events.

Secondary Outcome Measures: Clinical cure rate; Treatment failures; Recurrence of disease.

**Example 8 - Sustained Release of TrkC Agonist Antibody (TrkC Agonist is for comparative purposes only and is not part of the invention)**

Poloxamer 407 gel at 16% was prepared using the cold method. In brief, a 16% w/w stock solution of poloxamer 407 was prepared by slowly adding it to a cold buffer solution (10 mM PBS, pH 7.4). Sterilization was achieved by filtration. TrkC agonist mAb, as an example 2B7, was suspended with an appropriate amount of poloxamer 407 solution to reach a concentration of 1 mg/ml (0.1% dose) and 10 mg/ml (1% dose).

Female rats (Charles River) weighing 200-300 g of approximately 12-16 weeks of age served as subjects (N = 4 per group). Prior to any procedures, animals were anesthetized using a combination of xylazine (10 mg/kg) and ketamine (90 mg/kg) for up to an hour via the intraperitoneal route. If needed, an intraoperative booster was administered intraperitoneal representing a one-tenth of the original dose.

*Intratympanic injection* - Each animal was positioned so that the head was tilted at an angle to favor injection towards the round window niche. Briefly, under visualization with an operating microscope, 20 μL of the formulation was injected using a 25G (Gauge) 11/2 needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 $^0$C) heating pad until consciousness was regained at which time they were returned to the vivarium.

*Perilymph sampling procedure* - The skin behind the ear of anesthetized rats was shaved and disinfected with povidone-iodine. An incision was then made behind the ear, and muscles were carefully retracted from over the bulla. A hole was drilled through the bulla using a dental burr so that the middle ear was exposed and accessed. The cochlea and the round window membrane were visualized under a stereo surgical microscope. The basal turn of bulla was cleaned by using small cotton ball. A unique microhole was hand drilled through the bony shell of the cochlea (cochlear capsule) adjacent to the round window. Perilymph (about 2 μL) was then collected using a microcapillary inserted into the cochlear scala tympani. Perilymph samples were added to a vial containing 18 μL of water, stored at -80 °C until analysis.

Concentrations of TrkC agonist antibody (e.g., antibody 2B7) in perilymph samples were determined via a commercial ELISA. The limits of detection of human BDNF were 80 pg/mL. The limits of detection of human NT-3 were

< 4 pg/mL.

Fig. 2A and Fig. 2B show the perilymph concentrations of BDNF (Fig. 2A) and NT3 (Fig. 2B) after a single intratympanic injection of 0.1% BDNF (1.05 mg/ml) or 0.1% NT3 (1.05 mg/ml) to rats.

Fig. 3 shows perilymph concentrations of TrkC agonist antibody following a single intratympanic injection of 0.1% TrkC agonist antibody (1 mg/ml) (triangles) or 1% TrkC agonist antibody (10 mg/ml) (squares) in rats.

**Example 9 - Pharmacokinetics of human IgG intratympanic injections**

Poloxamer 407 gel at 16% was prepared using the cold method. In brief, a 16% w/w stock solution of poloxamer 407 was prepared by slowly adding it to a cold buffer solution (10 mM PBS, pH 7.4). Sterilization was achieved by filtration. Human IgG was suspended with an appropriate amount of poloxamer 407 solution to reach concentrations ranging from of 1 mg/ml (0.1%) to 50 mg/ml (5%).

Female rats (Charles River) weighing 200-300 g of approximately 12-16 weeks of age served as subjects (N = 4 per group). Prior to any procedures, animals were anesthetized using a combination of xylazine (10 mg/kg) and ketamine (90 mg/kg) for up to an hour via the intraperitoneal route. If needed, an intraoperative booster was administered intraperitoneal representing a one-tenth of the original dose.

*Intratympanic injection* - Each animal was positioned so that the head was tilted at an angle to favor injection towards the round window niche. Briefly, under visualization with an operating microscope, 20 μL of the formulation was injected using a 25G (Gauge) 11/2 needle through the tympanic membrane into the superior posterior quadrant. Formulations were delivered using a perfusion pump at the rate of 2 μL/sec. Contact with the round window membrane was maintained for 30 minutes by placing the animal in a recumbent position. During the procedure and until recovery, animals were placed on a temperature controlled (40 $^{0}$C) heating pad until consciousness was regained at which time they were returned to the vivarium.

*Perilymph sampling procedure* - The skin behind the ear of anesthetized rats was shaved and disinfected with povidone-iodine. An incision was then made behind the ear, and muscles were carefully retracted from over the bulla. A hole was drilled through the bulla using a dental burr so that the middle ear was exposed and accessed. The cochlea and the round window membrane were visualized under a stereo surgical microscope. The basal turn of bulla was cleaned by using small cotton ball. A unique microhole was hand drilled through the bony shell of the cochlea (cochlear capsule) adjacent to the round window. Perilymph (about 2 μL) was then collected using a microcapillary inserted into the cochlear scala tympani. Perilymph samples were added to a vial containing 18 μL of water, stored at -80 °C until analysis.

Concentrations of IgG in perilymph samples were determined using commercially available ELISA kits.

Fig. 4 shows perilymph concentrations of human IgG following a single intratympanic injection of 0.1% Hu IgG (circles) and 1.0% Hu IgG (squares) in rats.

**Example 10 - TrkB and TrkC receptor assays (TrkC receptor assay is for comparative purposes only as TrkC receptor is not a target of the invention)**

**Cells and incubation with test antibodies:** Cell lines stably expressing human TrkB or TrkC (in an HEK293 or 3T3 cell background, respectively) were maintained in culture with Dulbecco's modified Eagle's medium (DMEM) with 10% fetal bovine serum and 1% penicillin and streptomycin. 48 hours prior to assay, cells were transferred to a 96-well plate (5000 cells/well for HEK293 cells; 2500 cells/well for 3T3 cells). On the day of the assay, cells were serum-starved by replacing the culture medium with Dulbecco's modified Eagle's medium (DMEM; 100μl/well) and incubation for 3-4 hours at 37°C. Cells were then incubated (50μl/well) with BDNF, NT-3, test antibodies and appropriate isotype controls for 20 min in phosphate-buffered saline (PBS) at room temperature. After aspiration of the incubation media, cells were lysed by the addition of lysis buffer (1× AlphaSure Lysis Buffer Ultra, Perkin Elmer; 100μl/well or 50μl/well for 3T3 or HEK293 cells, respectively).

**Determination of phospho-ERK (p-ERK) using AlphaLisa:** 10μl of the cell lysates were placed in a 384-well plate. 10μl of antibody reagents were added to each well followed by 10μl of the acceptor/donor bead mix according to the kit instructions (Perkin Elmer kit: ALSU-PERK-A10K). Incubation was continued for 4 hours at room temperature in the dark and the 384-well plates were read at 680/520-620 excitation/emission using an Enspire (Perkin Elmer) plate reader. Values obtained (relative light units) are a quantitative representation of p-ERK in the cells. After subtraction of background from untreated cells, values for NT-3 and test antibodies were expressed relative to that for 10nM BDNF or 10nM NT-3 (100%) for TrkB or TrkC, respectively and dose-response curves generated in GraphPad Prism. $EC_{50}$ and maximum effect values were calculated for individual dose-response curves using a curve fitting program in GraphPad Prism.

Phosphorylation of ERK is an important downstream consequence of TrkB or TrkC receptor activation. The natural ligands BDNF and NT-3 increase intracellular levels of p-ERK in a dose-dependent manner through their respective receptors, TrkB and TrkC. Consequently, an increase of p-ERK in cell lines selectively expressing TrkB or TrkC is a measure of how well a test antibody activates that receptor, relative to NT-3 or BDNF. This can be assessed by determining both the EC50 value (a measure of affinity) and the maximal effect relative to NT-3 or BDNF (a measure of efficacy). Fig. 5 and Table 6 exemplify that NT-3 caused a dose-dependent increase in p-ERK in cells expressing TrkC with an EC50 of 0.3nM. M1 and M2 also increased p-ERK in a dose-dependent manner with EC50 and maximal effect values close to those for NT-3, indicating that these antibodies are TrkC agonists with affinities and maximal responses similar to NT-3. 2B7 also increased p-ERK in TrkC-expressing cells but with a lower affinity and maximal effect compared with NT-3. The TrkC antibody C44H5 that was generated using a peptide within the presumed D1 domain of TrkC had no agonist effect at concentrations up to 100nM. Fig. 6 and Table 7 exemplify that BDNF caused a dose-dependent increase in p-ERK in cells expressing TrkB with an EC50 of 0.3nM. M4 and M5 also increased p-ERK in a dose-dependent manner with EC50 values close to that of NT-3, but with lower maximum effects, indicating that these antibodies are TrkB agonists with affinities similar to BDNF. M3 also increased p-ERK in TrkB-expressing cells but with a lower affinity and maximal effect compared with BDNF.

**Table 6. $EC_{50}$ and maximum effect values for p-ERK responses in in 3T3 cells expressing human TrkC**

| | $EC_{50}$ nM | Maximal Effect % |
|---|---|---|
| NT-3 | 0.3 | 100 |
| M1 | 0.3 | 82 |
| M2 | 0.2 | 82 |
| 2B7 | 5.6 | 75 |

**Table 7. $EC_{50}$ and maximum effect values for p-ERK responses in in HEK293 cells expressing human TrkB**

| | $EC_{50}$ nM | Maximal Effect % |
|---|---|---|
| BDNF | 0.3 | 100 |
| M3 | 1.9 | 49 |
| M4 | 0.2 | 74 |
| M5 | 0.3 | 44 |

**Example 11 - Neurotrophic effects of Trk agonists in rat spiral ganglion neuron cultures (TrkC Agonist is for comparative purposes only and is not part of the invention)**

NT-3 and BDNF are known to provide trophic support to spiral ganglion neurons in the cochlea through activation of TrkC and TrkB receptors, respectively. Consequently, survival of rat spiral ganglion neurons in culture can be used to determine the ability of test antibodies to activate TrkB or TrkC in rat cochlea tissue and thereby provide trophic support to spiral ganglion neurons.

*Spiral ganglion dissection and culture:*

Postnatal Sprague Dawley rats (P2-4) of both sexes were anesthetized with isoflurane and decapitated. Temporal bones were removed and transferred to a cell culture dish with ice-cold $Ca^{2+}/Mg^{2+}$-containing phosphate-buffered saline (PBS; Invitrogen). Under microscopic visualization, the cochlear capsule was carefully removed from the temporal bone using forceps and transferred to a new cell culture dish containing ice-cold PBS. The cochlea was then dissected from the cochlear capsule using fine forceps. The stria vascularis and the organ of Corti were removed from the cochlear tissue, and the spiral ganglion neurons were subsequently detached from the modiolus. This strand, containing spiral ganglion neurons, was transferred to a 1.5mL microcentrofuge tube containing 0.5mL ice-cold $Ca^{2+}/Mg^{2+}$-free Hank's balanced salt solution (HBSS; Invitrogen). Once ~12 of these strands (representing 6 animals) were collected in cold HBSS, enzymatic and mechanical dissociation proceeded as described below.

0.5mL of warm (37°C) HBSS mixed with 1mg/mL Thermolysin (Promega) was added to the spiral ganglion collection (for a final concentration of 0.5mg/mL Thermolysin in a volume of ~1mL) and incubated at 37°C for 30-35 minutes. The cells were then briefly centrifuged, the supernatant discarded and the cells were washed twice with culture medium (Dulbecco's modified Eagle's medium with 10% fetal bovine serum; see below). The cells were resuspended

in 1 ml of culture medium and mechanically dissociated with a 1000 μl pipette, for 4 triturations. After 4 triturations, the cells were briefly centrifuged and the supernatant applied to a 40μm cell strainer (Millipore). This was repeated until the tissue was fully dissociated with no visible cell clusters remaining. Surviving cells were counted using the Countess II (Thermo Fisher) using trypan blue, and then seeded into a 96-well plate (pre-coated with poly-L-ornithine and laminin (Corning) and then incubated for 3-4 hours with 10ug/mL poly-L-lysine) at a density of $1.4 \times 10^4$ cells per well.

Treatments with NT-3, BDNF, and test antibodies were conducted for 4 days at 37°C. Immediately after seeding, test agents were added to the culture medium prepared at 10x concentration and the volume was then diluted 10-fold when added to the seeded cells. The next day, the adhered cells were washed once with serum-free culture medium and then refilled with fresh serum-free medium. 10x concentrated treatments were again added to the cells with 10-fold dilution. The cultures were kept in the incubator for an additional 3 days before being fixed, stained, and imaged.

Immunohistochemistry: Cells were fixed in cold 4% paraformaldehyde for 20 minutes, then washed twice in PBS containing 0.5% triton (PBS-T). Cells were then incubated 1-2hr at room temperature on a 15 RPM rotator in primary antibody (Anti-200 kD Neurofilament Heavy antibody; Abcam) in PBS-T containing 10% goat serum. After washing three times in PBS, the cells were then incubated for 1hr at room temperature on a 15 RPM nutator with secondary antibody (Goat Anti-Chicken IgY H&L (Alexa Fluor® 488) preadsorbed; Abcam). Cells were then washed twice, treated with DAPI nuclear stain for 5-10 minutes, and then washed two more times with PBS before imaging. Numbers of spiral ganglion neurons (identified by neurofilament staining) surviving in each well were counted.

NT-3 and BDNF both supported spiral ganglion neuron survival in culture (Fig. 7). Typically, approximately 10-20 neurons/well were present with 1nM NT-3, compares with 0-1 neurons in untreated wells. Compared with 1nM NT-3 (normalized to 100%), the effect of 10nM BDNF was 92%, and the antibodies tested (M1-5, M7, 2B7, 1D7 and ANT-020) provided varying levels of trophic support. Isotypes used as controls (mouse IgG1 or human IgG4) did not support SGN survival.

### Example 12 -TrkC agonist mAb 2B7 binds to TrkC-FL but not to TrkC.T1 (TrkC Agonist is for comparative purposes only and is not part of the invention)

**Cells:** HEK293 cells were transfected with plasmids encoding human or rat full-length TrkC (293-TrkC-FL) or coding for human TrkC.T1 (293-TrkC.T1). Stably transfected cell lines that express high levels of TrkC-FL or TrkC.T1 receptors were generated and subcloned under drug selection (depending on the vector, 0.5 mg/ml G418, or 2 mg/ml puromycin, or 10 mg/ml blastocidin).

**FACs analyses** were performed as described (Guillemard et al. Dev Neurobiol 70:150-164, 2010). Briefly, cells were resuspended in 0.1 mL of binding buffer were incubated with mAb 2B7 or control mIgG for 20 min at 4°C, washed in binding buffer to remove excess primary antibody, and immunostained with FITC-mIgG secondary antibody for 20 min at 4°C. Cells were acquired and analyzed on a FACScan-BD Sciences using the Cell Quest program. As negative controls, no primary (background fluorescence) or irrelevant mouse IgG (Sigma) were used followed by secondary antibody.

**Western Blots:** for quantification of TrkC protein, detergent lysates of 293-TrkC or 293-TrkC.T1 cells were analyzed by Western blotting with MAb 2B7, or antibody 750 specific for TrkC.T1.

As shown in Fig. 8A, 2B7 showed binding to cell surface TrkC-FL protein, at mean channel fluorescence -300. Several isotype-matched controls are shown for background, all at mean channel fluorescence -10. No significant binding to the cell surface was detected using mAb 2B7 on 293-TrkC.T1 cells, mean channel fluorescence ~15. In Fig. 8B, non-reducing Western blots of HEK293-TrkC-FL or HEK293-TrkC.T1 cells show that 2B7 only recognizes lysates form TrkC-FL cells. A control antibody 750 (against an intracellular neo-epitope that appears due to mRNA splicing) only recognizes TrkC.T1 and demonstrates that the cells express TrkC.T1 protein. These data indicate that 2B7 binds specifically to the full length and not the truncated form of TrkC.
While preferred embodiments of the present invention have been shown and described herein, such embodiments are provided by way of example only. It is intended that the following claims define the scope of the invention.

## Claims

**1.** An otic pharmaceutical composition comprising:

a therapeutically effective amount of a TrkB agonist, wherein the TrkB agonist is an antibody or a binding fragment thereof comprising light chain complementarity-determining regions (CDRs) comprising SEQ ID NOs: 14-16 and heavy chain CDRs comprising SEQ ID NOs: 17-19;
and a pharmaceutical acceptable carrier, for use in a method of treating an otic condition, wherein the otic condition is selected from ototoxicity, chemotherapy induced hearing loss, excitotoxicity, sensorineural hearing

loss, noise induced hearing loss, Meniere's Disease/Syndrome, endolymphatic hydrops, labyrinthitis, Ramsay Hunt's Syndrome, vestibular neuronitis, tinnitus, presbycusis, and microvascular compression syndrome, wherein the otic composition is capable of being administered on or near the round window membrane via intratympanic injection.

**2.** The otic pharmaceutical composition for use according to claim 1, further comprising two or more characteristics selected from:

(i) between about 0.001% and about 60% by weight of the TrkB agonist, or pharmaceutically acceptable prodrug or salt thereof;
(ii) between about 14% and about 21% by weight of a polyoxyethylene-polyoxypropylene triblock copolymer;
(iii) sterile water, q.s., buffered to provide a pH between about 5.5 and about 8.0;
(iv) a gelation temperature between about 19 °C and about 42 °C; and
(v) an apparent viscosity of about 100,000 cP to about 500,000 cP.

**3.** The otic pharmaceutical composition for use according to claim 1 or claim 2, wherein the antibody or binding fragment thereof is a monoclonal antibody, a diabody, a linear antibody, a single-chain antibody, a bi-specific antibody, a multispecific antibody formed from antibody fragments, a tandem antibody, a chimeric antibody, a murine antibody, a humanized antibody, a veneered antibody, a F(ab')2 fragment, a Fab' fragment, a Fab fragment, a Fv fragment, a rIgG fragment, or a scFv fragment.

**4.** The otic pharmaceutical composition for use according to any one of claims 1-3, wherein the composition comprises between about 14% and about 17% by weight of a polyoxyethylene-polyoxypropylene triblock copolymer, and provides extended sustained release of the TrkB agonist over a period of from about 1 week to about 3 weeks.

**5.** The otic pharmaceutical composition for use according to any one of claim 1-4, wherein the pharmaceutical composition is an auris-acceptable thermoreversible gel.

**6.** The otic pharmaceutical composition for use according to any one of claim 1-5, wherein the polyoxyethylene-polyoxypropylene triblock copolymer comprises poloxamer 407, poloxamer 188, poloxamer 237, or poloxamer 338.

**7.** The otic pharmaceutical composition for use according to any one of claim 1-6, wherein the composition has a gelation temperature of between about 19 °C and about 42 °C.

**8.** The otic pharmaceutical composition for use according to any one of claim 1-7, wherein the polyoxyethylene-polyoxypropylene copolymer is poloxamer 407.

**9.** The otic pharmaceutical composition for use according to any preceding claim , wherein the otic condition is sensorineural hearing loss.

**Patentansprüche**

**1.** Otische pharmazeutische Zusammensetzung, umfassend:

eine therapeutisch wirksame Menge eines TrkB-Agonisten, wobei der TrkB-Agonist ein Antikörper oder ein Bindungsfragment davon ist, der/das die Komplementarität bestimmende Regionen der leichten Kette (CDRs) umfasst, umfassend SEQ ID NOs: 14-16 und CDRs der schweren Kette, umfassend SEQ ID NOs: 17-19;
und einen pharmazeutisch verträglichen Träger zur Verwendung in einem Verfahren zur Behandlung einer Erkrankung des Ohrs , wobei die Erkrankung des Ohrs ausgewählt ist aus Ototoxizität, chemotherapieinduziertem Hörverlust, Exzitotoxizität, sensorineuralem Hörverlust, lärminduziertem Hörverlust, Morbus Menière/Syndrom, endolymphatischem Hydrops, Labyrinthitis, Ramsay-Hunt-Syndrom, vestibulärer Neuronitis, Tinnitus, Presbycusis und mikrovaskulärem Kompressionssyndrom,
wobei die otische Zusammensetzung in der Lage ist, auf oder in der Nähe der runden Fenstermembran durch intratympanische Injektion verabreicht zu werden.

**2.** Otische pharmazeutische Zusammensetzung zur Verwendung nach Anspruch 1, ferner umfassend zwei oder mehr Eigenschaften, ausgewählt aus:

(i) zwischen etwa 0,001 und etwa 60 Gew.-% des TrkB-Agonisten oder eines pharmazeutisch verträglichen Prodrugs oder Salzes davon;
(ii) zwischen etwa 14 und etwa 21 Gew.-% eines Polyoxyethylen-Polyoxypropylen-Triblock-Copolymers;
(iii) steriles Wasser, q.s., gepuffert, um einen pH-Wert zwischen etwa 5,5 und etwa 8,0 bereitzustellen;
(iv) eine Gelierungstemperatur zwischen etwa 19 °C und etwa 42 °C; und
(v) eine scheinbare Viskosität von etwa 100.000 cP bis etwa 500.000 cP.

**3.** Otische pharmazeutische Zusammensetzung zur Verwendung nach Anspruch 1 oder Anspruch 2, wobei der Antikörper oder das Bindungsfragment davon ein monoklonaler Antikörper, ein Diakörper, ein linearer Antikörper, ein einkettiger Antikörper, ein bispezifischer Antikörper, ein aus Antikörperfragmenten gebildeter multispezifischer Antikörper, ein Tandem-Antikörper, ein chimärer Antikörper, ein muriner Antikörper, ein humanisierter Antikörper, ein verblendeter Antikörper, ein F(ab')2-Fragment, ein Fab'-Fragment, ein Fab-Fragment, ein Fv-Fragment, ein rIgG-Fragment oder ein scFv-Fragment ist.

**4.** Otische pharmazeutische Zusammensetzung zur Verwendung nach einem der Ansprüche 1-3, wobei die Zusammensetzung zwischen etwa 14 Gew.-% und etwa 17 Gew.-% eines Polyoxyethylen-Polyoxypropylen-Triblock-Copolymers umfasst und eine verlängerte anhaltende Freisetzung des TrkB-Agonisten über einen Zeitraum von etwa 1 Woche bis etwa 3 Wochen bereitstellt.

**5.** Otische pharmazeutische Zusammensetzung zur Verwendung nach einem der Ansprüche 1-4, wobei die pharmazeutische Zusammensetzung ein Auris-verträgliches thermoreversibles Gel ist.

**6.** Otische pharmazeutische Zusammensetzung zur Verwendung nach einem der Ansprüche 1-5, wobei das Polyoxyethylen-Polyoxypropylen-Triblock-Copolymer Poloxamer 407, Poloxamer 188, Poloxamer 237 oder Poloxamer 338 umfasst.

**7.** Otische pharmazeutische Zusammensetzung zur Verwendung nach einem der Ansprüche 1-6, wobei die Zusammensetzung eine Gelierungstemperatur zwischen etwa 19 °C und etwa 42 °C aufweist.

**8.** Otische pharmazeutische Zusammensetzung zur Verwendung nach einem der Ansprüche 1-7, wobei das Polyoxyethylen-Polyoxypropylen-Copolymer Poloxamer 407 ist.

**9.** Otische pharmazeutische Zusammensetzung zur Verwendung nach einem der vorhergehenden Ansprüche, wobei der Erkrankung des Ohrs ein sensorineuraler Hörverlust ist.

## Revendications

**1.** Composition pharmaceutique otique comprenant :

une quantité thérapeutiquement efficace d'un agoniste de la TrkB, où l'agoniste de la TrkB est un anticorps ou un fragment de liaison de celui-ci comprenant des régions déterminant la complémentarité (CDR) de la chaîne légère comprenant les SEQ ID NO : 14-16 et des CDR de la chaîne lourde comprenant les SEQ ID NO : 17-19 ; et un véhicule pharmaceutiquement acceptable, destinée à être utilisée dans un procédé de traitement d'un trouble otique, où le trouble otique est choisi parmi l'ototoxicité, la perte auditive induite par la chimiothérapie, l'excitotoxicité, la perte auditive neurosensorielle, la perte auditive induite par le bruit, la maladie / le syndrome de Ménière, l'hydrops endolymphatique, la labyrinthite, le syndrome de Ramsay Hunt, la neuronite vestibulaire, l'acouphène, la presbyacousie et le syndrome de compression microvasculaire,
où la composition otique est capable d'être administrée sur ou à proximité de la membrane de la fenêtre ronde par l'intermédiaire d'une injection intratympanique.

**2.** Composition pharmaceutique otique destinée à être utilisée selon la revendication 1, comprenant en outre deux caractéristiques ou plus choisies parmi :

(i) d'environ 0,001 % à environ 60 % en poids de l'agoniste de la TrkB, ou d'un promédicament pharmaceutiquement acceptable ou d'un sel de celui-ci ;
(ii) d'environ 14 % à environ 21 % en poids d'un copolymère triséquencé polyoxyéthylène-polyoxypropylène ;
(iii) de l'eau stérile, en quantité suffisante, tamponnée pour fournir un pH situé dans l'intervalle allant d'environ

5,5 àenviron 8,0 ;
(iv) une température de gélification située dans l'intervalle allant d'environ 19 °C à environ 42 °C ; et
(v) une viscosité apparente située dans l'intervalle allant d'environ 100 000 cP à environ 500 000 cP.

**3.** Composition pharmaceutique otique destinée à être utilisée selon la revendication 1 ou la revendication 2, où l'anticorps ou son fragment de liaison est un anticorps monoclonal, un "diabody", un anticorps linéaire, un anticorps monocaténaire, un anticorps bispécifique, un anticorps multispécifique formé à partir de fragments d'anticorps, un anticorps en tandem, un anticorps chimérique, un anticorps murin, un anticorps humanisé, un anticorps issu de la technique de "veneering", un fragment F(ab')2, un fragment Fab', un fragment Fab, un fragment Fv, un fragment rIgG ou un fragment scFv.

**4.** Composition pharmaceutique otique destinée à être utilisée selon l'une quelconque des revendications 1 à 3, où la composition comprend d'environ 14 % à environ 17 % en poids d'un copolymère triséquencé polyoxyéthylène-polyoxypropylène, et fournit une libération prolongée étendue de l'agoniste de la TrkB sur une période allant d'environ 1 semaine à environ 3 semaines.

**5.** Composition pharmaceutique otique destinée à être utilisée selon l'une quelconque des revendications 1 à 4, où la composition pharmaceutique est un gel thermoréversible acceptable pour l'oreille.

**6.** Composition pharmaceutique otique destinée à être utilisée selon l'une quelconque des revendications 1 à 5, où le copolymère triséquencé polyoxyéthylène-polyoxypropylène comprend du poloxamère 407, du poloxamère 188, du poloxamère 237 ou du poloxamère 338.

**7.** Composition pharmaceutique otique destinée à être utilisée selon l'une quelconque des revendications 1 à 6, où la composition présente une température de gélification située dans la plage allant d'environ 19 °C à environ 42 °C.

**8.** Composition pharmaceutique otique destinée à être utilisée selon l'une quelconque des revendications 1 à 7, où le copolymère de polyoxyéthylène-polyoxypropylène est le poloxamère 407.

**9.** Composition pharmaceutique otique destinée à être utilisée selon l'une quelconque des revendications précédentes, où la maladie otique est une perte auditive neurosensorielle.

Fig. 1

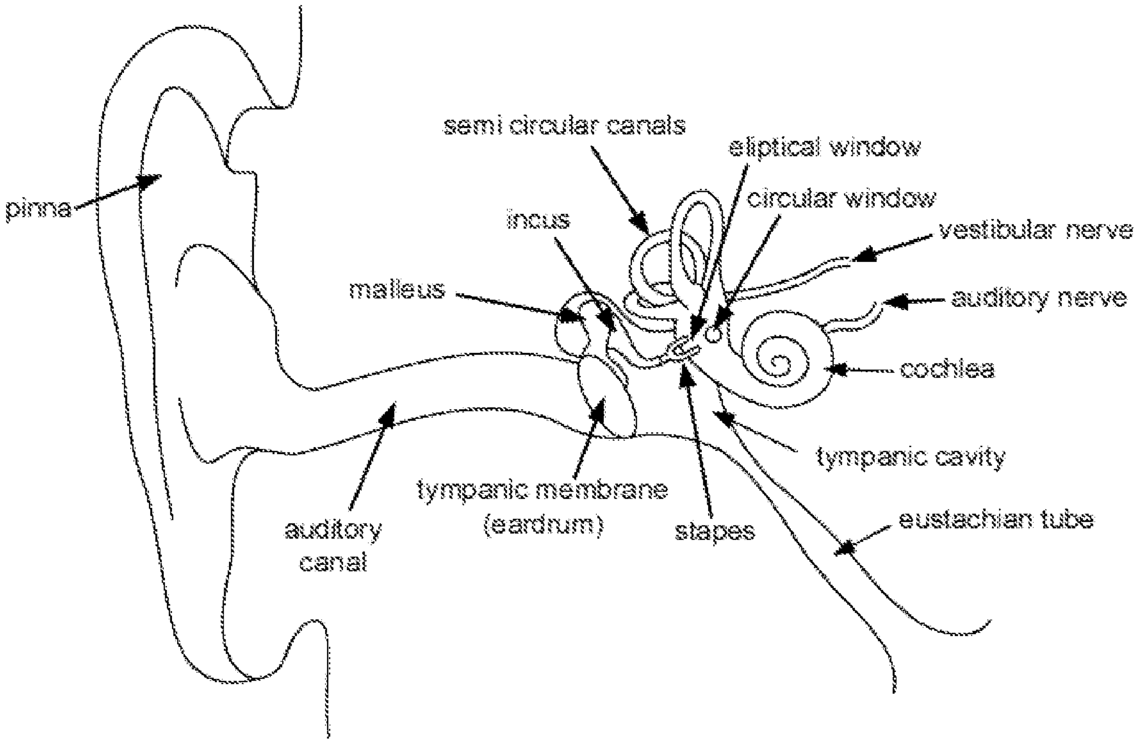
semi circular canals
eliptical window
circular window
vestibular nerve
auditory nerve
pinna
incus
malleus
cochlea
tympanic cavity
tympanic membrane
(eardrum)
stapes
eustachian tube
auditory
canal

Fig. 2A

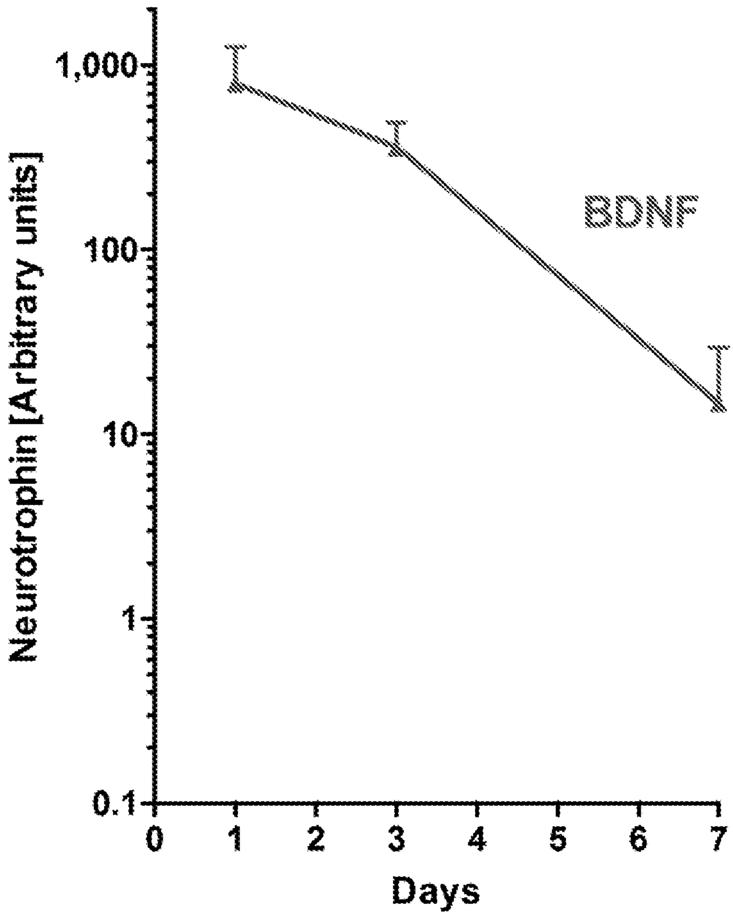
Neurotrophin [Arbitrary units]
1,000
100
10
1
0.1
0
1
2
3
4
5
6
7
Days
BDNF

Fig. 2B

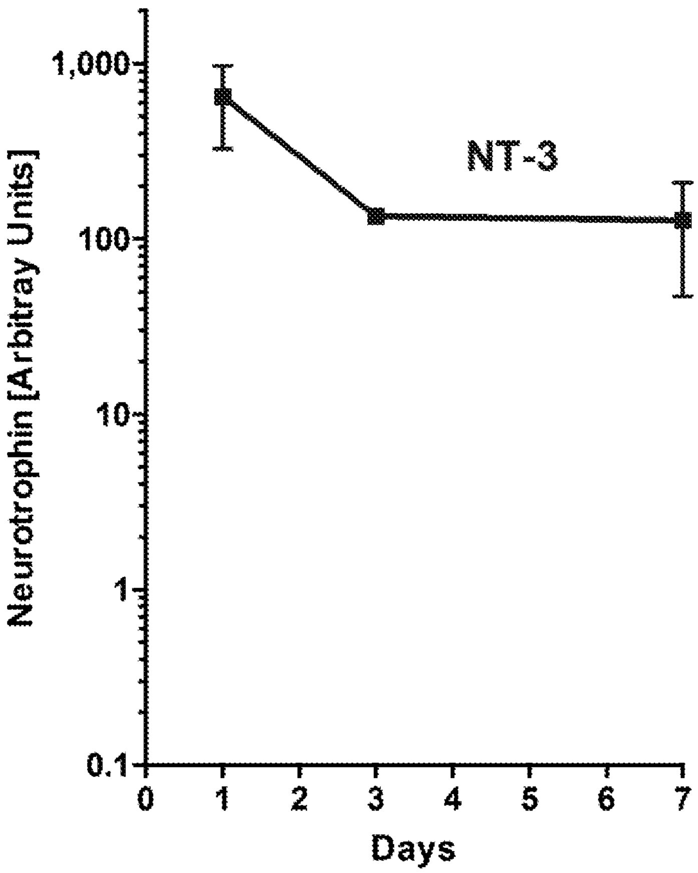
NT-3
Neurotrophin [Arbitray Units]
1,000
100
10
1
0.1
0
1
2
3
4
5
6
7
Days

Fig. 3

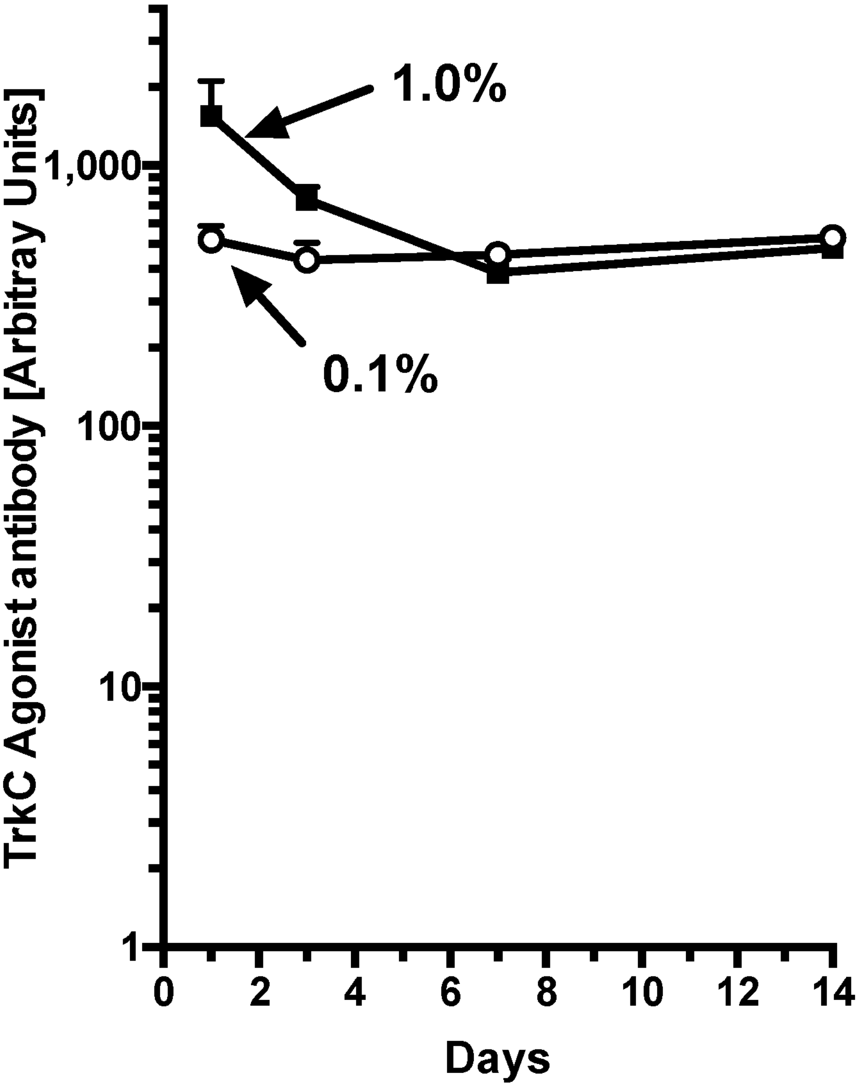
1.0%
0.1%
TrkC Agonist antibody [Arbitray Units]
1,000
100
10
1
0
2
4
6
8
10
12
14
Days

Fig. 4

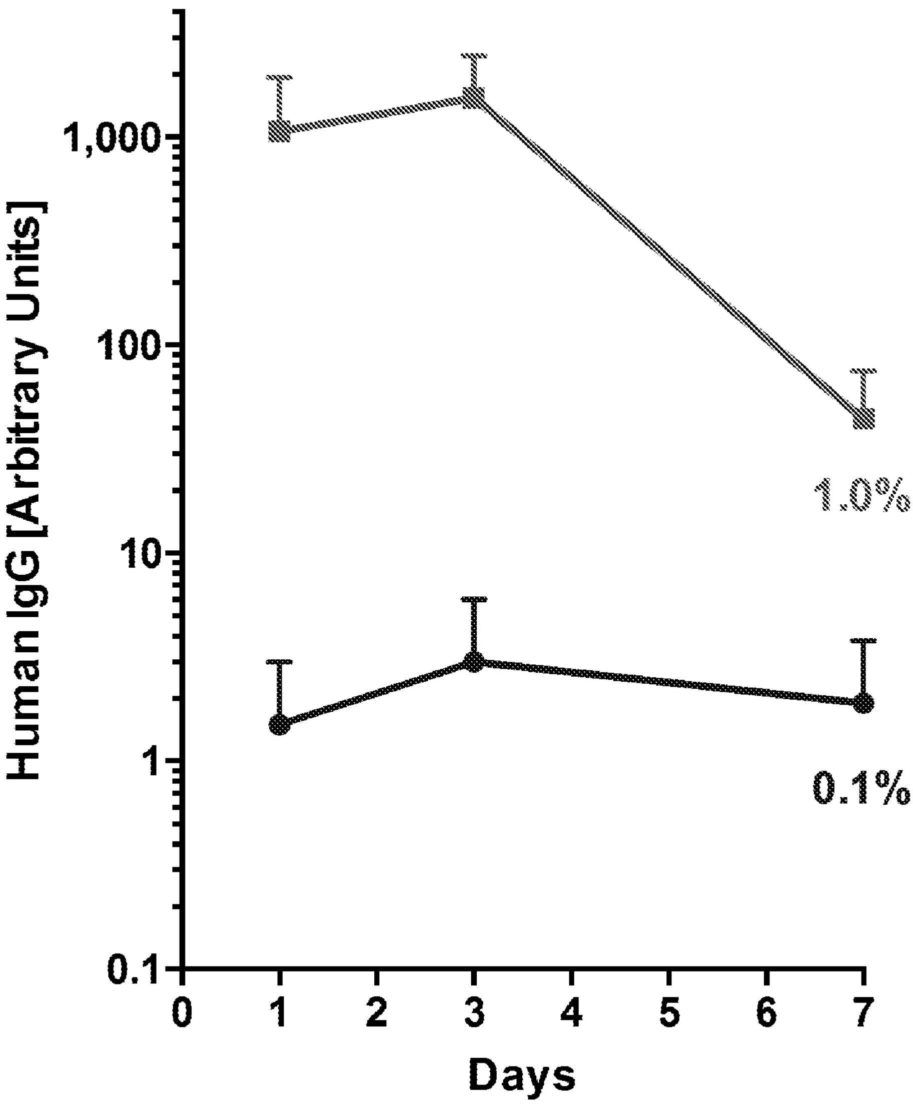

Human IgG [Arbitrary Units]
1,000
100
10
1
0.1
0
1
2
3
4
5
6
7
Days
1.0%
0.1%

Fig. 5

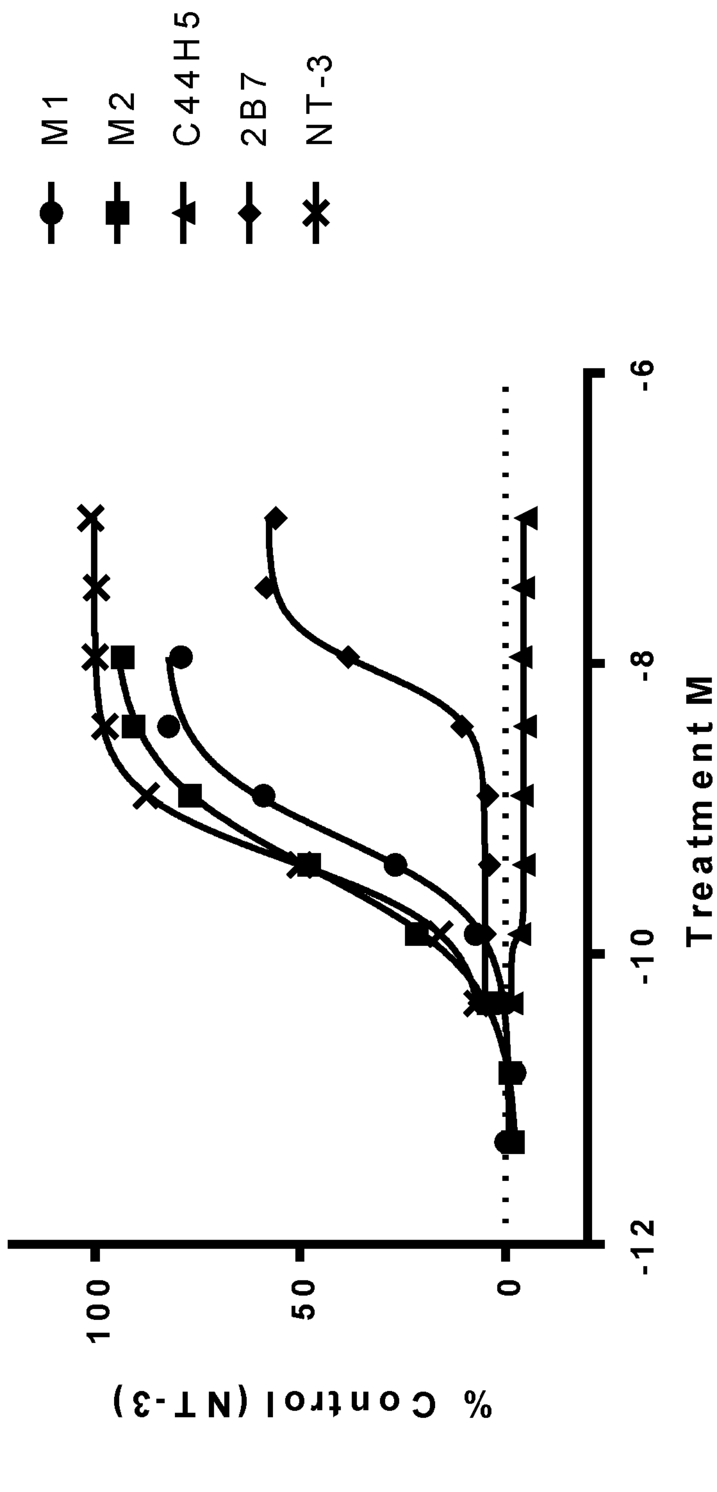

Fig. 6

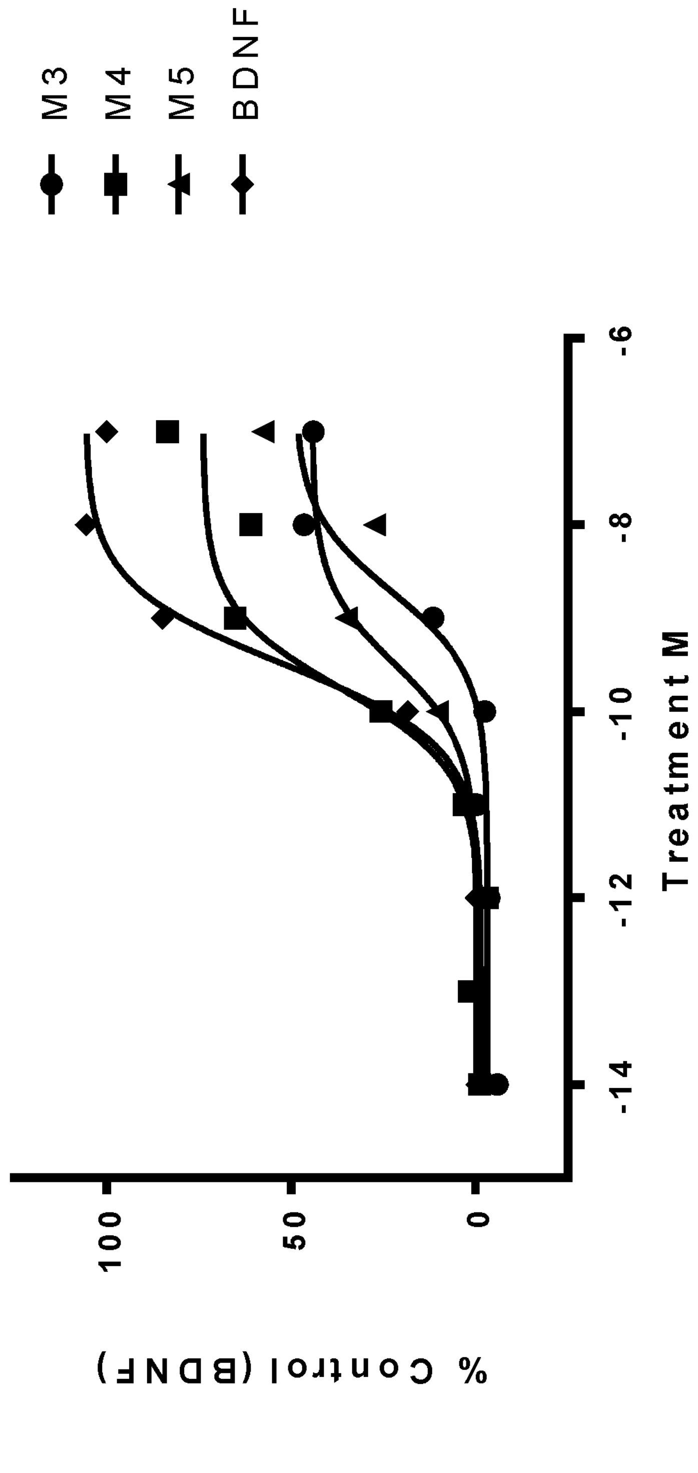

Fig. 7

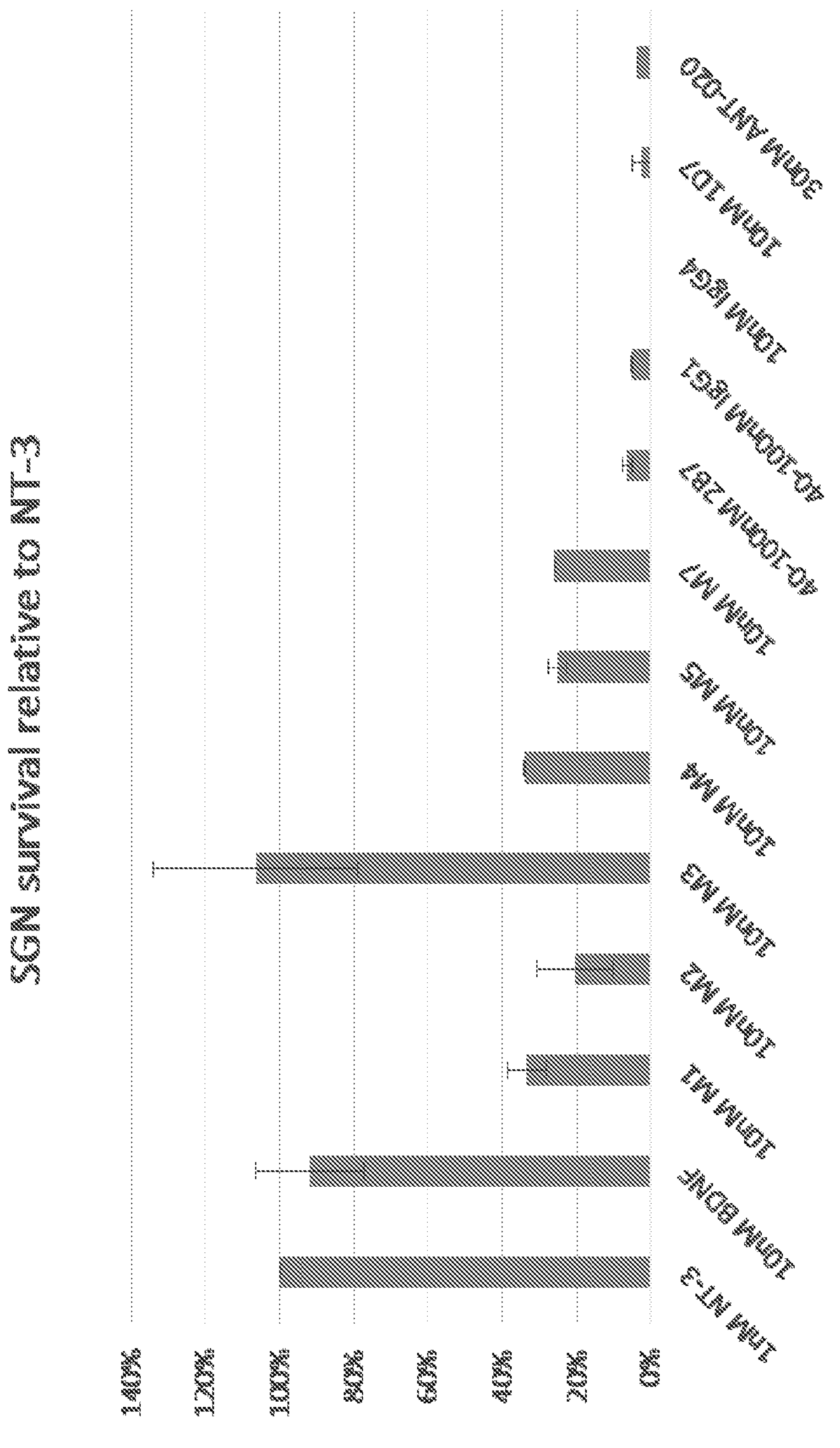
SGN survival relative to NT-3
140%
120%
100%
80%
60%
40%
20%
0%
1nM NT-3
10nM BDNF
10nM M1
10nM M2
10nM M3
10nM M4
10nM M5
10nM M7
40-100nM 2B7
40-100nM IgG1
10nM IgG4
10nM 1D7
30nM ANT-020

Fig. 8A

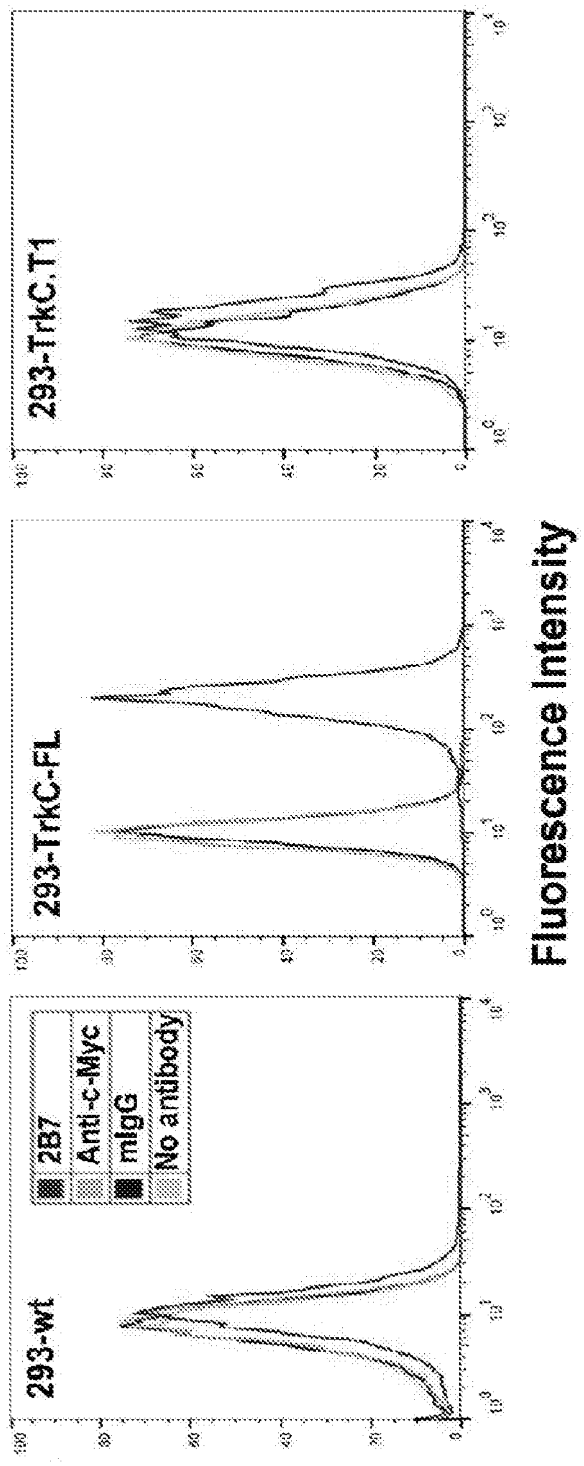

Cell Number
293-wt
2B7
Anti-c-Myc
mIgG
No antibody
293-TrkC-FL
293-TrkC.T1
Fluorescence Intensity


Fig. 8B

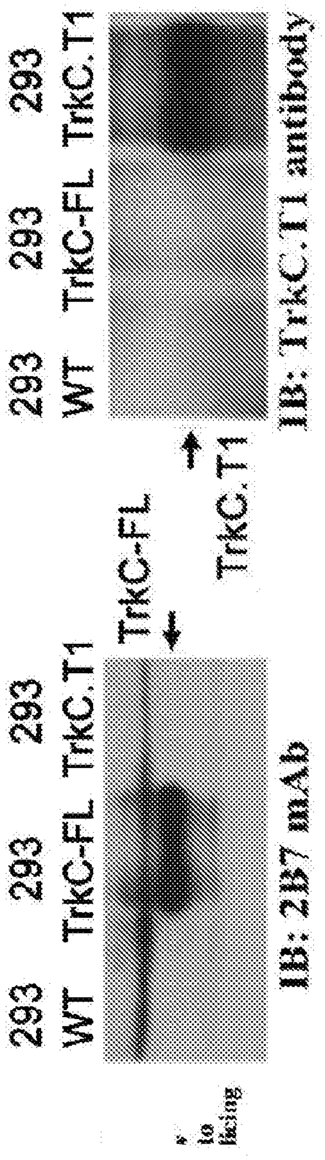

293 WT
293 TrkC-FL
293 TrkC.T1
TrkC-FL
IB: 2B7 mAb
TrkC.T1
IB: TrkC.T1 antibody

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2002039995 A
- WO 2010011609 A
- WO 2009132050 A
- WO 2010086828 A
- US 20140004119 A
- US 13820715 B
- EP 2402756 A
- EP 11183081
- US 7384632 B
- US 6139870 A
- US 6004573 A
- US 6117949 A
- US 6201072 B
- US 6287588 B
- US 906041
- US 6589549 B2
- US 09559799 B
- US 7018645 B1
- US 10919603 B
- US 20060034889 A1
- US 5324519 A
- US 4938763 A
- US 5702716 A
- US 5744153 A
- US 5990194 A
- EP 0551626 A
- US 6638521 B
- US 6562363 B
- US 6509028 B
- US 6348502 B
- US 6319513 B
- US 6306789 B
- US 5814330 A
- US 4900552 A
- US 4530840 A
- US 4675189 A
- US 4758435 A
- US 3773919 A
- US 4474572 A
- US 6458387 B
- US 6268053 B
- US 6090925 A
- US 5981719 A
- US 5578709 A
- US 6083534 A
- US 6036978 A
- US 3737337 A
- US 4389330 A
- US 338805 A
- US 7151191 B
- US 6221367 B
- US 5714167 A
- US 5421818 A
- US 5474529 A
- US 5476446 A
- US 874208
- US 6045528 A
- US 20070167918
- US 5323907 A
- US 5052558 A
- US 5033252 A


### Non-patent literature cited in the description


- **SANGHI et al.** *Eur. Heart J.,* 2005, vol. 26, 538-543
- **PALM et al.** *Nephrol. Dial Transplant,* 1999, vol. 14, 2559-2562
- **LESAUTEUR et al.** *J. Neurosci.,* 1996, vol. 16, 1308-1316
- *Eur. J. Immunol.,* 1999, vol. 29, 2613-2624
- **URFER et al.** The binding epitopes of neurotrophin-3 to its receptors TrkC and gp75 and the design of a multifunctional human neurotrophin. *EMBO,* 1994, vol. 13 (24), 5896-5909
- **ILAG et al.** Pan-neurotrophin 1: A genetically engineered neurotrophic factor displaying multiple specificities in peripheral neurons in vitro and in vivo. *PNAS,* 1995, vol. 92, 607-611
- **IBANEZ et al.** An extended surface of binding to Trk tyrosine kinase receptors in NGF and BDNF allows the engineering of a multifunctional pan-neurotrophin. *EMBO,* 1993, vol. 12 (6), 2281-2293
- **IBANEZ et al.** Chimeric molecules with multiple neurotrophic activities reveal structural elements determining the specificities of NGF and BDNF. *EMBO,* 1991, vol. 10 (8), 2105-2110
- **STEWART, J. M. et al.** Solid Phase Peptide Synthesis. Pierce Chemical Co, 1984

- **BODANSZKY, M. et al.** The Practice of Peptide Synthesis. Springer-Verlag, 1984
- Chemical Sterilization. Remington: The Science and Practice of Pharmacy. Lippincott, Williams & Wilkins
- Microbiological Evaluation of Filters for Sterilizing Liquids. Health Industry Manufacturers Association, 1981, vol. 4
- **RICHARD et al.** *International Journal of Pharmaceutics,* 2006, vol. 312, 144-50
- **TAKTAK et al.** *J. Pharm. Pharmacol.,* 1990, vol. 43, 578-82
- **VIEGAS.** *Int. J. Pharm.,* 1998, vol. 160, 157-162
- **JEONG et al.** *Nature,* 1997, vol. 388, 860-2
- **JEONG et al.** *J. Control. Release,* 2000, vol. 63, 155-63
- **JEONG et al.** *Adv. Drug Delivery Rev.,* 2002, vol. 54, 37-51
- **MAJITHIYA et al.** *AAPS PharmSciTech,* 2006, vol. 7 (3), E1
- **LUZZI, L. A.** *J. Pharm. Psy.,* 1970, vol. 59, 1367
- Controlled Release of Bioactive Agents from Lactides/Glycolide Polymers. **LEWIS, D. H.** Biodegradable Polymers as Drug Delivery Systems. Marcel Decker, 1990
- Poly(lactic acid) and Poly(lactic acid-co-glycolic acid) Contraceptive Delivery Systems. **BECK et al.** Long Acting Steroid Contraception. Raven Press, 1983
- **PARK et al.** *Pharm. Res.,* 1987, vol. 4 (6), 457-464
- **NAHA et al.** *Journal of Microencapsulation,* 04 February 2008
- Handbook of Pharmaceutical Excipients. American Pharmaceutical Association and The Pharmaceutical Society of Great Britain (The Pharmaceutical Press, 1986
- **HANSEN et al.** *J Phys Chem,* 1988, vol. 92, 2189-96
- **GUILLEMARD et al.** *Dev Neurobiol,* 2010, vol. 70, 150-164